# EUROPEAN PATENT SPECIFICATION

(11) **EP 3 901 157 B1**
(45) Date of publication and mention of the grant of the patent: **23.04.2025**
(21) Application number: 19896434.8
(22) Date of filing: 11.12.2019
(51) Int. Cl.: A61K 31/407, A61K 31/436, C07D 498/18, A61P 17/14

(54) **COMPOUND AND COMPOSITION COMPRISING SAME FOR STIMULATING HAIR GROWTH**
VERBINDUNG UND DIESE ENTHALTENDE ZUSAMMENSETZUNG ZUR STIMULIERUNG DES HAARWUCHSES
COMPOSÉ ET COMPOSITION LE COMPRENANT POUR STIMULER LA POUSSE DES CHEVEUX

(30) Priority: 11.12.2018 KR 20180158863; 17.07.2019 KR 20190086645
(43) Date of publication of application: 27.10.2021
(73) Proprietor: Molgenbio Co. Ltd., Seoul 08826 (KR)
(72) Inventor: YOON, Yeo Joon, Seoul 06000 (KR); CHEONG, Eunji, Seoul 07983 (KR); CHO, Seung-Woo, Seoul 06602 (KR); SONG, Myoung-Chong, Seoul 07422 (KR); BEOM, Ji Yoon, Seoul 07213 (KR); JUNG, Jin A, Seoul 01858 (KR); LEE, Jong Seung, Seoul 03481 (KR); LEE, Heon-Joo, Seoul 06504 (KR)
(74) Representative: Sagittarius IP
(86) International application number: PCT/KR2019/017523
(87) International publication number: WO 2020/122611

(56) References cited:
- WO-A1-90/14826
- KR-A- 20050 071 491
- KR-A- 20120 019 360
- US-A- 5 494 820
- US-A1- 2007 078 175
- PARK J W ET AL: "Liquid chromatography-mass spectrometry characterization of FK506 biosynthetic intermediates in Streptomyces clavuligerus KCTC 10561BP", ANALYTICAL BIOCHEMISTRY, ACADEMIC PRESS, AMSTERDAM, NL, vol. 393, no. 1, 1 October 2009 (2009-10-01), pages 1 - 7, XP026419315, ISSN: 0003-2697, [retrieved on 20090617]
- GOULET M T ET AL: "Alkyl ether derivatives of the FK-506 related, immunosuppressive macrolide L-683,742 (C31-O-desmethyl ascomycin)", BIOORGANIC & MEDICINAL CHEMISTRY LETTERS, ELSEVIER, AMSTERDAM NL, vol. 4, no. 7, 1 January 1994 (1994-01-01), pages 927 - 930, XP026646494, ISSN: 0960-894X, [retrieved on 19940101], DOI: 10.1016/S0960-894X(01)80265-1
- SATOSHI YAMAMOTO ET AL: "Stimulation of Hair Growth by Topical Application of FK506, a Potent Immunosuppressive Agent", JOURNAL OF INVESTIGATIVE DERMATOLOGY, vol. 102, no. 2, 1 February 1994 (1994-02-01), NL, pages 160 - 164, XP055679945, ISSN: 0022-202X, DOI: 10.1111/1523-1747.ep12371755
- YAMAMOTO, S.: "Stimulation of Hair Growth by Topical Application of FK506, a Potent Immunosuppressive Agent", JOURNAL OF INVESTIGATIVE DERMATOLOGY, vol. 1201, no. 425, 1994, pages 160 - 164, XP055679945
- IWABUCHI, T. ET AL.: "Effects of immunosuppressive peptidyl-prolyl cis-trans isomerase (PPIase) inhibitors, cyclosporin A, FK506, ascomycin and rapamycin, on hair growth initiation in mouse: immunosuppression is not required for new hair growth", JOURNAL OF DERMATOLOGICAL SCIENCE, 1995, XP055247408

## Description

### [Technical Field]

The present invention relates to preparation and use of compounds available as main ingredients of a composition for promoting hair growth, wherein the compounds are 9-deoxo-31-*O*-demethyl-prolyl-FK506. 9-deoxo-36,37-dihydro-FK506, 31-O-demethyl-36,37-dihydro-FK506, 9-deoxo-31-*O*-demethyl-36,37-dihydro-FK506, 9-deoxo-FK520, 31-*O*-demethyl-FK520, 9-deoxo-31-O-demethyl-FK520, 9-deoxo-FK523, and 9-deoxo-31-*O*-demethyl-FK523. More particularly, the present invention relates to methods of preparing the compounds and compositions for promoting hair growth including each of the compounds as an active ingredient

### [Background Art]

Hair loss (alopecia) in modern people has become more common not only due to aging or genetic causes but also due to the influence of acquired factors such as hormone imbalance caused by environmental contamination, smoking, stress from work, and changes in dietary patterns. While the population with hair loss has increased mainly in middle-aged and elderly male people around in their 40s to 50s, the number of younger people and females suffering from hair loss has also been increasing in recent years.

According to statistical analysis of the Korean Society of Hair Restoration Treatment, the population with hair loss domestically accounts for about 10 million including potential alopecia patients. The medical expenditure therefor was about 35.5 billion won in 2016, and the number of people with hair loss is increasing abroad as well as in Korea. The market size for hair loss management in the U.S., measured by IBIS World Industry Market Research, was 3.6 billion dollars in 2016 and is projected to grow at a rate of 0.7% every year until 2021.

Meanwhile, according to a survey of the Korean Association of Chronic Disease Management, seven out of 10 Korean adults recognize hair loss as a disease and think that hair loss causes direct or indirect losses in social lives. Also, about 23% of adults experience hair loss.

Alopecia is classified into non-cicatricial alopecia, which is temporary hair loss, and cicatricial alopecia caused by permanent destruction of hair follicles or hair roots. Non-cicatricial alopecia is a common form of hair loss, and is classified into infectious hair loss, traumatic hair loss, inflammatory hair loss, congenital hair loss, endocrine hair loss, neoplastic alopecia, malnutrition hair loss, drug-induced hair loss. Hair loss caused by abnormal hair structure, male- and female-pattern hair loss, and alopecia areata also belong to non-cicatricial alopecia.

As the economy grows and society ages, hair loss has been considered as a disease rather than an uncontrollable phenomenon, and there is a need to develop drugs effective in the treatment of hair loss, which causes psychological anxiety and stress in work and social lives.

However, although the number of people with hair loss is increasing, the exact causes of hair loss have not been identified, and there are no effective methods for preventing hair loss. Due to the current situation, effective technologies for preventing hair loss and promoting hair growth have gained more interest, and a variety of prevention agents are present on the market.

Currently, Minoxidil for topical administration and Propecia for oral administration are drugs approved by the FDA. However, these drugs do not provide permanent therapeutic effects, but only provide effects on delaying the progression of hair loss or maintaining the current state. In addition, topical hair growth-promoting agents such as Minoxidil may be troublesome since they need to be applied every day to maintain the effects on promoting hair growth, and have lesser effects than orally administered agents. In addition, Propecia may increase the likelihood of congenital malformations in infants when administered to females, and may cause side effects such as sexual dysfunction. Therefore, there is a need to develop drugs capable of providing fundamental therapeutic effects beyond the level of temporary effects. Moreover, drugs available not only for therapeutic use but also for preventive use may be more valuable.

Although Korean Patent Laid-open Publication No. 10-2005-0071491 (Title of Invention: Use of tacrolimus (FK506) derivatives combined with beta2-agonists for treatment of asthma) discloses a novel use of FK506 derivatives and beta2-agonists for manufacturing a medicament for simultaneous, separate, or sequential use to treat or prevent acute or chronic asthma, there have been no studies on applying FK506 derivatives, FK520 derivatives, or FK523 derivatives to promote hair growth.

US5494820A discloses a new macrocyclic lactone immunosuppressant agent, a process for its production and its use in a human host for treatment of autoimmune diseases and/or prevention of organ transplant rejections.

WO9014826A1 discloses a novel treatment of reversible obstructive airways disease, more particularly to the use of macrocyclic compounds in the treatment of reversible obstructive airways disease, and to compositions containing such compounds.

Park, J. W. et al. (2009). (Analytical biochemistry, 393(1), 1-7) discloses a method for detecting the biosynthetic intermediates involved in the FK506 pathway of *Streptomyces clavuligerus* KCTC 10561BP.

Goulet, M. T et al. (1994). (Bioorganic & Medicinal Chemistry Letters, 4(7), 927-930) discloses ether derivatives of the FK-506 related macrolide L-683,742 (C31-O-desmethyl ascomycin).

Yamamoto, S et al. (1994). (Journal of investigative dermatology, 102(2), 160-164) discloses topical application of FK506 for stimulating hair growth.

### [Disclosure]

### [Technical Problem]

An object of the present invention is to provide the nine compounds, or pharmaceutically acceptable salts thereof.

Another object of the present invention is to provide a composition for promoting hair growth including at least one selected from the nine compounds as an active ingredient. In this regard, the composition for promoting hair growth refers to a composition for alleviating, preventing, or treating hair loss.

Another object of the present invention is to provide a pharmaceutical composition for alleviating, preventing, or treating hair loss including at least one selected from the nine compounds or pharmaceutically acceptable salts thereof as an active ingredient.

Another object of the present invention is to provide biological manufacturing processes of each of the nine compounds.

Another object of the present invention is to provide production strains available in biological manufacturing processes the nine novel compounds, *Streptomyces kanamyceticus* ΔfkbD-fkbM (accession number: KCTC13581BP), *Streptomyces kanamyceticus* ΔfkbD,tcsD (accession number: KCTC13580BP), *Streptomyces kanamyceticus* ΔfkbM,tcsD (accession number: KCTC13584BP), *Streptomyces kanamyceticus* ΔfkbD-fkbM,tcsD (accession number: KCTC13585BP), *Streptomyces kanamyceticus* ΔfkbD,tcsB (accession number: KCTC13579BP), *Streptomyces kanamyceticus* ΔfkbM,tcsB (accession number: KCTC13583BP), and *Streptomyces kanamyceticus* ΔfkbD-fkbM,tcsB (accession number: KCTC13582BP).

Another object of the present invention is to provide an over-the-counter (OTC) composition for promoting hair growth including at least one selected from the nine compounds, or a pharmaceutically acceptable salt thereof as an active ingredient.

Another object of the present invention is to provide a functional food composition for promoting hair growth including at least one selected from the nine compounds, or a sitologically acceptable salt thereof as an active ingredient

Another object of the present invention is to provide a cosmetic composition for promoting hair growth including at least one selected from the nine compounds or a cosmetically acceptable salt thereof as an active ingredient.

### [Technical Solution]

As a result of intensive efforts, the present inventors have found hair growth-promoting effects of novel compounds, 9-deoxo-31-*O*-demethyl-prolyl-FK506, 9-deoxo-36,37-dihydro-FK506, 31-*O*-demethyl-36,37-dihydro-FK506, 9-deoxo-31-*O*-demethyl-36,37-dihydro-FK506, 9-deoxo-FK520, 31-*O*-demethyl-FK520, 9-deoxo-31-*O*-demethyl-FK520, 9-deoxo-FK523, and 9-deoxo-31-*O*-demethyl-FK523 (hereinafter collectively referred to as nine compounds), available as main ingredients of compositions for promoting hair growth, have developed manufacturing processes thereof and compositions for promoting hair growth using the compounds as active ingredients, and confirmed that the compositions may be effectively used as compositions for promoting hair growth, thereby completing the present invention.

### [Advantageous Effects]

The composition for promoting hair growth including at least one selected from the nine compounds according to the present invention as an active ingredient may provide effects on promoting hair growth in alleviation, prevention, and treatment of hair loss, thereby providing fundamental preventive and therapeutic effects.

### [Brief Description of Drawings]

FIG. 1 shows high-performance liquid chromatography analysis results of 9-deoxo-31-*O*-demethyl-prolyl-FK506.
FIG. 2 shows nuclear magnetic resonance analysis (¹H-NMR) results of 9-deoxo-31-*O*-demethyl-prolyl-FK506.
FIG. 3 shows nuclear magnetic resonance analysis (¹³C-NMR) results of 9-deoxo-31-*O*-demethyl-prolyl-FK506.
FIG. 4 shows nuclear magnetic resonance analysis (COSY-NMR) results of 9-deoxo-31-*O*-demethyl-prolyl-FK506.
FIG. 5 shows nuclear magnetic resonance analysis (HSQC-NMR) results of 9-deoxo-31-*O*-demethyl-prolyl-FK506.
FIG. 6 shows nuclear magnetic resonance analysis (HMBC-NMR) results of 9-deoxo-31-*O*-demethyl-prolyl-FK506.
FIG. 7 shows high-performance liquid chromatography analysis results of 9-deoxo-36,37-dihydro-FK506.
FIG. 8 shows nuclear magnetic resonance analysis (¹H-NMR) results of 9-deoxo-36,37-dihydro-FK506.
FIG. 9 shows nuclear magnetic resonance analysis (¹³C-NMR) results of 9-deoxo-36,37-dihydro-FK506.
FIG. 10 shows nuclear magnetic resonance analysis (COSY-NMR) results of 9-deoxo-36,37-dihydro-FK506.
FIG. 11 shows nuclear magnetic resonance analysis (HSQC-NMR) results of 9-deoxo-36,37-dihydro-FK506.
FIG. 12 shows nuclear magnetic resonance analysis (HMBC-NMR) results of 9-deoxo-36,37-dihydro-FK506.
FIG. 13 shows high-performance liquid chromatography analysis results of 31-*O*-demethyl-36,37-dihydro-FK506.
FIG. 14 shows nuclear magnetic resonance analysis (¹H-NMR) results of 31-*O*-demethyl-36,37-dihydro-FK506.
FIG. 15 shows nuclear magnetic resonance analysis (¹³C-NMR) results of 31-*O*-demethyl-36,37-dihydro-FK506.
FIG. 16 shows nuclear magnetic resonance analysis (COSY-NMR) results of 31-*O*-demethyl-36,37-dihydro-FK506.
FIG. 17 shows nuclear magnetic resonance analysis (HSQC-NMR) results of 31-*O*-demethyl-36,37-dihydro-FK506.
FIG. 18 shows nuclear magnetic resonance analysis (HMBC-NMR) results of 31-*O*-demethyl-36,37-dihydro-FK506.
FIG. 19 shows high-performance liquid chromatography analysis results of 9-deoxo-31-*O*-demethyl-36,37-dihydro-FK506.
FIG. 20 shows nuclear magnetic resonance analysis (¹H-NMR) results of 9-deoxo-31-*O*-demethyl-36,37-dihydro-FK506.
FIG. 21 shows nuclear magnetic resonance analysis (¹³C-NMR) results of 9-deoxo-31-*O*-demethyl-36,37-dihydro-FK506.
FIG. 22 shows nuclear magnetic resonance analysis (COSY-NMR) results of 9-deoxo-31-*O*-demethyl-36,37-dihydro-FK506.
FIG. 23 shows nuclear magnetic resonance analysis (HSQC-NMR) results of 9-deoxo-31-*O*-demethyl-36,37-dihydro-FK506.
FIG. 24 shows nuclear magnetic resonance analysis (HMBC-NMR) results of 9-deoxo-31-*O*-demethyl-36,37-dihydro-FK506.
FIG. 25 shows high-performance liquid chromatography analysis results of 9-deoxo-FK520.
FIG. 26 shows nuclear magnetic resonance analysis (¹H-NMR) results of 9-deoxo-FK520.
FIG. 27 shows nuclear magnetic resonance analysis (¹³C-NMR) results of 9-deoxo-FK520.
FIG. 28 shows nuclear magnetic resonance analysis (COSY-NMR) results of 9-deoxo-FK520.
FIG. 29 shows nuclear magnetic resonance analysis (HSQC-NMR) results of 9-deoxo-FK520.
FIG. 30 shows nuclear magnetic resonance analysis (HMBC-NMR) results of 9-deoxo-FK520.
FIG. 31 shows high-performance liquid chromatography analysis results of 31-*O*-demethyl-FK520.
FIG. 32 shows nuclear magnetic resonance analysis (¹H-NMR) results of 31-*O*-demethyl-FK520.
FIG. 33 shows nuclear magnetic resonance analysis (¹³C-NMR) results of 31-*O*-demethyl-FK520.
FIG. 34 shows nuclear magnetic resonance analysis (COSY-NMR) results of 31-*O*-demethyl-FK520.
FIG. 35 shows nuclear magnetic resonance analysis (HSQC-NMR) results of 31-*O*-demethyl-FK520.
FIG. 36 shows nuclear magnetic resonance analysis (HMBC-NMR) results of 31-*O*-demethyl-FK520.
FIG. 37 shows high-performance liquid chromatography analysis results of 9-deoxo-31-*O*-demethyl-FK520.
FIG. 38 shows nuclear magnetic resonance analysis (¹H-NMR) results of 9-deoxo-31-*O*-demethyl-FK520.
FIG. 39 shows nuclear magnetic resonance analysis (¹³C-NMR) results of 9-deoxo-31-*O*-demethyl-FK520.
FIG. 40 shows nuclear magnetic resonance analysis (COSY-NMR) results of 9-deoxo-31-*O*-demethyl-FK520.
FIG. 41 shows nuclear magnetic resonance analysis (HSQC-NMR) results of 9-deoxo-31-*O*-demethyl-FK520.
FIG. 42 shows nuclear magnetic resonance analysis (HMBC-NMR) results of 9-deoxo-31-*O*-demethyl-FK520.
FIG. 43 shows high-performance liquid chromatography analysis results of 9-deoxo-FK523.
FIG. 44 shows nuclear magnetic resonance analysis (¹H-NMR) results of 9-deoxo-FK523.
FIG. 45 shows nuclear magnetic resonance analysis (¹³C-NMR) results of 9-deoxo-FK523.
FIG. 46 shows nuclear magnetic resonance analysis (COSY-NMR) results of 9-deoxo-FK523.
FIG. 47 shows nuclear magnetic resonance analysis (HSQC-NMR) results of 9-deoxo-FK523.
FIG. 48 shows nuclear magnetic resonance analysis (HMBC-NMR) results of 9-deoxo-FK523.
FIG. 49 shows high-performance liquid chromatography analysis results of 9-deoxo-31-*O*-demethyl-FK523.
FIG. 50 shows nuclear magnetic resonance analysis (¹H-NMR) results of 9-deoxo-31-*O*-demethyl-FK523.
FIG. 51 shows nuclear magnetic resonance analysis (¹³C-NMR) results of 9-deoxo-31-*O*-demethyl-FK523.
FIG. 52 shows nuclear magnetic resonance analysis (COSY-NMR) results of 9-deoxo-31-*O*-demethyl-FK523.
FIG. 53 shows nuclear magnetic resonance analysis (HSQC-NMR) results of 9-deoxo-31-*O*-demethyl-FK523.
FIG. 54 shows nuclear magnetic resonance analysis (HMBC-NMR) results of 9-deoxo-31-*O*-demethyl-FK523.
FIG. 55 shows degrees of decreases in immunosuppressive activity of the nine novel compounds of the present invention.
FIG. 56 shows hair growth-promoting activity of the novel compounds of the present invention.
FIG. 57 shows hair growth-promoting activity of the novel compounds of the present invention by identifying increases in length of vibrissae.
FIG. 58 shows results of investigation of hair growth-promoting activity based on histological results of vibrissae treated with the novel compounds of the present invention.

### [Best Mode]

Hereinafter, the present invention will be described in detail.

To solve these problems, the present invention provides methods of preparing nine compounds, compositions for promoting hair growth including each of the compounds by application of the preparation methods, and methods of alleviating, preventing, and treating hair loss.

An aspect of the present invention to achieve the above objects provides a compound selected from the group consisting of nine compounds, i.e. 9-deoxo-31-*O*-demethyl-prolyl-FK506 represented by Formula 1 below, 9-deoxo-36,37-dihydro-FK506 represented by Formula 2 below, 31-*O*-demethyl-36,37-dihydro-FK506 represented by Formula 3 below, 9-deoxo-31-*O*-demethyl-36,37-dihydro-FK506 represented by Formula 4 below, 9-deoxo-FK520 represented by Formula 5 below, 31-*O*-demethyl-FK520 represented by Formula 6 below, 9-deoxo-31-*O*-demethyl-FK520 represented by Formula 7 below, 9-deoxo-FK523 represented by Formula 8 below, and 9-deoxo-31-*O*-demethyl-FK523 represented by Formula 9 below, or a pharmaceutically acceptable salt thereof.

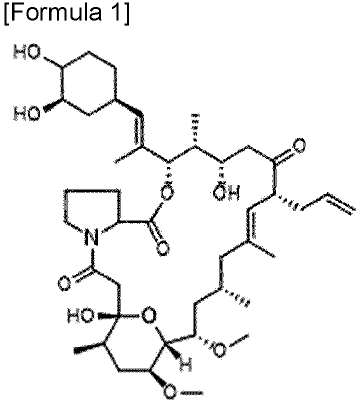

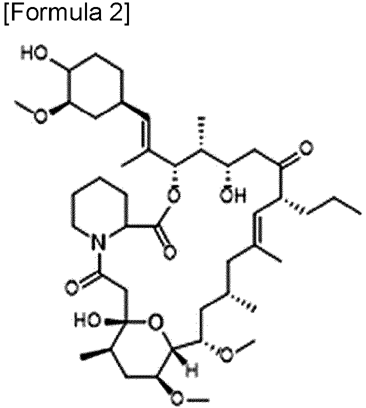

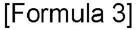

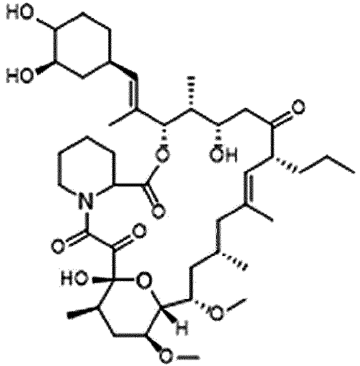

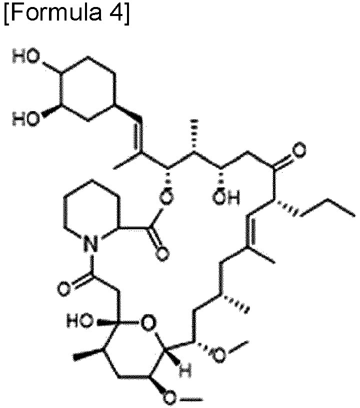

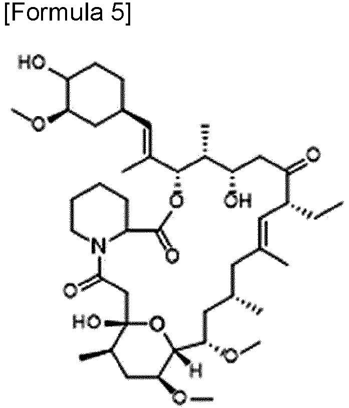

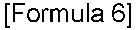

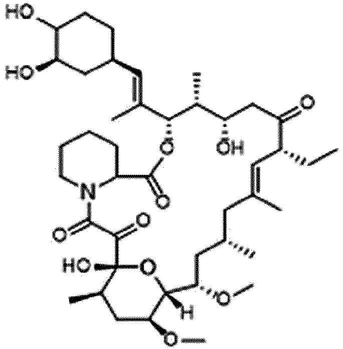

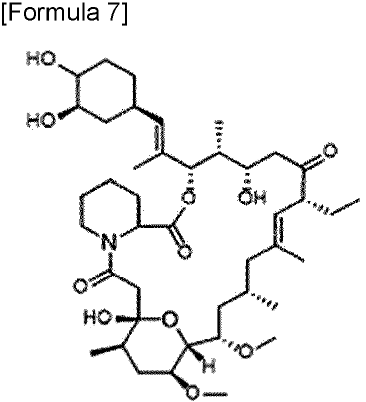

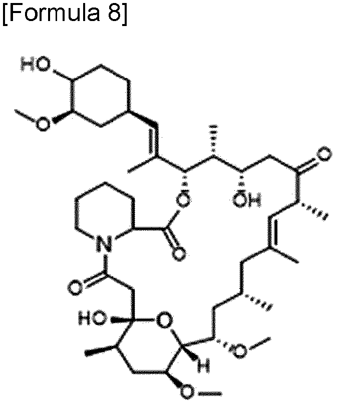

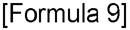

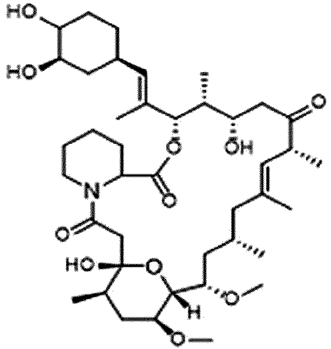

In a specific embodiment, the compound of the present invention may include pharmaceutically acceptable salts thereof.

Isomers refer to compounds with the same chemical formula but a different atomic arrangement and may include, for example, structural isomers, geometric isomers, optical isomers (enantiomers), stereoisomers, and diastereomers.

The pharmaceutically acceptable salts may refer to any organic or inorganic addition salts of an original compound which do not cause side effects and impairing beneficial effects of the compound in a concentration effective for patients, but which are relatively non-toxic and harmless to the patients. For example, the pharmaceutically acceptable salt may be an acid addition salt formed of a pharmaceutically acceptable free acid. The acid addition salt may be prepared by way of any method commonly used in the art, for example, by dissolving the compound in an excess amount of an aqueous solution and precipitating a salt using a water-miscible organic solvent, *e.g.*, methanol, ethanol, acetone, or acetonitrile. The compound and acid or alcohol (*e.g.*, glycol monomethyl ether) in water in the same molar amount may be heated followed by drying of the mixture by evaporation, or a precipitated salt may be filtered by suction. In this regard, the free acid may be an organic or inorganic acid. The salt may be a pharmaceutically acceptable metal salt prepared using a base.

In another specific embodiment, the compound of the present invention may be in the form of a solvate which is within the scope of the present invention. The solvate may preferably include a hydrate and an ethanolate.

The composition of the present invention may be used as a single formulation or combined with various drugs known to have preventive or therapeutic effects on hair loss, and the composition may be formulated using a pharmaceutically acceptable carrier or excipient into a single-dose dosage form or a unit dosage form in a multi-dose container.

As used herein, the term "pharmaceutically acceptable carrier" refers to a carrier or diluent used without causing irritation to a living organism and damaging biological activities or properties of the administered compound. Types of the carrier available in the present invention are not particularly limited, and any type of pharmaceutically acceptable carrier commonly used in the art may be used. Non-limiting examples of the carrier may include a co-surfactant such as Transcutol, polyethylene glycol, Triacetin, and any mixture thereof; a surfactant such as Cremophor, Tween, Myrj, Poloxamer, Pluronic, Lutrol, Imwitor, Span, and Labrafil, alone or in a mixture; an oil such as Miglyol, Captex, and ethyl oleate, alone or in a mixture; and an organic acid such as erythorobic acid and citric acid, alone or in a mixture. These carriers may be used alone or in a mixture of at least two thereof.

Also, if required, the composition may further include any known additive such as an antioxidant, a buffer, and/or a bacteriostatic agent. The composition may be formulated into injectable dosage forms such as aqueous solutions, suspensions, and emulsions, pills, capsules, granules, or tablets, in combination with a diluent, a dispersant, a surfactant, a binder, a lubricant, or the like.

Another aspect of the present invention to achieve the above objects provides cosmetic methods of preventing or treating hair loss, the methods including administering the composition to an individual.

As used herein, the term "individual" refers to all animals with hair loss or at risk of developing hair loss.

The composition of the present invention may include at least one selected from the nine compounds, or a salt thereof in a pharmaceutically effective amount. As used herein, the term "pharmaceutically effective amount" refers to an amount sufficient for treating a disease at a reasonable benefit/risk ratio applicable to medical treatment, and a daily dosage of the composition may generally be from 0.001 mg/kg to 1000 mg/kg, preferably 0.05 mg/kg to 200 mg/kg, and more preferably 0.1 mg/kg to 100 mg/kg once to several times per day. However, for the purpose of the present invention, it is preferred that a specific therapeutically effective amount for a specific patient be differently applied depending on various factors including the type and extent of a response to be achieved, a specific composition, including whether other formulations are used according to the case, the age, body weight, general health status, gender, and diet of the patient, administration time, administration route, excretion rate of the composition, duration of treatment, and a drug used in combination or concurrently with the specific composition, and similar factors well known in the medical field.

Although not particularly limited thereto, the composition of the present invention may be administered once a day or several times a day in divided doses.

The composition of the present invention may be administered alone or in combination with other therapeutic agents and may be administered sequentially or simultaneously with existing therapeutic agents. The composition may be administered in a single- or multiple-dosage form. It is important to administer the composition in a minimum amount that may exhibit a maximum effect without causing side effects, considering all of the above-described factors. The amount may be readily determined by those skilled in the art.

As used herein, the term "administration" refers to introducing the composition of the present invention into a patient by way of any appropriate method. The composition of the present invention may be administered by way of any oral or parenteral routes as long as the composition reaches a target tissue.

Administration modes of the composition of the present invention are not particularly limited, and any administration mode commonly available in the art may be used. Non-limiting examples of the administration mode include oral or parenteral administration modes. The composition of the present invention may be prepared into various dosage forms, depending on desired administration modes.

The composition for promoting hair growth of the present invention may be used to alleviate, prevent, or treat hair loss.

In the present invention, alopecia includes both non-cicatricial alopecia, which is temporary hair loss, and cicatricial alopecia caused by permanent destruction of hair follicles or hair roots, and non-cicatricial alopecia includes infectious hair loss, traumatic hair loss, inflammatory hair loss, congenital hair loss, endocrine hair loss, alopecia neoplastica, malnutrition hair loss, drug-induced hair loss, hair loss caused by abnormal hair structure, male-pattern hair loss, female-pattern hair loss, and alopecia areata.

As used herein, the term "alleviation" refers to all actions intended to delay progression of hair loss or alleviate symptoms of hair loss by administering the composition of the present invention to an individual.

As used herein, the term "prevention" refers to all actions intended to inhibit or delay hair loss by administration of the composition of the present invention to an individual.

As used herein, the term "treatment" refers to all actions intended to alleviate or beneficially change symptoms of hair loss by administering the composition of the present invention to an individual suspected to have hair loss. In a specific embodiment of the present invention, immunosuppressive effects of the nine compounds were confirmed by *in vitro* T cell activity assay. As a result, decreased immunosuppressive effects thereof were confirmed compared to those of FK506.

Also, in another specific embodiment of the present invention, effects of the nine compounds on promoting the growth of vibrissae were confirmed

In another specific embodiment of the present invention, the effects of the nine compounds on promoting the growth of vibrissae in the length were confirmed, and the cell growth marker was confirmed based on histological results, and thus it was confirmed that the growth of vibrissae was continuously maintained.

These results indicate that the nine compounds have effects on promoting hair growth without side effects caused by immunosuppressive activity and can thereby be effectively used to alleviate, prevent, or treat hair loss.

Another aspect of the present invention to achieve the above objects provides biologically manufacturing processes of nine compounds. 9-deoxo-31-*O*-demethyl-prolyl-FK506, 9-deoxo-36,37-dihydro-FK506, 31-*O*-demethyl-36,37-dihydro-FK506, 9-deoxo-31-*O*-demethyl-36,37-dihydro-FK506, 9-deoxo-FK520, 31-*O*-demethyl-FK520, 9-deoxo-31-*O*-demethyl-FK520, 9-deoxo-FK523, and 9-deoxo-31-*O*-demethyl-FK523.

In the biological manufacturing processes, a culture temperature adopted in general processes of culturing microorganisms belonging to the genus *Streptomyces* is used. The culture temperature appropriate for the embodiment of the present invention may preferably be in the range of 23°C to 30°C, more preferably, in the range of 25°C to 28°C.

Also, in the manufacturing processes, the pH of the culturing process is maintained in the range of 6.5 to 9, preferably in the range of 7 to 8.

Meanwhile, it is important to maintain a high level of dissolved oxygen in the manufacturing processes. When the dissolved oxygen level in the early stage of culturing is assumed to be 100%, it is important to maintain a dissolved oxygen level of 30% or more until the culturing is terminated. To this end, the stirring may be performed at a rate of 800 rpm to 1,500 rpm.

The nine compounds produced by the cultured microbial cells in the manufacturing process may be extracted by way of a first extraction process, a second extraction process, and a third extraction process. In the present invention, the first extraction process is performed by way of an organic solvent extraction method, and a solvent used therefor may be ethyl acetate, methanol, or acetone, preferably ethyl acetate or methanol. The second extraction process may be performed using silica gel chromatography, and methanol or methylene chloride may be used as solvents. In addition, the third extraction process may be performed using chromatography, and a solvent used therefor may be acetonitrile, an ammonium acetate buffer, acetic acid, or formic acid, preferably acetonitrile. Application of these methods facilitates the recovery of the nine novel compounds and also increases the yield.

Another aspect of the present invention to achieve the above objects provides production strains available in the manufacture of nine compounds, i.e. *Streptomyces kanamyceticus* ΔfkbD-fkbM (accession number: KCTC13581BP), *Streptomyces kanamyceticus* ΔfkbD,tcsD (accession number: KCTC13580BP), *Streptomyces kanamyceticus* ΔfkbM,tcsD (accession number: KCTC13584BP), *Streptomyces kanamyceticus* ΔfkbD-fkbM,tcsD (accession number: KCTC13585BP), *Streptomyces kanamyceticus* ΔfkbD,tcsB (accession number: KCTC13579BP), *Streptomyces kanamyceticus* ΔfkbM,tcsB (accession number: KCTC13583BP), and *Streptomyces kanamyceticus* ΔfkbD-fkbM,tcsB (accession number: KCTC13582BP).

Another object of the present invention is to provide an over-the-counter (OTC) composition for promoting hair growth including at least one compound selected from nine compounds, or a pharmaceutically acceptable salt thereof as an active ingredient.

When the composition including at least one selected from the nine compounds is added to an OTC composition for the purpose of promoting hair growth, the compound may be added as it is or may be used together with other OTC components, and may be used appropriately according to any methods commonly used in the art. A mixing amount of the active ingredient may be appropriately determined according to the purpose of use.

As used herein, the term "OTC composition" refers to fibers or rubber products, or similar products used for the purpose of medical care, alleviation, treatment, or prevention of diseases in humans or animals; non-appliances, non-machinery, or similar products which have insignificant influences on or do not directly act upon the human body; and preparations used for sterilization, insecticide, and purposes similar thereto in order to prevent communicable diseases, and the OTC product does not include products used for the purposes of diagnosis, medical care, alleviation, treatment, or prevention of diseases of humans or animals, excluding appliances, machinery, and equipment; or products, other than appliances, machinery, or equipment, used for the purpose of exerting pharmacological effects upon the structure or functions of humans or animals. The OTC product may include formulations for external application and personal hygiene products.

Although not particularly limited thereto, the skin external agent may be prepared specifically as an ointment, a lotion, a spray, a patch, a cream, a powder, a suspension, a gel agent, or a form of gel. The personal hygiene product may be, but is not particularly limited to, specifically a soap, a cosmetic, a wet tissue, a tissue, a shampoo, a skin cream, a face cream, a toothpaste, a lipstick, a perfume, a makeup base, a foundation, a blusher, a mascara, an eye shadow, a sunscreen lotion, a hair care product, an air freshener gel, or a wash gel.

Also, the OTC composition of the present invention may be a disinfectant cleaner, shower foam, ointment, wet tissue, coating agent, or the like, but is not limited thereto, and formulating methods, dosages, methods of use, components, and the like of the OTC composition may be appropriately selected from conventional techniques known in the art.

The OTC composition of the present invention may further include a pharmaceutically acceptable carrier, excipient, or diluent in addition to the above-described components. The pharmaceutically acceptable carrier, excipient, or diluent is not limited as long as it does not adversely affect the effects of the present invention and may include a filler, an extender, a binder, a humectant, a disintegrant, a surfactant, a lubricant, a sweetener, an aromatic, and a preservative.

In an embodiment of the present invention, it was confirmed that the nine compounds have effects on promoting hair growth and may be used as OTC compositions for preventing hair loss and promoting hair growth.

Another object of the present invention is to provide a functional food composition for promoting hair growth including at least one of the nine compounds, or a sitologically acceptable salt thereof as an active ingredient.

Because hair growth-promoting effects and significantly reduced immunosuppressive activity are confirmed, the composition including at least one compound selected from the nine compounds may be prepared in the form of foods for preventing or alleviating hair loss-related diseases.

As used herein, the term "functional food" is the same as food for special health use, health functional food, and health food, and refers to a food having high medicinal and medical effects which is processed to effectively exert a body-regulating function as well as to supply nutrients. The food may be prepared in various forms such as tablets, capsules, powders, granules, liquids, and pills to obtain a beneficial effect in preventing or alleviating diseases related to hair loss.

The functional food of the present invention may be prepared by way of a method commonly used in the art and by adding raw materials and ingredients which are generally added in the art during preparation. In addition, the formulation of the functional food may be prepared without limitation as long as the formulation is acceptable as a food. The functional food of the present invention may be prepared in various types of formulations. Unlike general drugs, the food composition includes a food as a raw material, and therefore, it has advantages of being free from side effects that may occur when taken for a long period of time. The food composition is also excellent in portability, and therefore, the food of the present invention may be taken as a supplement agent for enhancing hair growth-promoting effects.

Specifically, the functional food is a food prepared by adding at least one compound selected from the nine compounds to a food material such as beverages, teas, flavors, gums, confectionery, or a food prepared as a capsule, powder, or suspension, which may be used as various foods such as beverages, gum, teas, vitamin complexes, and functional foods.

The food may be prepared in formulations such as tablets, granules, powders, capsules, liquid solutions, and pills according to any known manufacturing methods, and the amount of the composition of the present invention may be adjusted according to the formulation. The other ingredients except for the at least one compound selected from the nine compounds according to the present invention, as an active ingredient, are not particularly limited, and the composition may further include various flavoring agents or natural carbohydrates as an additional ingredient.

Examples of the natural carbohydrate include typical sugars, e.g.: monosaccharides such as glucose and fructose; disaccharides such as maltose and sucrose; and polysaccharides such as dextrin and cyclodextrin, and sugar alcohols such as xylitol, sorbitol, and erythritol. In addition to the foregoing, flavoring agents including natural flavoring agents such as taumatin and stevia extracts (*e.g.*, rebaudioside A and glycyrrhizin) and synthetic flavoring agents (*e.g.*, saccharin and aspartame) may advantageously be used.

Additionally, the composition or the functional food of the present invention may include a variety of nutrients, vitamins, minerals (electrolytes), synthetic and natural flavoring agents, colorants and fillers (cheese, chocolate, *etc.*), pectic acid or salts thereof, alginic acid or salts thereof, organic acids, protective colloidal thickeners, pH modifiers, stabilizers, preservatives, glycerin, alcohols, and carbonating agents used in carbonated beverages.

In addition, the functional food of the present invention may include fruit pulp for natural fruit juice, fruit juice drinks, and vegetable drinks. These ingredients may be used alone or in combination.

When the formulation is powder, the carrier may be lactose, talc, silica, aluminum hydroxide, calcium silicate, polyamide powder, or any mixture thereof.

When the formulation of the present invention is a solution or emulsion, the carrier may be a solvent, a solubilizer, or an emulsifier, *e.g.*, water, ethanol, isopropanol, ethyl carbonate, ethyl acetate, benzyl alcohol, benzyl benzoate, propylene glycol, 1,3-butyl glycol oil, specifically cotton seed oil, peanut oil, corn seed oil, olive oil, castor oil, sesame oil, glycerol, an aliphatic ester, polyethylene glycol, or a fatty acid ester of sorbitan.

When the formulation is a suspension, the carrier may be a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth.

Another object of the present invention is to provide a cosmetic composition for promoting hair growth including at least one compound selected from the nine compounds, or a cosmetically acceptable salt thereof as an active ingredient.

As used herein, the "cosmetic composition" may be prepared in any formulation that is commercially manufactured, for example, a solution, an emulsion, a suspension, a paste, a cream, a lotion, a gel, a powder, a spray, a surfactant-containing cleaner, an oil, a soap, a liquid cleaner, a bath bomb, a foundation, a makeup base, an essence, a nourishing lotion, a foam, a pack, a softening water, a sunscreen cream, a sun oil, or the like.

When the formulation of the present invention is a solution or emulsion, the carrier may be a solvent, a solubilizer, or an emulsifier such as water, ethanol, isopropanol, ethyl carbonate, ethyl acetate, benzyl alcohol, benzyl benzoate, propylene glycol, 1,3-butyl glycol oil, glycerol, an aliphatic ester, polyethylene glycol, or a fatty acid ester of sorbitan.

When the formulation is a suspension, the carrier may be a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth.

When the formulation of the present invention is a paste, a cream, or a gel, the carrier may be animal oil, vegetable oil, wax, paraffin, starch, tragacanth, a cellulose derivative, polyethylene glycol, silicone, bentonite, silica, talc, or zinc oxide.

When the formulation of the present invention is a powder or spray, the carrier may be lactose, talc, silica, aluminum hydroxide, calcium silicate, or polyamide powder. Particularly, in the case of a spray, the formulation may further include a propellant such as a chlorofluorohydrocarbon, propane/butane, or dimethyl ether.

When the formulation of the present invention is a surfactant-containing cleaner, the carrier may be an aliphatic alcohol sulfate, aliphatic alcohol ether sulfate, sulfosuccinic acid monoester, isethionate, imidazolinium derivative, methyl taurate, sarcosinate, fatty acid amide ether sulfate, alkyl amido betaine, aliphatic alcohol, fatty acid glyceride, fatty acid diethanolamide, vegetable oil, lanolin derivative, or ethoxylated glycerol fatty acid ester.

In an embodiment of the present invention, effects of the nine compounds on promoting hair growth were confirmed, and the use of compositions including the same as cosmetic compositions for preventing hair loss and promoting hair growth was confirmed.

### Example 1: Preparation of 9-deoxo-31-O-demethyl-prolyl-FK506

Streptomyces kanamyceticus ΔfkbD-fkbM (accession number: KCTC13581BP), which is a 9-deoxo-31-*O*-demethyl-prolyl-FK506-producing strain, was constructed by inactivating *fkbD-fkbM* gene of *Streptomyces kanamyceticus,* which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD* and *fkbM* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD-fkbM* gene, a primer pair of a left-flanking fragment, FkbD-MLF/FkbD-MLR, and a pair of a right-flanking fragment, FkbD-MRF/FkbD-MRR, were designed. All PCR fragments were isolated, digested with HindIII-Xbal or Xbal-EcoRI, and cloned to the pKC1139 vector. Information on the strains, plasmids, and primers used in this example are shown in Tables 1 and 2 below.

The plasmids used for construction of the gene-deleted strain are shown in Table 1. A plasmid for removing both C9 hydroxylase and 31-*O* -methyltransferase, pΔfkbD-fkbM, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, i.e., ΔfkbD-fkbM, was selected as a phenotype of apramycin sensitivity and identified by PCR, and optionally by Southern blotting.

The constructed *fkbD-fkbM* gene-deleted strain, *Streptomyces kanamyceticus* ΔfkbD-fkbM, was deposited at the Korean Collection for Type Cultures (KCTC) of the Korea Research Institute of Bioscience and Biotechnology on July 17, 2018 (accession number: KCTC13581BP).

**Table 1**

| Information on Strains and Plasmids Used | |
|---|---|
| Strain/vector | Relevant characteristic |
| Bacterial strains | |
| *Escherichia coli* | |
| DH5*α* | Host for general cloning |
| ET12567/pUZ8002 | Donor strain for intergeneric conjugation between *E. coli* and *Streptomyces* |
| *Streptomyces* | |
| *Streptomyces kanamyceticus* | Wild-type FK506 producing strain |
| ΔfkbD-fkbM | Mutant of *S. kanamyceticus* with an in-frame deletion of fkbD-fkbM |
| ΔfkbD-fkbM,tcsB | Mutant of *S. kanamyceticus* with an in-frame deletion of fkbD-fkbM,tcsB |
| ΔfkbD-fkbM,tcsD | Mutant of *S. kanamyceticus* with an in-frame deletion of fkbD-fkbM,tcsD |
| ΔfkbD,tcsD | Mutant of *S. kanamyceticus* with an in-frame deletion of fkbD,tcsD |
| ΔfkbD,tcsB | Mutant of *S. kanamyceticus* with an in-frame deletion of fkbD,tcsB |
| ΔfkbM,tcsD | Mutant of *S. kanamyceticus* with an in-frame deletion of fkbM,tcsD |
| ΔfkbM,tcsB | Mutant of *S. kanamyceticus* with an in-frame deletion of fkbM,tcsB |
| Plasmid | |
| pKC1139 | High-copy-number temperature-sensitive *E. coli-Streptomyces* shuttle vector |
| pΔfkbD | Deletion plasmid with in-frame deletion of 51 bp internal fkbD fragment |
| pΔfkbM | Deletion plasmid with in-frame deletion of 507 bp internal fkbM fragment |
| pΔfkbD-fkbM | Deletion plasmid with in-frame deletion of 1100 bp internal fkbDM fragment |
| pΔtcsB | Deletion plasmid with in-frame deletion of 2090 bp internal tcsB fragment |
| pΔtcsD | Deletion plasmid with in-frame deletion of 1154 bp internal tcsD fragment |

**Table 2**

| Information on Primers Used | | |
|---|---|---|
| Primer | Sequence 5' to 3' (Restriction site underlined) | Restriction enzyme |
| FkbDLF | TATAAAGCTTCGGAGCCCCGGTGGACCT | HindIII |
| FkbDLR | TTAATCTAGACGTCGCCTCGTCGTCGCT | Xbal |
| FkbDRF | GTAATCTAGAGTCGGCTACTGCCTCTAC | Xbal |
| FkbDRR | GAATGAATTCCGACGAACAGCGGTTCCT | EcoRI |
| FkbMLF | TACGAAGCTTTCTGTTCGGCATCCAGCA | HindIII |
| FkbMLR | TAGCTCTAGAGTCACCCGGGAGCAGTTC | Xbal |
| FkbMRF | TATATCTAGAGACACCGAAGGCGCGCTC | Xbal |
| FkbMRR | TTAAGAATTCGAACACCGAGGCCGTCCA | EcoRI |
| FkbD-MLF | TATAAAGCTTCGGAGCCCCGGTGGACCT | HindIII |
| FkbD-MLR | TTAATCTAGACGTCGCCTCGTCGTCGCT | Xbal |
| FkbD-MRF | TATATCTAGAGACACCGAAGGCGCGCTC | Xbal |
| FkbD-MRR | TTAAGAATTCGAACACCGAGGCCGTCCA | EcoRI |
| TcsBLF | GACAAGCTTATGCTGGCGGTGAAGGCG | HindIII |
| TcsBLR | CCGTCTAGACCAGAAGGAATCGAGCCGGAA | Xbal |
| TcsBRF | CAGTCTAGAGTGATCCGTGCCCTGCACTCC | Xbal |
| TcsBRR | GCCGAATTCGATGACGATGTCCGGGTCG | EcoRI |
| TcsDLF | GCTAAGCTTCTCAGGCGTCTGCGGATGC | HidIII |
| TcsDLR | ATCGGATCCTTCGCTCACCGGGGCTGCC | BamHI |
| TcsDRF | AGCAGATCTGGCATGTTCTGGTCAGTCC | BgII |
| TcsDRR | GTCGAATTCCATGCCACGAACGGGTCGA | EcoRI |

9-Deoxo-31-*O*-demethyl-prolyl-FK506 was prepared by culturing the constructed production strain, *Streptomyces kanamyceticus* ΔfkbD-fkbM (accession number: KCTC13581BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-31-*O*-demethyl-prolyl-FK506 was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was reconstituted in water, ethyl acetate was added in twice the volume of the water and shaken for the extraction. The mixture was left until aqueous and organic layers were separated. After the layers were separated, an upper layer, *i.e.*, an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-31-O-demethyl-prolyl-FK506 was confirmed. Aliquot 3 obtained as described above was concentrated using a rotary evaporator and ultimately purified by HPLC.

The concentrate was lyophilized to obtain 9-deoxo-31-*O*-demethyl-prolyl-FK506 represented by Formula 1 in the form of powder.

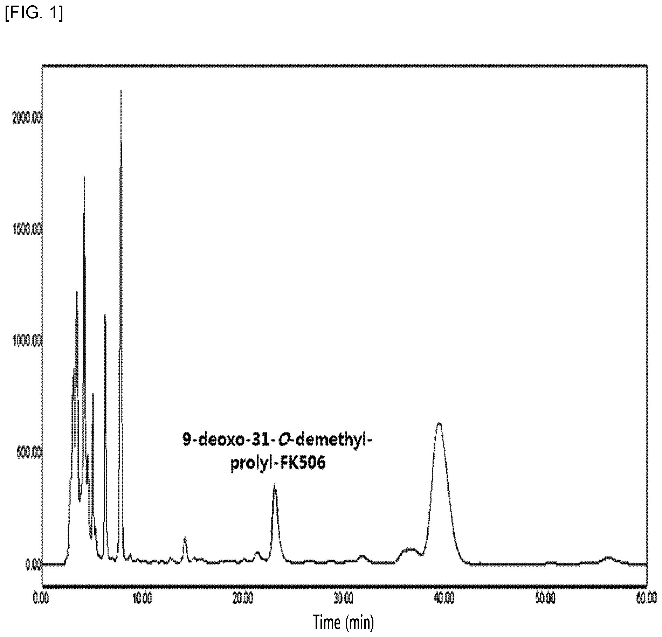

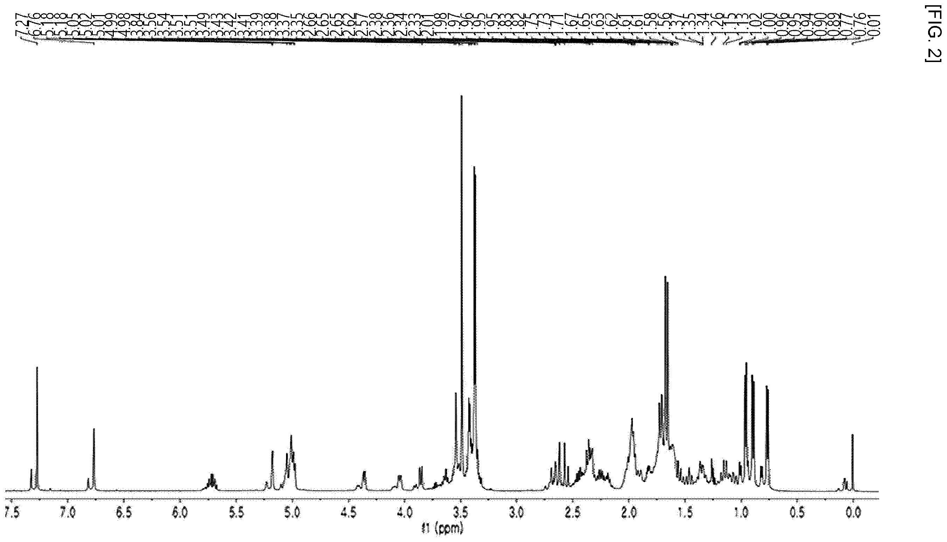

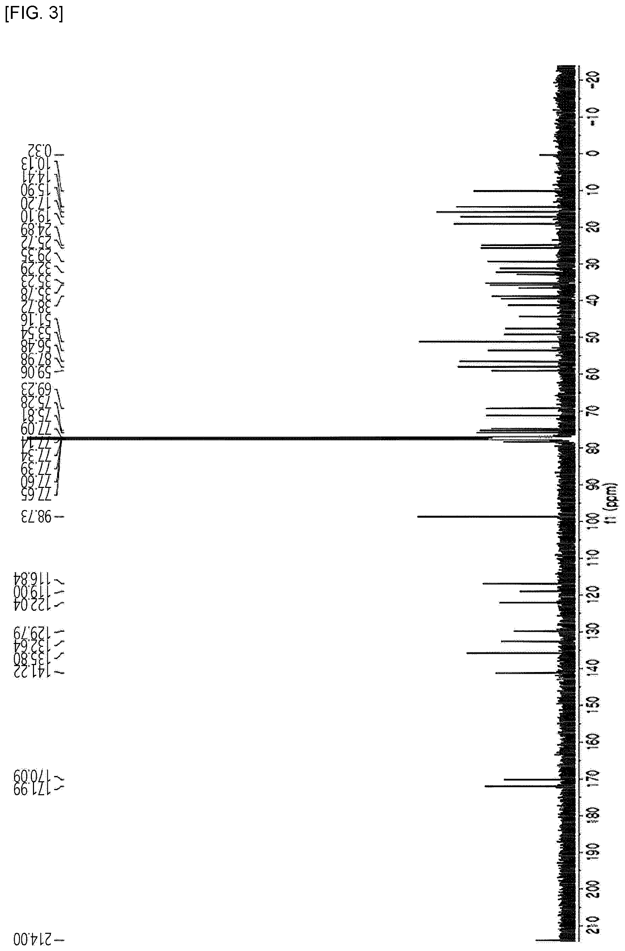

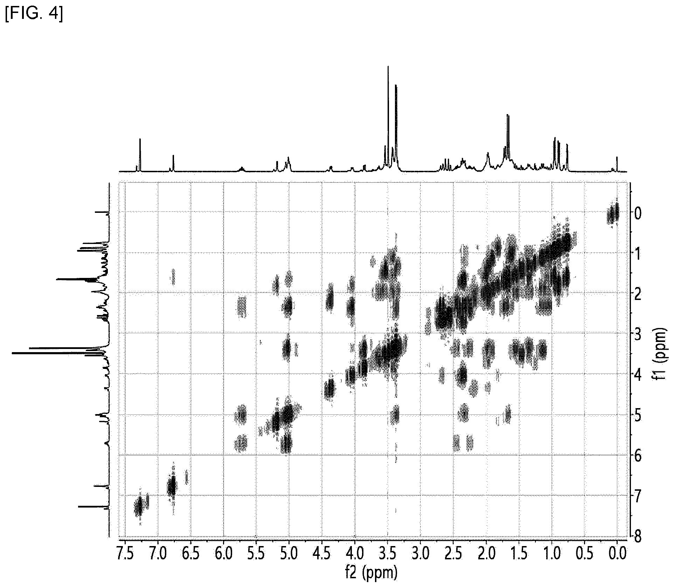

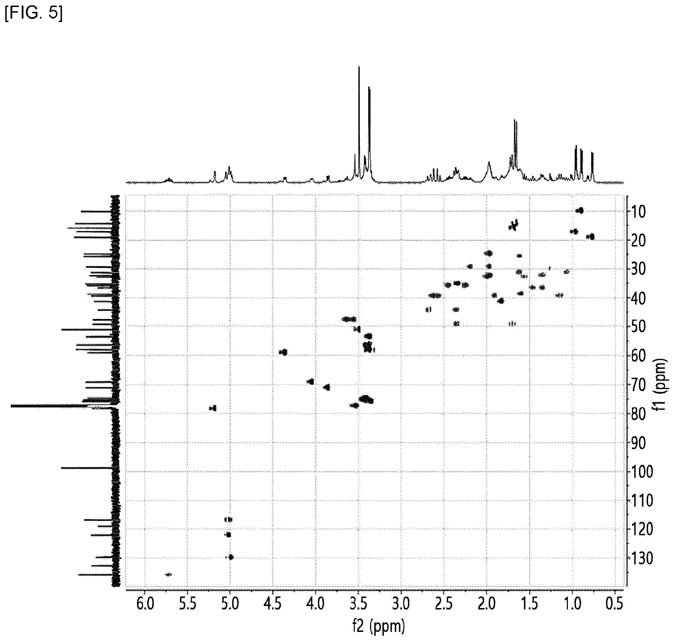

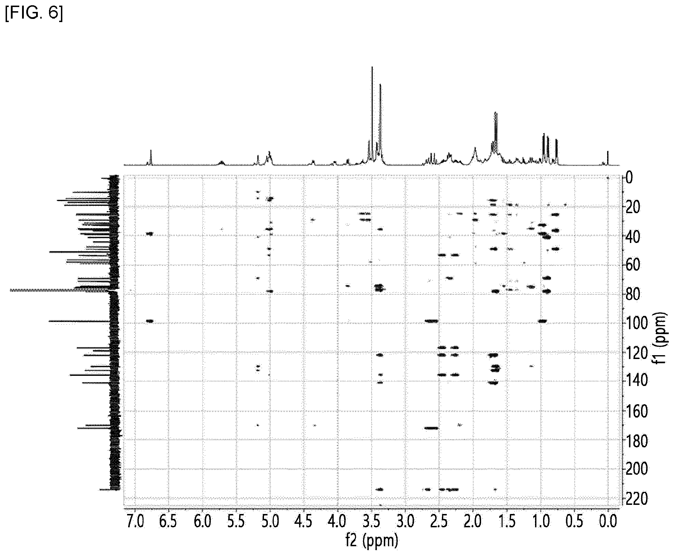

The prepared 9-deoxo-31-*O*-demethyl-prolyl-FK506 was identified as follows. Specifically, high-performance liquid chromatography analysis, mass spectrometry, and nuclear magnetic resonance analysis were conducted. Analysis results of 9-deoxo-31-O-demethyl-prolyl-FK506 are shown in Table 3 and FIGS. 1 to 6, and it was confirmed that 9-deoxo-31-*O*-demethyl-prolyl-FK506 was produced by the constructed production strain *Streptomyces kanamyceticus* ΔfkbD-fkbM based on the results.

Analysis results of 9-deoxo-31-*O*-demethyl-prolyl-FK506 (molecular formula: C₄₁H₅₇NO₁₁ and molecular weight: 749.4714) are shown in Table 3 below.

**Table 3**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-31-O-demethyl-prolyl-FK506 was 22 minutes. | | |
| Mass spectrometry | (ESI-HR-MS) Calcd. for C₄₂H₆₇NNaO₁₁⁺: 784.4606, found: *m*/*z* 784.4611 | | |
| Nuclear magnetic resonance analysis | No. | carbon (ppm) | proton (ppm) |
| | 1 | 170.09 | |
| | 2 | 59.06 | 4.36 (1H, dd, *J*=10.5 Hz, 3.5 Hz) |
| | 3 | 29.35 | 1.97 (1H, m), 2.19 (1H, m) |
| | 4 | 24.89 | 1.96 (1H, m), 1.98 (1H, m) |
| | 5 | | |
| | 6 | 47.61 | 3.55 (1H, m), 3.64 (1H, m) |
| | 7 | 171.99 | |
| | 8 | 39.34 | 2.56 (1H, d, *J=15.0* Hz), 2.63 (1H, d, *J=15.0* Hz) |
| | 9 | 98.73 | |
| | 10 | 38.72 | 1.59 (1H, m) |
| | 11 | 32.86 | 1.56 (1H, m), 1.99 (1H, m) |
| | 12 | 74.73 | 3.43 (1H, m) |
| | 13 | 71.14 | 3.85 (1H, dd, *J*=10.0 Hz, 5.0 Hz) |
| | 14 | 77.39 | 3.54 (1H, m) |
| | 15 | 36.57 | 1.34 (1H, m), 1.47 (1H, m) |
| | 16 | 25.72 | 1.61 (1H, m) |
| | 17 | 49.17 | 1.70 (1H, m), 2.35 (1H, m) |
| | 18 | 141.22 | |
| | 19 | 122.04 | 4.99 (1H, d, *J*=5.0 Hz) |
| | 20 | 53.54 | 3.38 (1H, d, *J*=5.0 Hz) |
| | 21 | 214.00 | |
| | 22 | 44.25 | 2.35 (1H, m), 2.67 (1H, m) |
| | 23 | 69.23 | 4.04 (1H, br d, *J*=10.0 Hz) |
| | 24 | 41.26 | 1.83 (1H, dd, *J*=10.0 Hz, 2.0 Hz) |
| | 25 | 78.28 | 5.18 (1H, d, *J*=2.0 Hz) |
| | 26 | 132.64 | |
| | 27 | 129.79 | 5.01 (1H, d, *J*=2.0 Hz) |
| | 28 | 35.23 | 2.35 (1H, m) |
| | 29 | 39.39 | 1.14 (1H, m), 1.90 (1H, m) |
| | 30 | 75.81 | 3.35 (1H, m) |
| | 31 | 75.28 | 3.44 (1H, m) |
| | 32 | 32.29 | 1.36 (1H, m), 1.98 (1H, m) |
| | 33 | 31.22 | 1.06 (1H, m), 1.61 (1H, m) |
| | 34 | 35.78 | 2.25 (1H, m), 2.45 (1H, m) |
| | 35 | 135.80 | 5.72 (1H, m) |
| | 36 | 116.84 | 5.00 (1H, dd, *J*=15.0 Hz, 10.0 Hz), 5.03 (1H, dd, *J*=15.0 Hz, 5.0 Hz) |
| | 37 | 17.20 | 0.96 (3H, d, *J*=5 Hz) |
| | 38 | 19.10 | 0.77 (3H, d, *J*=5 Hz) |
| | 39 | 15.90 | 1.67 (3H, s) |
| | 40 | 10.13 | 0.90 (3H, d, *J*=5 Hz) |
| | 41 | 14.41 | 1.65 (3H, s) |
| | 42 | 56.48 | 3.38 (3H, s) |
| | 43 | 57.98 | 3.37 (3H, s) |

Based on ¹H-NMR and ¹³C-NMR results, a ketone carbon (δ_{C} 214.00), two carbonyl carbons (δ_{C} 171.99 and 170.09), an exomethylene backbone (δ_{C} 116.84 and 135.80), and 2 olefin backbones (δ_{C} 141.22 and 122.04; and δ_{C} 132.64 and 129.79) were confirmed as characteristic functional groups, and a dioxygenated quaternary carbon (δ_{C} 98.73), 7 oxygenated methine carbons (δ_{C} 78.28, 77.39, 75.28, 75.81, 74.73, 71.14, and 69.23), 2 methoxy carbons (δ_{C} 57.98 and 56.48), and 5 methyl carbons (δ_{C} 19.10, 17.20, 15.90, 14.41, and 10.13) were observed. The obtained compound was confirmed as an FK506 derivative consisting of 42 carbon atoms. In order to accurately identify the structure of the compound, 2D-NMR was confirmed. As a result of identifying proton coupling using gCOSY, it was confirmed that the compound is not FK506 having a pipecolyl backbone but a compound including a prolyl backbone without a CH₂ functional group, based on couplings of H-2 to H-4. Based on gHMBC data, it was confirmed that C-9 of the compound was not a ketone but a backbone reduced to CH₂ based on correlation of H-9 (δ_{H} 2.56, 2.63) with C-8 (δ_{C} 171.99) and C-10 (δ_{C} 98.73). In addition, since two methoxy functional groups are linked to C-13 and C-15, it was confirmed that a methoxy group was not present at C-31. In conclusion, the compound was confirmed as 9-deoxo-31-O-demethyl-prolyl-FK506.

### Example 2: Preparation of 9-deoxo-36,37-dihydro-FK506

*Streptomyces kanamyceticus* ΔfkbD,tcsD (accession number: KCTC13580BP), which is a 9-deoxo-36,37-dihydro-FK506-producing strain, was constructed by inactivating *fkbD* and *tcsD* genes of *Streptomyces kanamyceticus,* which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD* gene, a primer pair of a left-flanking fragment, FkbDLF/FkbDLR, and a pair of a right-flanking fragment, FkbDRF/FkbDRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with HindIII-Xbal or Xbal-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing C9 hydroxylase, pΔfkbD, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, *i.e.*, ΔfkbD, was selected as a phenotype of apramycin sensitivity and identified by PCR, and optionally by Southern blotting.

The *tcsD* gene was deleted using the same method as that used in deletion of the *fkbD* gene by introducing pΔtcsD into the constructed *fkbD* gene-deleted *Streptomyces kanamyceticus* ΔfkbD. ΔfkbD,tcsD was selected as a phenotype of apramycin sensitivity and identified by PCR, and optionally by Southern blotting.

The constructed strain *Streptomyces kanamyceticus* ΔfkbD,tcsD from which *fkbD* and *tcsD* genes were deleted was deposited at the Korean Collection for Type Cultures (KCTC) of the Korea Research Institute of Bioscience and Biotechnology on July 17, 2018 (accession number: KCTC13580BP).

9-Deoxo-36,37-dihydro-FK506 was prepared by culturing the mutant strain, *Streptomyces kanamyceticus* ΔfkbD,tcsD (accession number: KCTC13580BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-36,37-dihydro-FK506 produced thereby was extracted by using a first extraction process.

The first extraction process was performed as follows. First, methanol was added to the culture broth in an equal volume and mixed for 30 minutes, and the microbial cells were removed by centrifugation, and then the supernatant was concentrated using a rotary evaporator. After the concentrated extract was dissolved in water, ethyl acetate was added in twice the volume and mixed. The mixture was left until layers were separated. After the layers were separated, an upper layer, i.e., an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-36,37-dihydro-FK506 was confirmed. Aliquot 3 obtained as described above was concentrated using a rotary evaporator and ultimately purified by HPLC.

The concentrate was lyophilized to obtain 9-deoxo-36,37-dihydro-FK506 represented by Formula 2 in the form of powder.

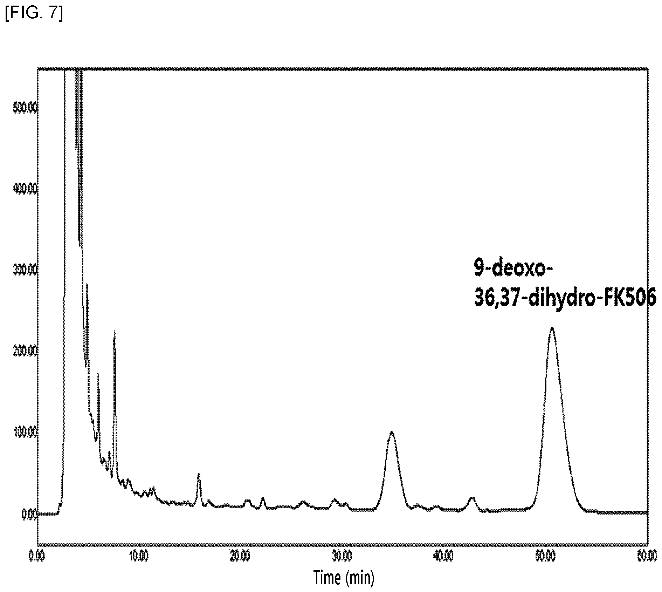

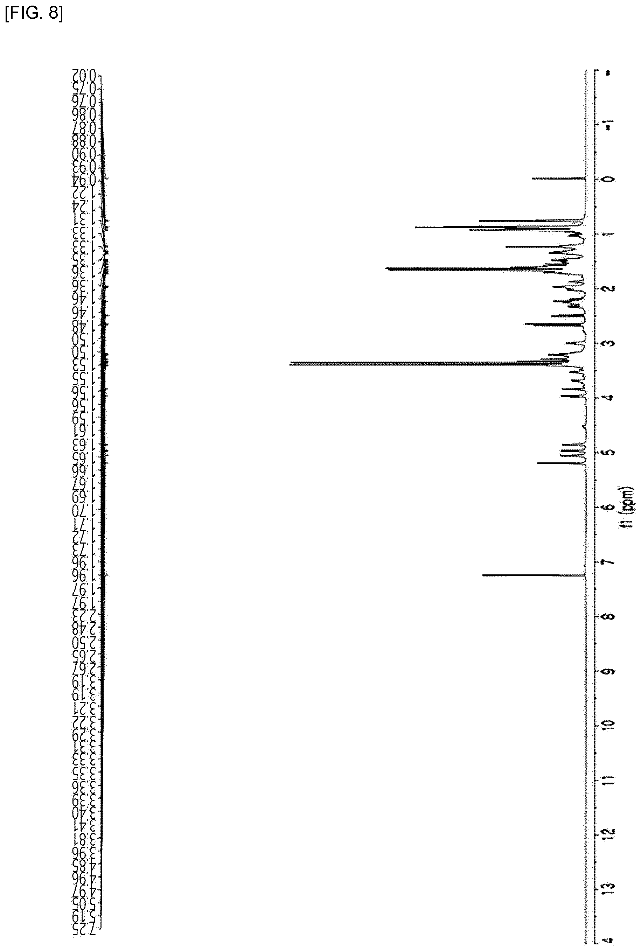

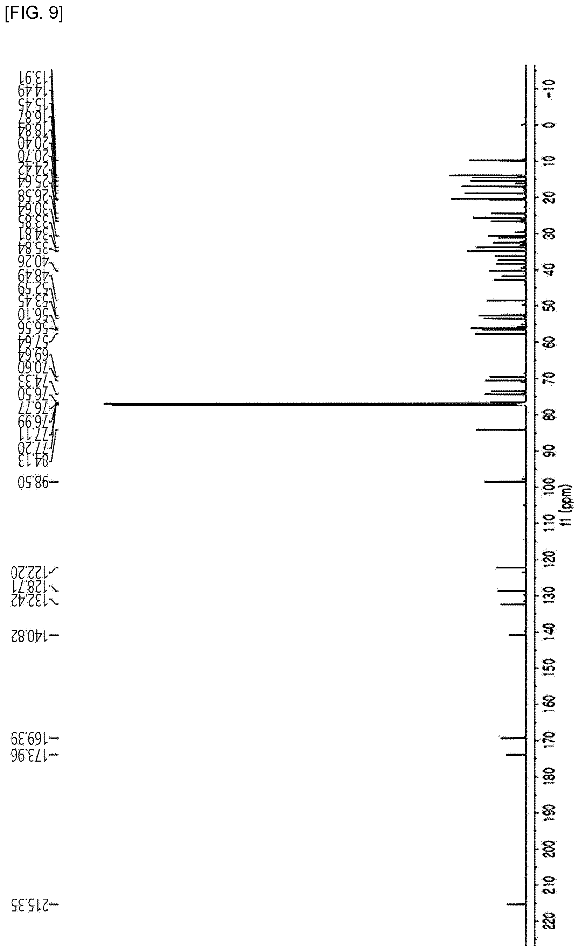

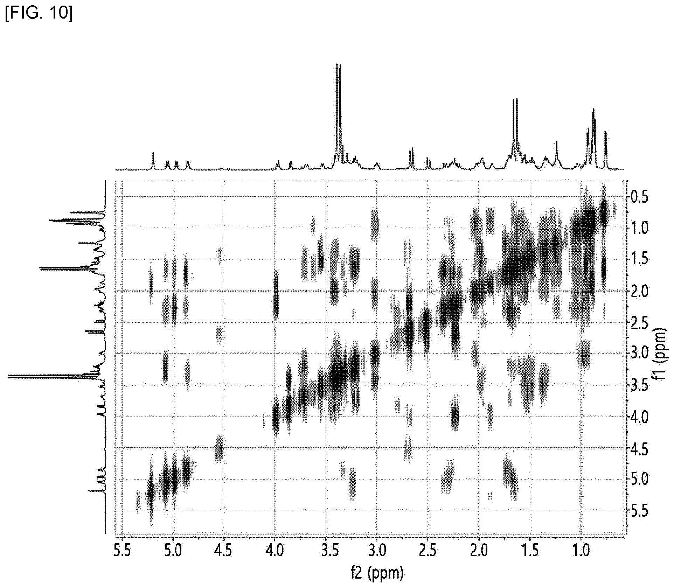

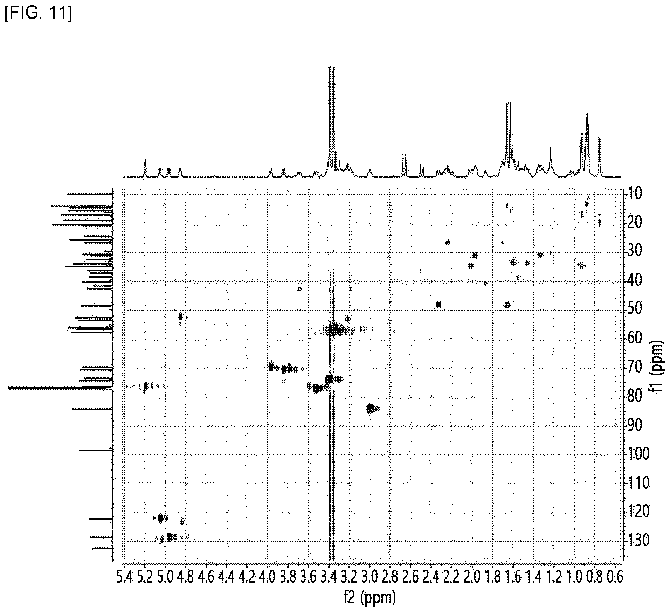

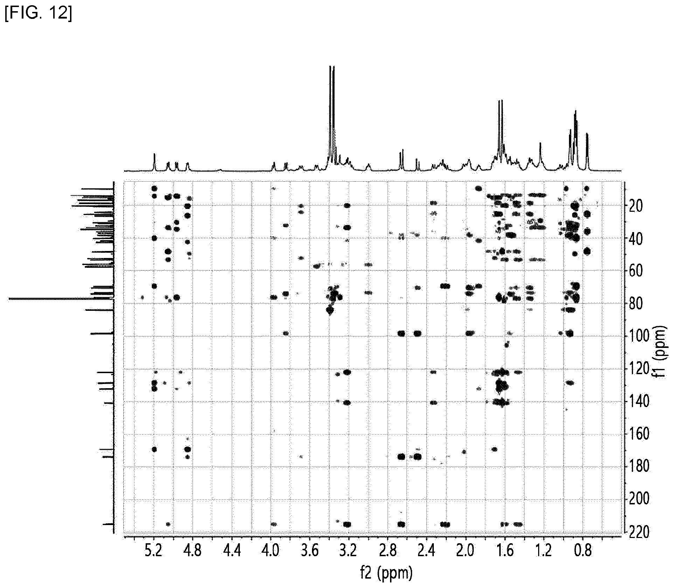

The prepared 9-deoxo-36,37-dihydro-FK506 was identified as follows. Specifically, high-performance liquid chromatography analysis, mass spectrometry, and nuclear magnetic resonance analysis were conducted. Analysis results of 9-deoxo-36,37-dihydro-FK506 are shown in Table 4 and FIGS. 7 to 12, and it was confirmed that 9-deoxo-36,37-dihydro-FK506 was produced by the constructed production strain *Streptomyces kanamyceticus* ΔfkbD,tcsD based on the results.

Analysis results of 9-deoxo-36,37-dihydro-FK506 (molecular formula: C₄₄H₇₃NO₁₁ and molecular weight: 792.06) are shown in Table 4 below.

**Table 4**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-36,37-dihydro-FK506 was 51 minutes. | | |
| Mass spectrometry | (ESI-HR-MS) Calcd. for C₄₄H₇₃NNaO₁₁⁺: 814.5076, found: *m*/*z* 814.5083 | | |
| Nuclear magnetic resonance analysis | No. | carbon (ppm) | proton (ppm) |
| | 1 | 169.39 | |
| | 2 | 52.59 | 4.85 (1H, br d, *J*=5.0 Hz) |
| | 3 | 26.58 | 1.70 (1H, m), 2.24 (1H, m) |
| | 4 | 20.70 | 1.31 (1H, m), 1.72 (1H, m) |
| | 5 | 24.42 | 1.51 (1H, m), 1.68 (1H, m) |
| | 6 | 42.65 | 3.19 (1H, m), 3.69 (1H, m) |
| | 7 | 173.96 | |
| | 8 | 36.25 | 2.49 (1H, d, *J*=15.0 Hz), 2.66 (1H, d, *J*=15.0 Hz) |
| | 9 | 98.50 | |
| | 10 | 38.39 | 1.56 (1H, m) |
| | 11 | 31.12 | 1.60 (1H, m), 1.94 (1H, m) |
| | 12 | 74.33 | 3.40 (1H, m) |
| | 13 | 70.6 | 3.84 (1H, dd, *J*=10.0 Hz, 5.0 Hz) |
| | 14 | 77.11 | 3.53 (1H, m) |
| | 15 | 36.57 | 1.34 (1H, m), 1.47 (1H, m) |
| | 16 | 25.64 | 1.50 (1H, m) |
| | 17 | 48.49 | 1.65 (1H, m), 2.32 (1H, m) |
| | 18 | 140.82 | |
| | 19 | 122.20 | 5.05 (1H, d, *J*=5.0 Hz) |
| | 20 | 53.45 | 3.21 (1H, d, *J*=5.0 Hz) |
| | 21 | 215.35 | |
| | 22 | 41.74 | 2.21 (1H, br d, *J*=15 Hz), 2.66 (1H, br d, *J*=15 Hz) |
| | 23 | 69.64 | 3.97 (1H, m) |
| | 24 | 40.26 | 1.87 (1H, m) |
| | 25 | 76.50 | 5.19 (1H, br s) |
| | 26 | 132.42 | |
| | 27 | 128.71 | 4.97 (1H, d, *J*=5.0 Hz) |
| | 28 | 34.81 | 2.35 (1H, m) |
| | 29 | 34.84 | 0.93 (1H, m), 2.01 (1H, m) |
| | 30 | 84.13 | 3.00 (1H, m) |
| | 31 | 73.58 | 3.39 (1H, m) |
| | 32 | 30.64 | 1.34 (1H, m), 1.98 (1H, m) |
| | 33 | 31.15 | 1.06 (1H, m), 1.61 (1H, m) |
| | 34 | 33.82 | 1.42 (1H, m), 1.65 (1H, m) |
| | 35 | 20.41 | 1.21 (2H, m) |
| | 36 | 13.91 | 0.88 (3H, t, *J*=7.5 Hz) |
| | 37 | 16.87 | 0.93 (3H, d, *J*=5 Hz) |
| | 38 | 18.84 | 0.76 (3H, d, *J*=5 Hz) |
| | 39 | 15.45 | 1.63 (3H, s) |
| | 40 | 10.13 | 0.90 (3H, d, *J*=5 Hz) |
| | 41 | 13.91 | 1.66 (3H, s) |
| | 42 | 56.10 | 3.35 (3H, s) |
| | 43 | 57.64 | 3.36 (3H, s) |
| | 44 | 56.56 | 3.39 (3H, s) |

Based on ¹H-NMR, ¹³C-NMR, and gHSQC results, the obtained compound consists of a total of 44 carbon atoms, and one methoxy group (δ_{H} 3.39 and δ_{C} 56.56) and one CH₂ group (δ_{H} 2.24, δ_{H} 1.70, and δ_{C} 20.70) were additionally observed, the exomethylene functional group was not observed, and a triplet coupling of a methyl group (δ_{H} 0.88 and δ_{C} 13.91) and a CH₂ group (δ_{H} 1.21 and δ_{C} 20.41) was observed. In order to accurately identify the structure, 2D-NMR was performed. As a result of identifying proton coupling by gCOSY, it was confirmed that the compound is FK506 including a pipecolyl backbone, and one CH₂ functional group (δ_{H} 2.24, δ_{H} 1.70, and δ_{C} 20.70) is present on the pipecolyl backbone based on couplings of H-2 to H-5. Based on gHMBC, it was confirmed that three methoxy hydrogens (δ_{H} 3.39, 3.36, and 3.35) were correlated with C-31 (δ_{C} 77.11), C-15 (δ_{C} 77.11), and C-13 (δ_{C} 74.33) respectively, and the methyl group (δ_{H} 0.88 and δ_{C} 13.91) and the CH₂ group (δ_{H} 1.21 and δ_{C} 20.41) observed in a triplet coupled state were correlated with C-21/33. Therefore, as a compound produced by the *tcsD* gene-deleted strain, 9-deoxo-36,37-dihydro-FK506 was determined to have a structure in the form where a double bond present between C-36/37 was reduced.

### Example 3: Preparation of 31-O-demethyl-36,37-dihydro-FK506

*Streptomyces kanamyceticus* ΔfkbM,tcsD (accession number: KCTC13584BP), which is a 31-O-demethyl-36,37-dihydro-FK506-producing strain, was constructed by inactivating *fkbM* and *tcsD* genes of *Streptomyces kanamyceticus*, which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbM* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbM* gene, a primer pair of a left-flanking fragment, FkbMLF/FkbMLR, and a pair of a right-flanking fragment, FkbMRF/FkbMRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with HindIII-Xbal or Xbal-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing 31-O-methyltransferase, pΔfkbM, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, i.e., ΔfkbM, was selected as a phenotype of apramycin sensitivity and identified by PCR, and optionally by Southern blotting.

The *tcsD* gene was deleted using the same method as that used in deletion of the *fkbM* gene by introducing pΔtcsD into the constructed *fkbM* gene-deleted *Streptomyces kanamyceticus* ΔfkbM. ΔfkbM,tcsD was selected as a phenotype of apramycin sensitivity and identified by PCR, and optionally by Southern blotting.

The constructed strain *Streptomyces kanamyceticus* ΔfkbM,tcsD from which *fkbM* and *tcsD* genes were deleted was deposited at the Korean Collection for Type Cultures (KCTC) of the Korea Research Institute of Bioscience and Biotechnology on July 17, 2018 (accession number: KCTC13584BP).

31-O-demethyl-36,37-dihydro-FK506 was prepared by culturing the constructed production strain, *Streptomyces kanamyceticus* ΔfkbM,tcsD (accession number: KCTC13584BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 31-*O*-demethyl-36,37-dihydro-FK506 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e.*, an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 31-*O*-demethyl-36,37-dihydro-FK506 was confirmed. Aliquot 3 obtained as described above was concentrated using a rotary evaporator and ultimately purified by HPLC.

The concentrate was lyophilized to obtain 31-*O*-demethyl-36,37-dihydro-FK506 represented by Formula 3 in the form of powder.

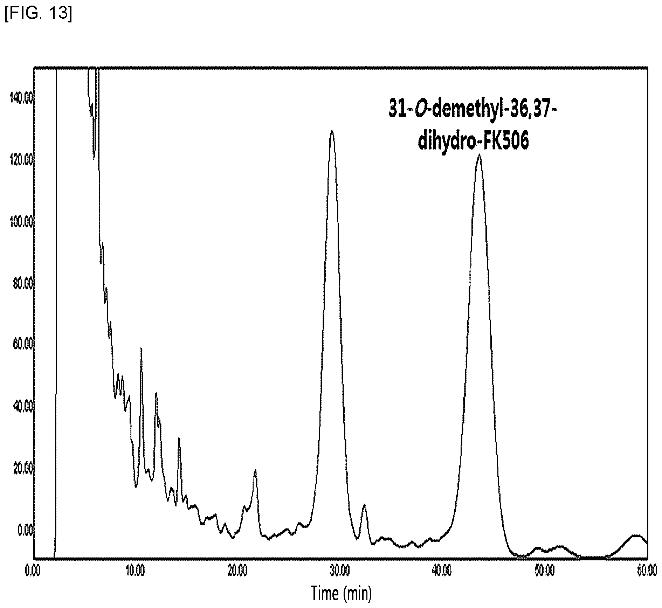

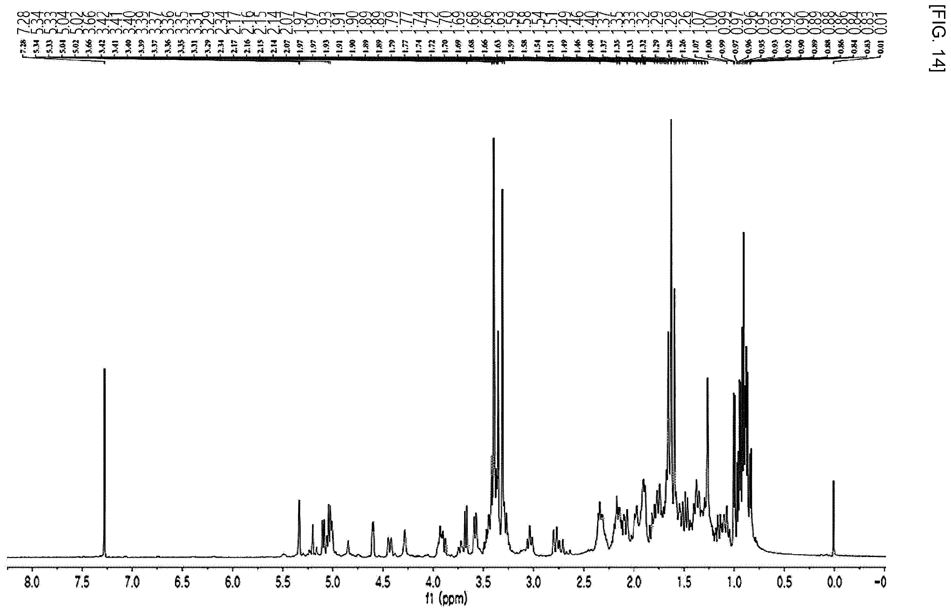

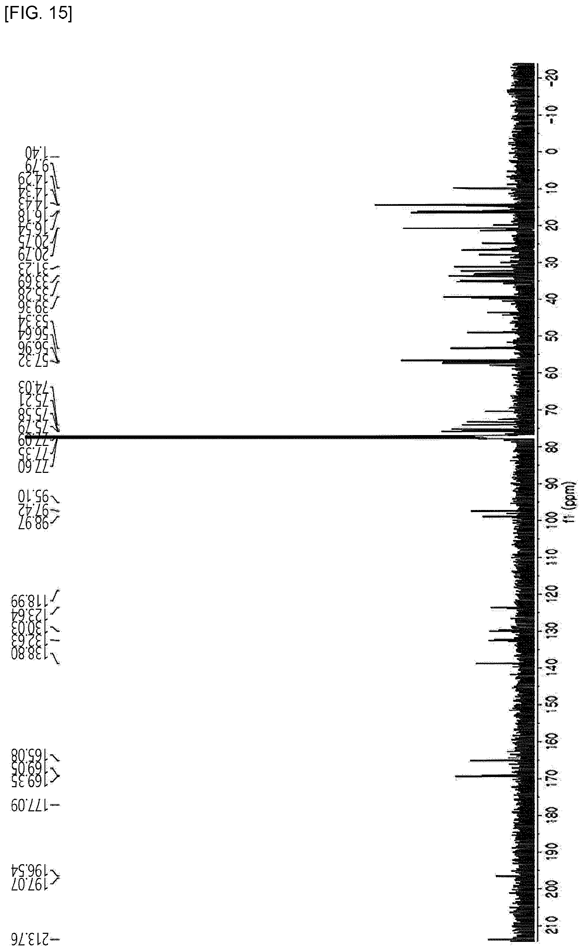

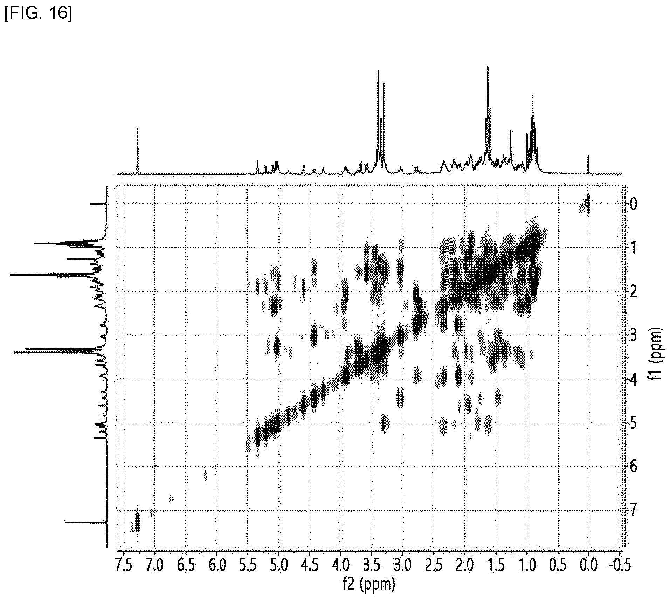

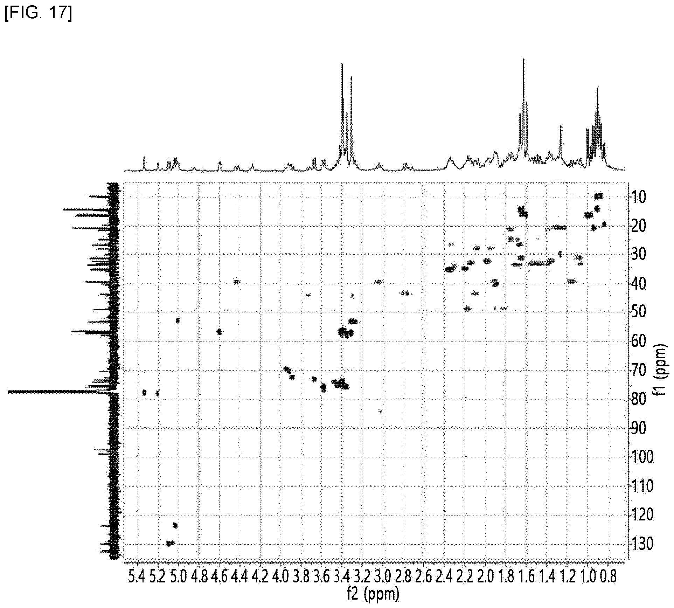

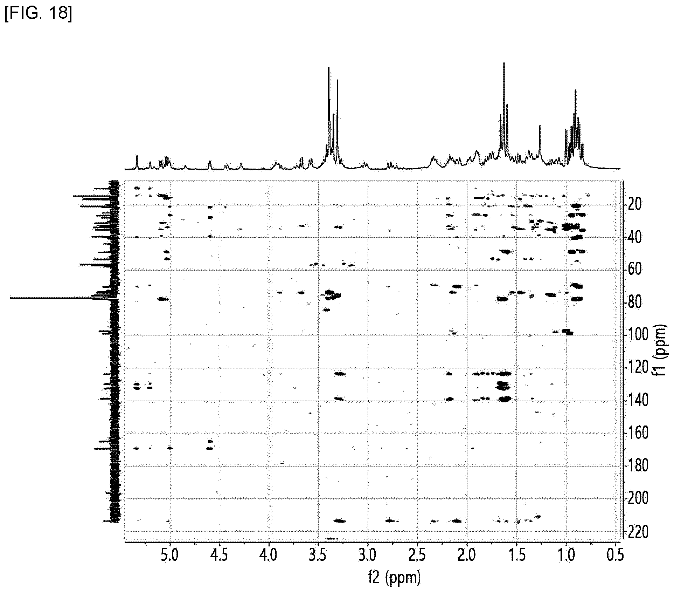

The prepared 31-*O*-demethyl-36,37-dihydro-FK506 was identified as follows. Specifically, high-performance liquid chromatography analysis, mass spectrometry, and nuclear magnetic resonance analysis were conducted. Analysis results of 31-*O*-demethyl-36,37-dihydro-FK506 are shown in Table 5 and FIGS. 13 to 18, and it was confirmed that 31-*O*-demethyl-36,37-dihydro-FK506 was produced by the constructed production strain *Streptomyces kanamyceticus* ΔfkbM,tcsD based on the results.

Analysis results of 31-*O*-demethyl-36,37-dihydro-FK506 (molecular formula: C₄₃H₆₉NO₁₂ and molecular weight: 792.02) are shown in Table 5 below.

**Table 5**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 31-O-demethyl-36,37-dihydro-FK506 was 43 minutes. | | |
| Mass spectrometry | (ESI-HR-MS) Calcd. for C₄₃H₆₉NNaO₁₂⁺: 814.4712, found: *m*/*z* 814.4715 | | |
| Nuclear magnetic resonance analysis | No. | carbon (ppm) | proton (ppm) |
| | 1 | 169.35 | |
| | 2 | 56.64 | 4.58 (1H, br d, *J*=5.0 Hz) |
| | 3 | 27.92 | 2.05 (1H, m), 1.90 (1H, m) |
| | 4 | 21.42 | 1.38 (1H, m), 1.74 (1H, m) |
| | 5 | 24.80 | 1.51 (1H, m), 1.68 (1H, m) |
| | 6 | 39.58 | 3.03 (1H, m), 4.44 (1H, m) |
| | 7 | 165.08 | |
| | 8 | 196.54 | |
| | 9 | 97.42 | |
| | 10 | 35.28 | 2.32 (1H, m) |
| | 11 | 33.01 | 1.52 (1H, m), 2.16 (1H, m) |
| | 12 | 74.03 | 3.39 (1H, m) |
| | 13 | 73.22 | 3.68 (1H, dd, *J*=10.0 Hz, 5.0 Hz) |
| | 14 | 75.21 | 3.39 (1H, m) |
| | 15 | 35.79 | 1.35 (1H, m), 1.56 (1H, m) |
| | 16 | 26.67 | 1.66 (1H, m) |
| | 17 | 49.02 | 1.80 (1H, m), 2.17 (1H, m) |
| | 18 | 138.80 | |
| | 19 | 123.64 | 5.03 (1H, d, *J*=5.0 Hz) |
| | 20 | 53.34 | 3.28 (1H, d, *J*=5.0 Hz) |
| | 21 | 213.76 | |
| | 22 | 43.64 | 2.10 (1H, br d, *J*=15 Hz), 2.77 (1H, br d, *J*=15 Hz) |
| | 23 | 70.36 | 3.92 (1H, m) |
| | 24 | 39.97 | 1.89 (1H, m) |
| | 25 | 77.82 | 5.33 (1H, br s) |
| | 26 | 132.63 | |
| | 27 | 130.03 | 5.09 (1H, d, *J*=5.0 Hz) |
| | 28 | 34.93 | 2.18 (1H, m) |
| | 29 | 39.36 | 1.14 (1H, m), 1.91 (1H, m) |
| | 30 | 75.79 | 3.59 (1H, m) |
| | 31 | 75.58 | 3.36 (1H, m) |
| | 32 | 32.36 | 1.35 (1H, m), 1.98 (1H, m) |
| | 33 | 31.23 | 1.06 (1H, m), 1.61 (1H, m) |
| | 34 | 33.34 | 1.42 (1H, m), 1.66 (1H, m) |
| | 35 | 20.76 | 1.27 (2H, m) |
| | 36 | 14.37 | 0.90 (3H, t, *J*=7.5 Hz) |
| | 37 | 16.54 | 1.00 (3H, d, *J*=5 Hz) |
| | 38 | 20.75 | 0.93 (3H, d, *J*=5 Hz) |
| | 39 | 16.18 | 1.59 (3H, s) |
| | 40 | 9.79 | 0.87 (3H, d, *J*=5 Hz) |
| | 41 | 14.43 | 1.62 (3H, s) |
| | 42 | 56.64 | 3.40 (3H, s) |
| | 43 | 57.32 | 3.30 (3H, s) |

Based on ¹H-NMR, ¹³C-NMR, and gHSQC results, the obtained compound consists of a total of 43 carbon atoms, and since a ketone carbon (δ_{C} 196.54) was additionally observed, and two methoxy groups were observed, it may be inferred that the compound was derived from 31-O-demethyl generated by the ΔfkbM gene. Based on gHMBC, it was confirmed that two methoxy hydrogens (δ_{H} 3.40 and 3.30) were correlated with C-15 (δ_{C} 75.21) and C-13 (δ_{C} 74.03), respectively. As a compound produced by the *tcsD* gene-deleted strain, 31-O-demethyl-35,37-dihydro-FK506 was determined to have a structure in which a double bond present between C-36/37 was reduced.

### Example 4: Preparation of 9-deoxo-31-O-demethyl-36,37-dihydro-FK506

*Streptomyces kanamyceticus* ΔfkbD-fkbM,tcsD (accession number: KCTC13585BP), which is a 9-deoxo-31-*O*-demethyl-36,37-dihydro-FK506-producing strain, was constructed by inactivating *fkbD-fkbM* and *tcsD* genes of *Streptomyces kanamyceticus*, which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD-fkbM* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD-fkbM* gene, a primer pair of a left-flanking fragment, FkbD-MLF/FkbD-MLR, and a pair of a right-flanking fragment, FkbD-MRF/FkbD-MRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with HindIII-Xbal or Xbal-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing both C9 hydroxylase and 31-*O*-methyltransferase, pΔfkbD-fkbM, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, i.e., ΔfkbD-fkbM, was selected as a phenotype of apramycin sensitivity and identified by PCR, and optionally by Southern blotting.

The *tcsD* gene was deleted using the same method as that used in deletion of the *fkbD-fkbM* gene by introducing pΔtcsD into the constructed *fkbD-fkbM* gene-deleted *Streptomyces kanamyceticus* ΔfkbD-fkbM. ΔfkbD-fkbM,tcsD was selected as a phenotype of apramycin sensitivity and identified by PCR, and optionally by Southern blotting.

The constructed strain *Streptomyces kanamyceticus* ΔfkbD-fkbM,tcsD from which *fkbD-fkbM* and *tcsD* genes were deleted was deposited at the Korean Collection for Type Cultures (KCTC) of the Korea Research Institute of Bioscience and Biotechnology on July 17, 2018 (accession number: KCTC13585BP).

9-Deoxo-31-*O*-demethyl-36,37-dihydro-FK506 was prepared by culturing the constructed production strain, *Streptomyces kanamyceticus* ΔfkbD-fkbM,tcsD (accession number: KCTC13585BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-31-*O*-demethyl-36,37-dihydro-FK506 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e.*, an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-31-O-demethyl-36,37-dihydro-FK506 was confirmed. Aliquot 3 obtained as described above was concentrated using a rotary evaporator and ultimately purified by HPLC.

The concentrate was lyophilized to obtain 9-deoxo-31-O-demethyl-36,37-dihydro-FK506 represented by Formula 4 in the form of powder.

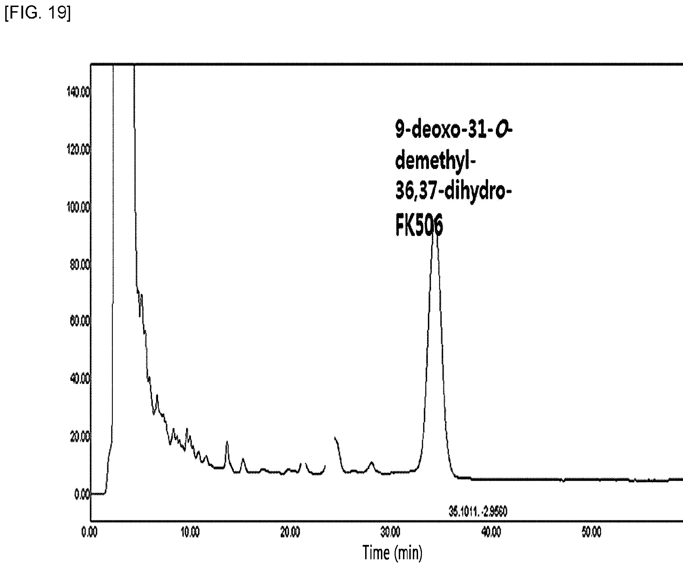

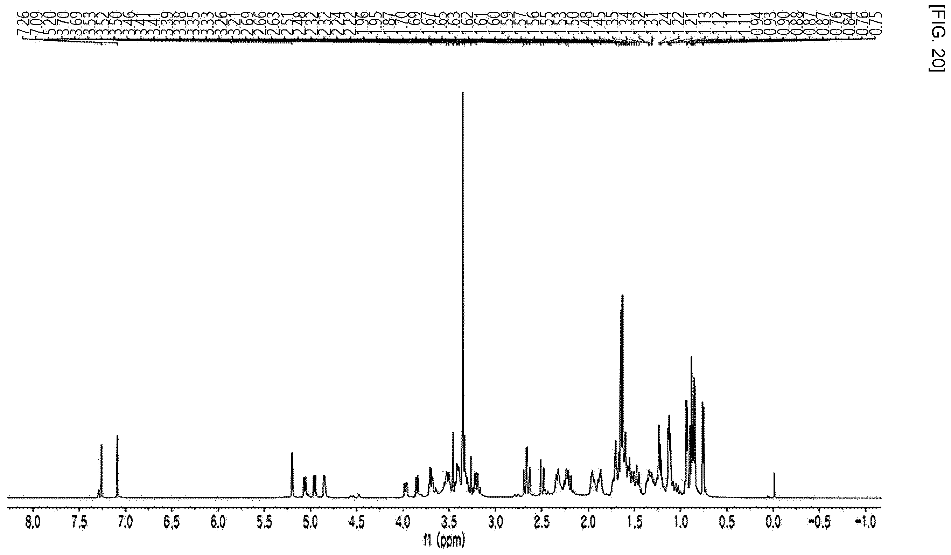

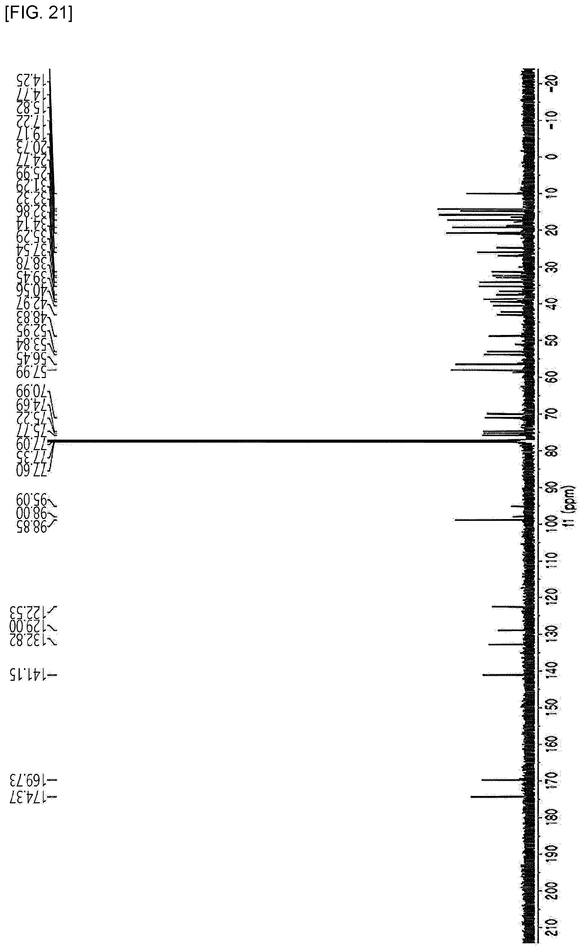

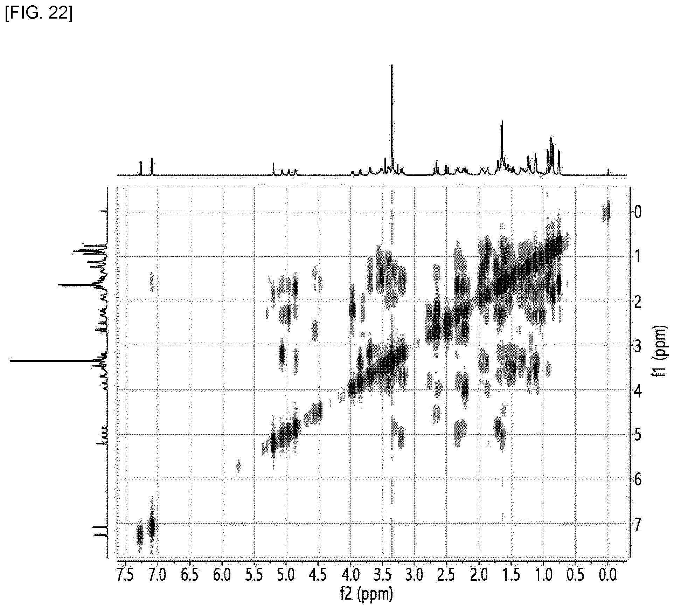

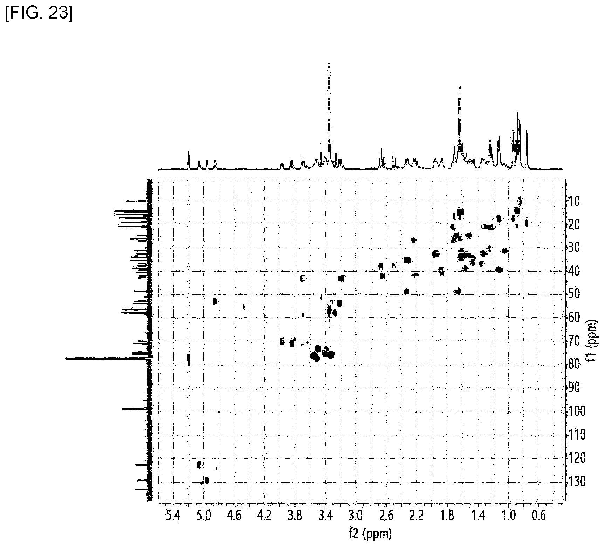

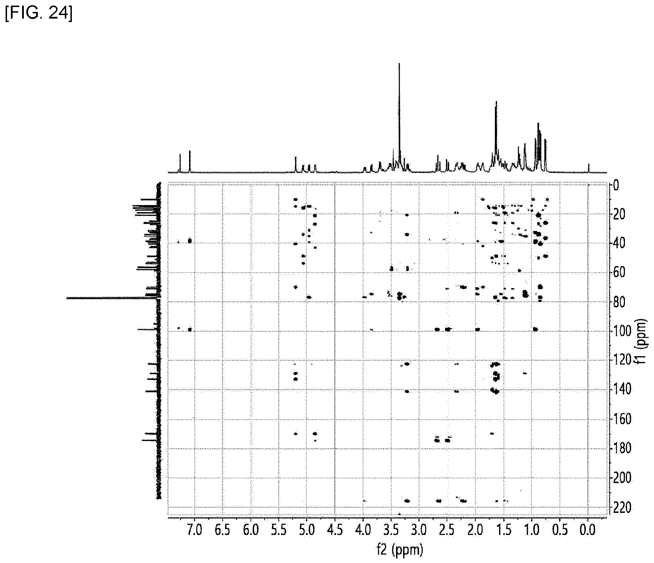

The prepared 9-deoxo-31-*O*-demethyl-36,37-dihydro-FK506 was identified as follows. Specifically, high-performance liquid chromatography analysis, mass spectrometry, and nuclear magnetic resonance analysis were conducted. Analysis results of 9-deoxo-31-O-demethyl-36,37-dihydro-FK506 are shown in Table 6 and FIGS. 19 to 24, and it was confirmed that 9-deoxo-31-*O*-demethyl-36,37-dihydro-FK506 was produced by the constructed production strain *Streptomyces kanamyceticus* ΔfkbD-fkbM,tcsD based on the results.

Analysis results of 9-deoxo-31-*O*-demethyl-36,37-dihydro-FK506 (molecular formula: C₄₃H₇₁ NO₁₁ and molecular weight: 778.04) are shown in Table 6 below.

**Table 6**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-31-O-demethyl-36,37-dihydro-FK506 was 34 minutes. | | |
| Mass spectrometry | (ESI-HR-MS) Calcd. for C₄₃H₇₁NNaO₁₁⁺: 800.4919, found: *m*/*z* 800.4924 | | |
| Nuclear magnetic resonance analysis | No. | carbon (ppm) | proton (ppm) |
| | 1 | 169.73 | |
| | 2 | 52.59 | 4.85 (1H, br d, *J*=5.0 Hz) |
| | 3 | 26.94 | 1.70 (1H, m), 2.23 (1H, m) |
| | 4 | 21.03 | 1.32 (1H, m), 1.72 (1H, m) |
| | 5 | 24.77 | 1.52 (1H, m), 1.68 (1H, m) |
| | 6 | 42.97 | 3.19 (1H, m), 3.70 (1H, m) |
| | 7 | 174.37 | |
| | 8 | 37.54 | 2.49 (1H, d, *J*=15.0 Hz), 2.67 (1H, d, *J*=15.0 Hz) |
| | 9 | 98.85 | |
| | 10 | 38.78 | 1.56 (1H, m) |
| | 11 | 32.86 | 1.60 (1H, m), 1.94 (1H, m) |
| | 12 | 74.69 | 3.40 (1H, m) |
| | 13 | 70.99 | 3.84 (1H, dd, *J*=10.0, 5.0 Hz) |
| | 14 | 77.21 | 3.53 (1H, m) |
| | 15 | 36.57 | 1.34 (1H, m), 1.47 (1H, m) |
| | 16 | 26.94 | 1.63 (1H, m) |
| | 17 | 48.49 | 1.64 (1H, m), 2.33 (1H, m) |
| | 18 | 141.15 | |
| | 19 | 122.53 | 5.05 (1H, d, *J*=5.0 Hz) |
| | 20 | 53.84 | 3.20 (1H, d, *J*=5.0 Hz) |
| | 21 | 215.35 | |
| | 22 | 42.20 | 2.20 (1H, br d, *J*=15 Hz), 2.64 (1H, br d, *J*=15 Hz) |
| | 23 | 69.96 | 3.97 (1H, m) |
| | 24 | 40.05 | 1.85 (1H, m) |
| | 25 | 76.90 | 5.20 (1H, br s) |
| | 26 | 132.82 | |
| | 27 | 129 | 4.97 (1H, d, *J*=5.0 Hz) |
| | 28 | 35.29 | 2.32 (1H, m) |
| | 29 | 39.45 | 1.12 (1H, m), 1.88 (1H, m) |
| | 30 | 75.22 | 3.53 (1H, m) |
| | 31 | 75.22 | 3.40 (1H, m) |
| | 32 | 32.32 | 1.33 (1H, m), 1.95 (1H, m) |
| | 33 | 31.19 | 1.04 (1H, m), 1.59 (1H, m) |
| | 34 | 34.14 | 1.46 (1H, m), 1.62 (1H, m) |
| | 35 | 20.73 | 1.22 (2H, m) |
| | 36 | 14.25 | 0.88 (3H, t, *J*=7.5 Hz) |
| | 37 | 17.22 | 0.94 (3H, d, *J*=5 Hz) |
| | 38 | 19.17 | 0.76 (3H, d, *J*=5 Hz) |
| | 39 | 15.82 | 1.63 (3H, s) |
| | 40 | 10.07 | 0.85 (3H, d, *J*=5 Hz) |
| | 41 | 14.77 | 1.65 (3H, s) |
| | 42 | 56.45 | 3.35 (3H, s) |
| | 43 | 57.99 | 3.35 (3H, s) |

Based on ¹H-NMR, ¹³C-NMR, and gHSQC results, although the obtained compound consists of a total of 43 carbon atoms, CH₂ functional groups (δ_{H} 2.67, 2.49, and δ_{C} 37.54) were observed instead of ketone by influence of ΔfkbD as in substances 1 and 2. Based on analysis results of 2D-NMR data, the structure of the compound No. 4 was determined as 9-deoxo-31-*O*-demethyl-35,37-dihydro-FK506 produced from ΔfkbD-fkbM gene.

### Example 5: Preparation of 9-deoxo-FK520

*Streptomyces kanamyceticus* ΔfkbD,tcsB (accession number: KCTC13579BP) or *Streptomyces kanamyceticus* ΔfkbD,tcsD (accession number: KCTC13580BP), which is a 9-deoxo-FK520-producing strain, was constructed by inactivating *fkbD* and *tcsB* genes or *fkbD* and *tcsD* genes of *Streptomyces kanamyceticus*, which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD* and *tcsB* genes or *fkbD* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD* gene, a primer pair of a left-flanking fragment, FkbDLF/FkbDLR, and a pair of a right-flanking fragment, FkbDRF/FkbDRR, were designed. For deletion of the *tcsB* gene, a primer pair of a left-flanking fragment, TcsBLF/TcsBLR, and a pair of a right-flanking fragment, TcsBRF/TcsBRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with Hindlll-Xbal or Xbal-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing C9 hydroxylase, pΔfkbD, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, *i.e.*, ΔfkbD, was selected as a phenotype of apramycin sensitivity and identified by PCR, and optionally by Southern blotting.

The *tcsB* gene or the *tcsD* gene was deleted using the same method as that used in deletion of the *fkbD* gene by introducing pΔtcsB or pΔtcsD into the constructed *fkbD* gene-deleted *Streptomyces kanamyceticus* ΔfkbD. ΔfkbD,tcsB or ΔfkbD,tcsD was selected as a phenotype of apramycin sensitivity and identified by PCR, and optionally by Southern blotting.

The constructed strain *Streptomyces kanamyceticus* ΔfkbD,tcsB from which *fkbD* and *tcsB* genes were deleted was deposited at the Korean Collection for Type Cultures (KCTC) of the Korea Research Institute of Bioscience and Biotechnology on July 17, 2018 (accession number: KCTC13579BP), and the constructed strain *Streptomyces kanamyceticus* ΔfkbD,tcsD from which *fkbD* and *tcsD* genes were deleted was also deposited at the Korean Collection for Type Cultures (KCTC) of the Korea Research Institute of Bioscience and Biotechnology on July 17, 2018 (accession number: KCTC13580BP).

9-Deoxo-FK520 was prepared by culturing the constructed production strain *Streptomyces kanamyceticus* ΔfkbD,tcsB (accession number: KCTC13579BP) or *Streptomyces kanamyceticus* ΔfkbD,tcsD (accession number: KCTC13580BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-FK520 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e.*, an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-FK520 was confirmed. Aliquot 3 obtained as described above was concentrated using a rotary evaporator and ultimately purified by HPLC.

The concentrate was lyophilized to obtain 9-deoxo-FK520 represented by Formula 5 in the form of powder.

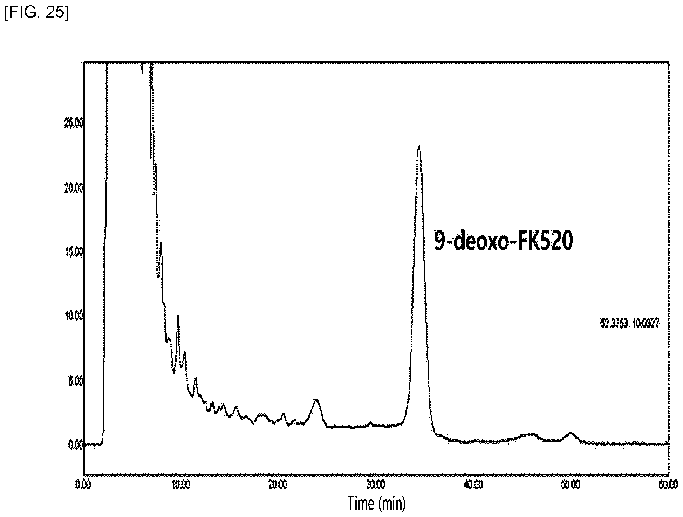

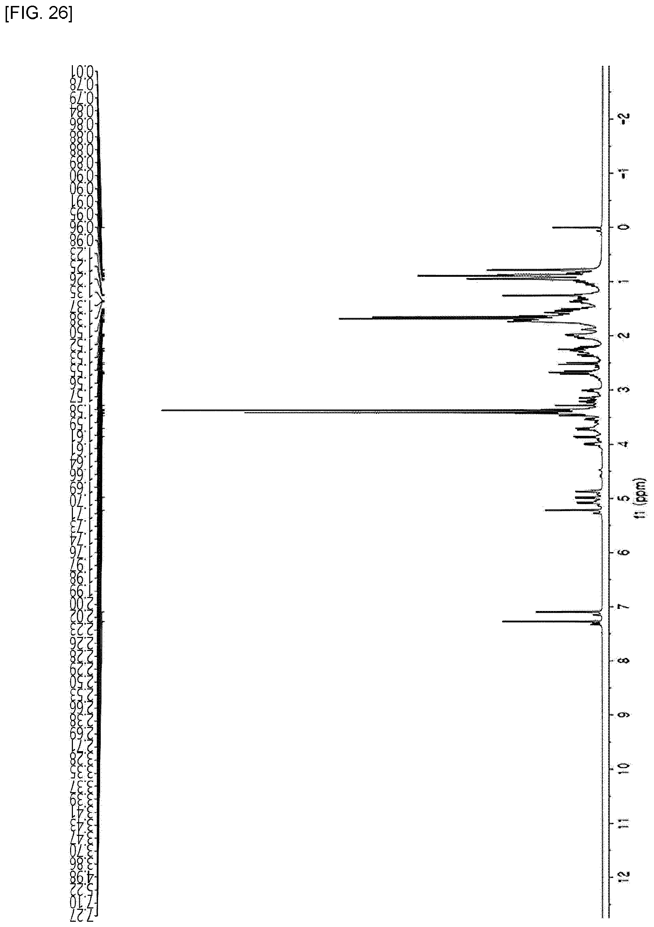

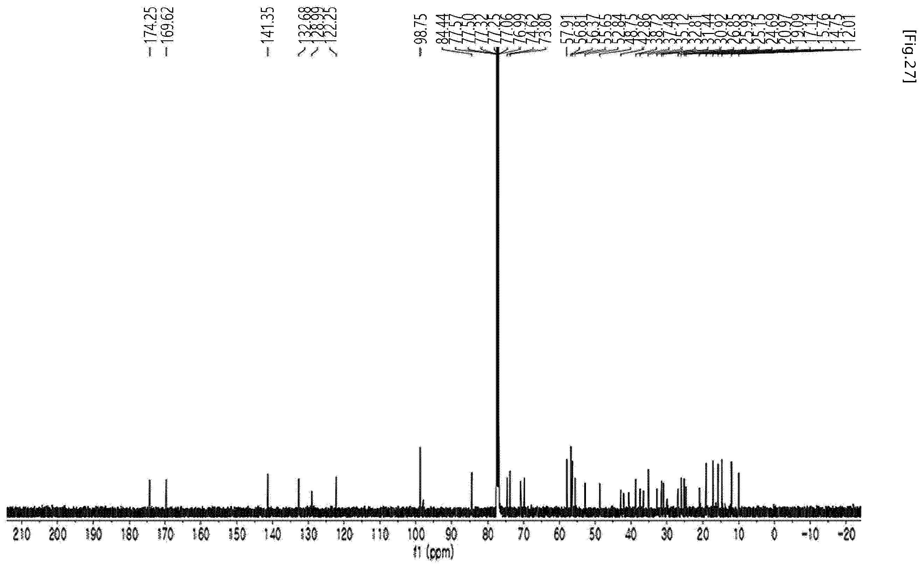

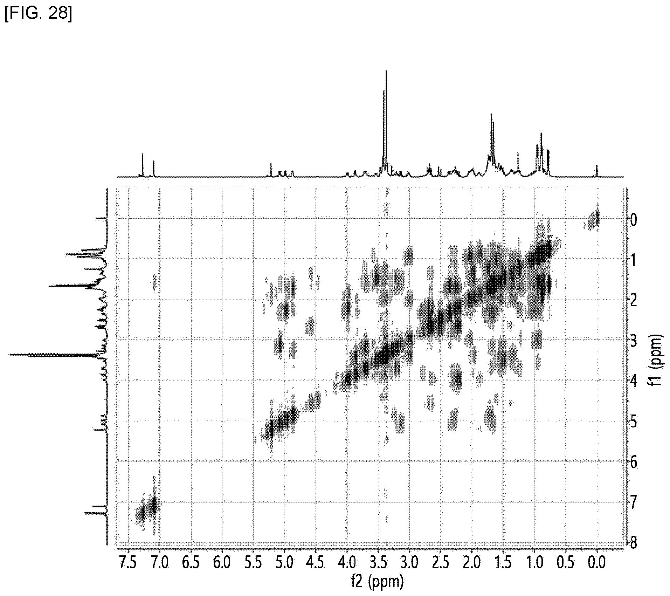

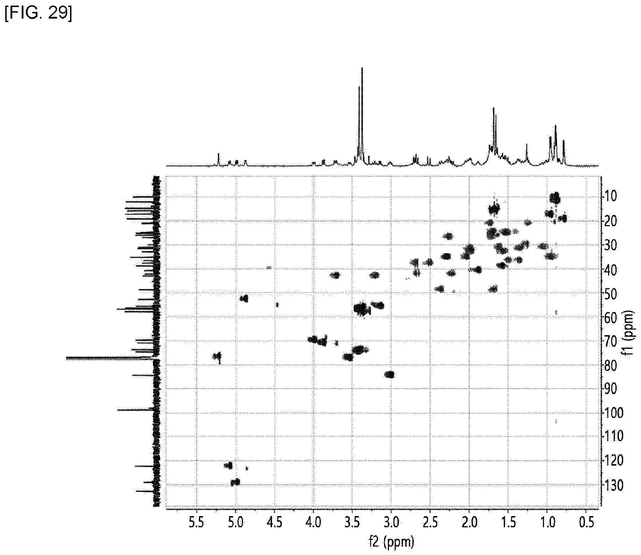

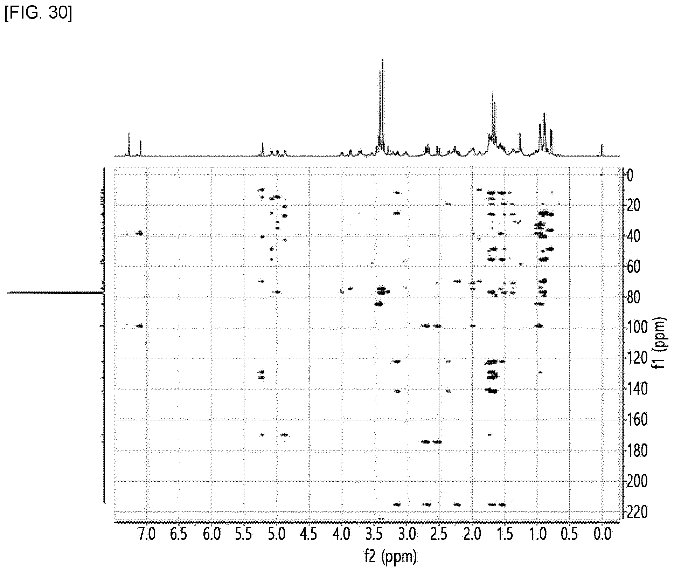

The prepared 9-deoxo-FK520 was identified as follows. Specifically, high-performance liquid chromatography analysis, mass spectrometry, and nuclear magnetic resonance analysis were conducted. Analysis results of 9-deoxo-FK520 are shown in Table 7 and FIGS. 25 to 30, and it was confirmed that 9-deoxo-FK520 was produced by the constructed production strain *Streptomyces kanamyceticus* ΔfkbD,tcsB or *Streptomyces kanamyceticus* ΔfkbD,tcsD based on the results.

Analysis results of 9-deoxo-FK520 (molecular formula: C₄₃H₇₁NO₁, and molecular weight: 778.04) are shown in Table 7 below.

**Table 7**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-31-*O*-demethyl-36,37-dihydro-FK506 was 34 minutes. | | |
| Mass spectrometry | (ESI-HR-MS) Calcd. for C₄₃H₇₁NNaO₁₁⁺: 800.4919, found: *m*/*z* 800.4924 | | |
| Nuclear magnetic resonance analysis | No. | carbon (ppm) | proton (ppm) |
| | 1 | 169.73 | |
| | 2 | 52.59 | 4.85 (1H, br d, *J*=5.0 Hz) |
| | 3 | 26.94 | 1.70 (1H, m), 2.23 (1H, m) |
| | 4 | 21.03 | 1.32 (1H, m), 1.72 (1H, m) |
| | 5 | 24.77 | 1.52 (1H, m), 1.68 (1H, m) |
| | 6 | 42.97 | 3.19 (1H, m), 3.70 (1H, m) |
| | 7 | 174.37 | |
| | 8 | 37.54 | 2.49 (1H, d, *J*=15.0 Hz), 2.67 (1H, d, *J*=15.0 Hz) |
| | 9 | 98.85 | |
| | 10 | 38.78 | 1.56 (1H, m) |
| | 11 | 32.86 | 1.60 (1H, m), 1.94 (1H, m) |
| | 12 | 74.69 | 3.40 (1H, m) |
| | 13 | 70.99 | 3.84 (1H, dd, *J*=10.0 Hz, 5.0 Hz) |
| | 14 | 77.21 | 3.53 (1H, m) |
| | 15 | 36.57 | 1.34 (1H, m), 1.47 (1H, m) |
| | 16 | 26.94 | 1.63 (1H, m) |
| | 17 | 48.49 | 1.64 (1H, m), 2.33 (1H, m) |
| | 18 | 141.15 | |
| | 19 | 122.53 | 5.05 (1H, d, *J*=5.0 Hz) |
| | 20 | 53.84 | 3.20 (1H, d, *J*=5.0 Hz) |
| | 21 | 215.35 | |
| | 22 | 42.20 | 2.20 (1H, br d, *J*=15 Hz), 2.64 (1H, br d, *J*=15 Hz) |
| | 23 | 69.96 | 3.97 (1H, m) |
| | 24 | 40.05 | 1.85 (1H, m) |
| | 25 | 76.90 | 5.20 (1H, br s) |
| | 26 | 132.82 | |
| | 27 | 129 | 4.97 (1H, d, *J*=5.0 Hz) |
| | 28 | 35.29 | 2.32 (1H, m) |
| | 29 | 39.45 | 1.12 (1H, m), 1.88 (1H, m) |
| | 30 | 75.22 | 3.53 (1H, m) |
| | 31 | 75.22 | 3.40 (1H, m) |
| | 32 | 32.32 | 1.33 (1H, m), 1.95 (1H, m) |
| | 33 | 31.19 | 1.04 (1H, m), 1.59 (1H, m) |
| | 34 | 34.14 | 1.46 (1H, m), 1.62 (1H, m) |
| | 35 | 20.73 | 1.22 (2H, m) |
| | 36 | 14.25 | 0.88 (3H, t, *J*=7.5 Hz) |
| | 37 | 17.22 | 0.94 (3H, d, *J*=5 Hz) |
| | 38 | 19.17 | 0.76 (3H, d, *J*=5 Hz) |
| | 39 | 15.82 | 1.63 (3H, s) |
| | 40 | 10.07 | 0.85 (3H, d, *J*=5 Hz) |
| | 41 | 14.77 | 1.65 (3H, s) |
| | 42 | 56.45 | 3.35 (3H, s) |
| | 43 | 57.99 | 3.35 (3H, s) |

Based on ¹H-NMR, ¹³C-NMR, and gHSQC data, although the compound consisting of a total of 43 carbon atoms was similar to 9-deoxo-31-O-demethyl-36,37-dihydro-FK506, the compound was confirmed as a structural isomer since a methoxy group (δ_{H} 3.41 and δ_{C} 56.81) was additionally observed, and one fewer CH₂ functional group was observed. As a result of identifying proton coupling by gCOSY, it was confirmed that the compound had a pipecolyl backbone based on couplings of H-2 to H-5. Based on the methyl group in which the triplet couplings are observed from gHMBC, and the CH₂ group (δ_{H} 1.72 and 1.52, and δ_{C} 20.41) which is formed by the correlation between C-21 (δ_{H} 1.72 and 1.52, and δ_{C} 20.41), it was confirmed that an ethyl group constituting FK520, not a propyl group of FK506, was present at C-21. Based thereon, the structure of the compound No. 5 was determined as 9-deoxo-FK520.

### Example 6: Preparation of 31-O-demethyl-FK520

*Streptomyces kanamyceticus* ΔfkbM,tcsB (accession number: KCTC13583BP) or *Streptomyces kanamyceticus* ΔfkbM,tcsD (accession number: KCTC13584BP), which is a 31-*O*-demethyl-FK520-producing strain, was constructed by inactivating *fkbM* and *tcsB* genes or *fkbM* and *tcsD* genes of *Streptomyces kanamyceticus*, which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbM* and *tcsB* genes or *fkbM* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbM* gene, a primer pair of a left-flanking fragment, FkbMLF/FkbMLR, and a pair of a right-flanking fragment, FkbMRF/FkbMRR, were designed. For deletion of the *tcsB* gene, a primer pair of a left-flanking fragment, TcsBLF/TcsBLR, and a pair of a right-flanking fragment, TcsBRF/TcsBRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with HindIII-Xbal or Xbal-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing 31-O-methyltransferase, pΔfkbM, was transferred to *E. coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, *i.e.*, ΔfkbM, was selected as a phenotype of apramycin sensitivity and identified by PCR, and optionally by Southern blotting.

The *tcsB* gene or the *tcsD* gene was deleted using the same method as that used in deletion of the *fkbM* gene by introducing pΔtcsB or pΔtcsD into the constructed *fkbM* gene-deleted *Streptomyces kanamyceticus* ΔfkbM. ΔfkbM,tcsB or ΔfkbM,tcsD was selected as a phenotype of apramycin sensitivity and identified by PCR, and optionally by Southern blotting.

The constructed strain *Streptomyces kanamyceticus* ΔfkbM,tcsB from which *fkbM* and *tcsB* genes were deleted was deposited at the Korean Collection for Type Cultures (KCTC) of the Korea Research Institute of Bioscience and Biotechnology on July 17, 2018 (accession number: KCTC13583BP), and the constructed strain *Streptomyces kanamyceticus* ΔfkbM,tcsD from which *fkbM* and *tcsD* genes were deleted was also deposited at the Korean Collection for Type Cultures (KCTC) of the Korea Research Institute of Bioscience and Biotechnology on July 17, 2018 (accession number: KCTC13584BP).

31-*O*-Demethyl-FK520 was prepared by culturing the constructed production strain *Streptomyces kanamyceticus* ΔfkbM,tcsB (accession number: KCTC13583BP) or *Streptomyces kanamyceticus* ΔfkbM,tcsD (accession number: KCTC13584BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 31-O-demethyl-FK520 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, i.e., an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 31-O-demethyl-FK520 was confirmed. Aliquot 3 obtained as described above was concentrated using a rotary evaporator and ultimately purified by HPLC.

The concentrate was lyophilized to obtain 31-O-demethyl-FK520 represented by Formula 6 in the form of powder.

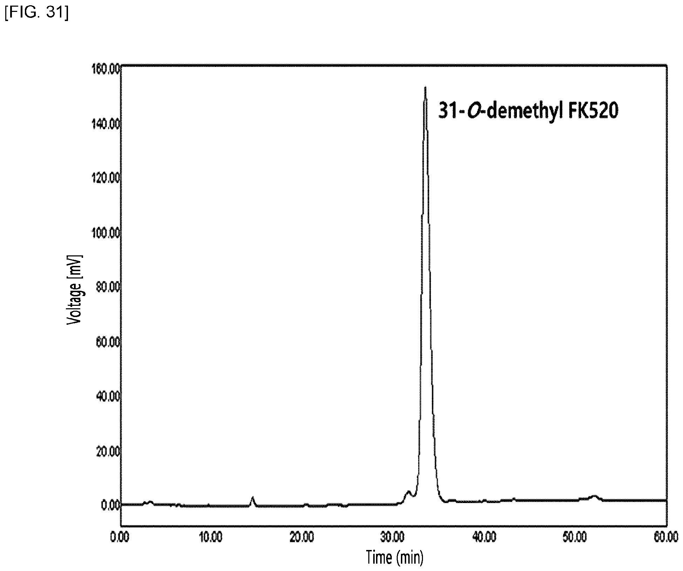

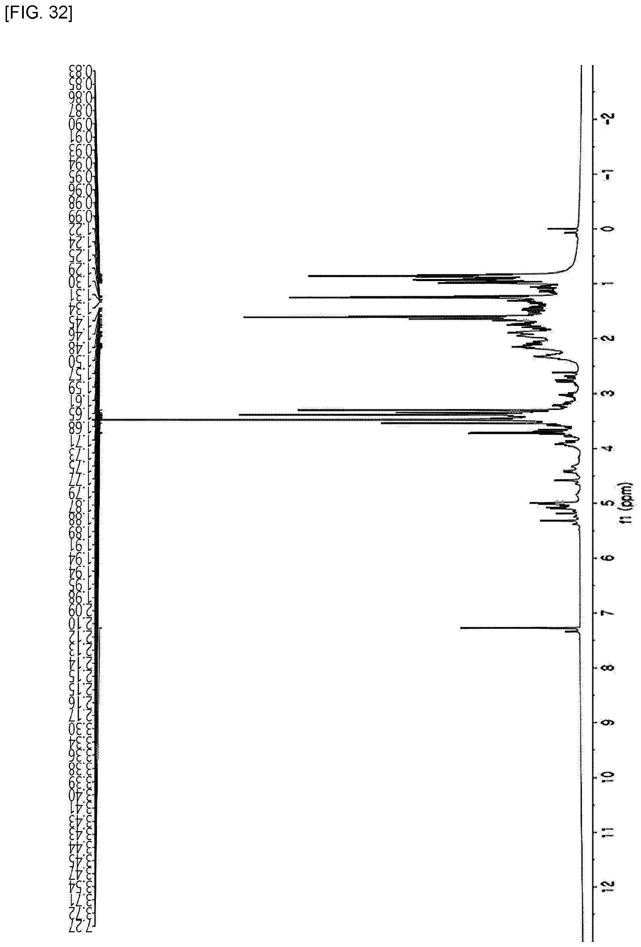

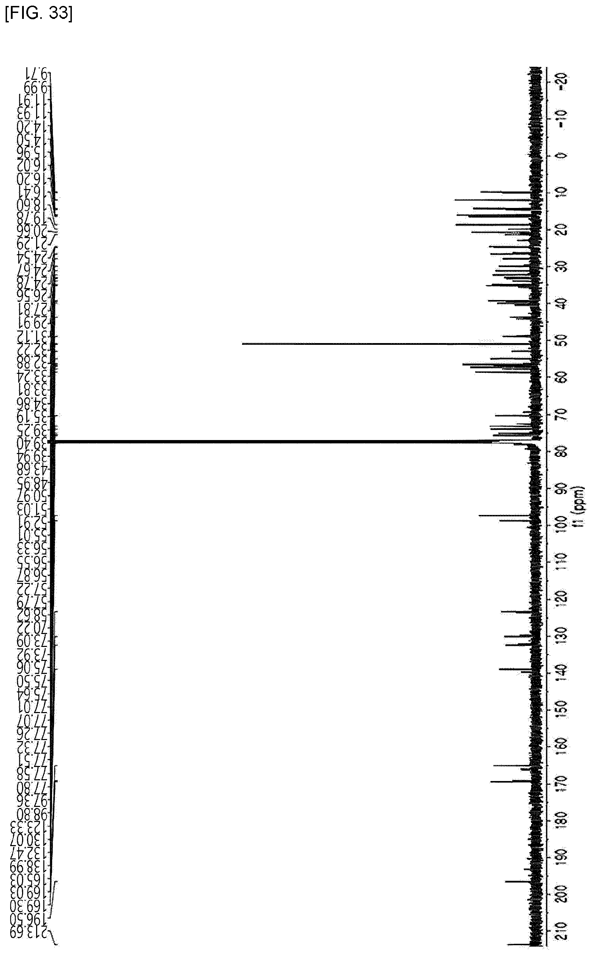

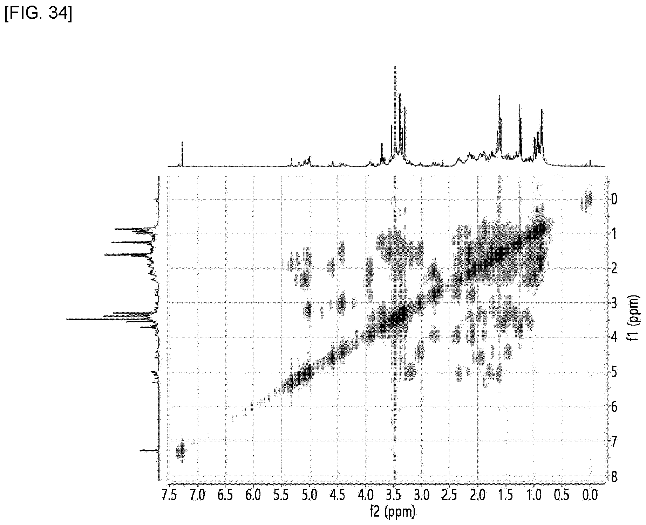

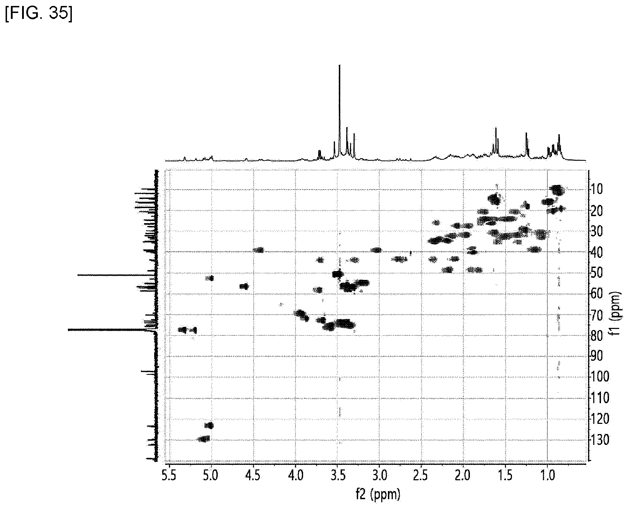

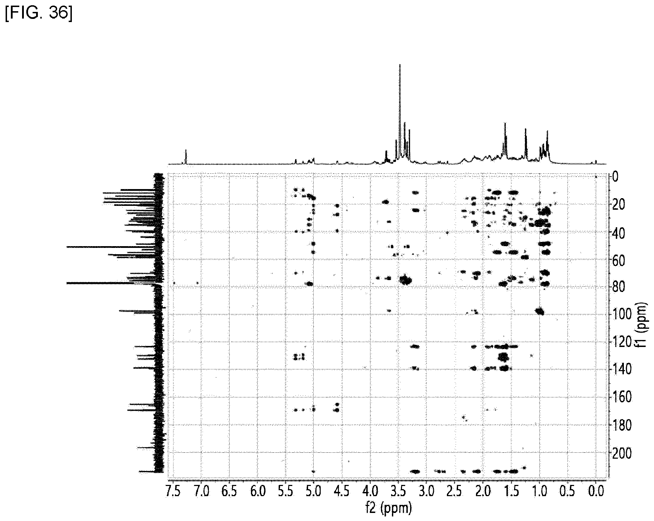

The prepared 31-O-demethyl-FK520 was identified as follows. Specifically, high-performance liquid chromatography analysis, mass spectrometry, and nuclear magnetic resonance analysis were conducted. Analysis results of 31-O-demethyl-FK520 are shown in Table 8 and FIGS. 31 to 36, and it was confirmed that 31-O-demethyl-FK520 was produced by the constructed production strain *Streptomyces kanamyceticus* ΔfkbM,tcsB or *Streptomyces kanamyceticus* ΔfkbM,tcsD based on the results.

Analysis results of 31-O-demethyl-FK520 (molecular formula: C₄₂H₆₇NO₁₂ and molecular weight: 777.99) are shown in Table 8 below.

**Table 8**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 31-O-demethyl-FK520 was 34 minutes. | | |
| Mass spectrometry | (ESI-HR-MS) Calcd. for C₄₂H₆₇NNaO₁₂⁺: 800.4555, found: *m*/*z* 800.4561 | | |
| Nuclear magnetic resonance analysis | No. | carbon (ppm) | proton (ppm) |
| | 1 | 169.30 | |
| | 2 | 56.64 | 4.58 (1H, br d, J=5.0 Hz) |
| | 3 | 27.92 | 2.05 (1H, m), 1.90 (1H, m) |
| | 4 | 21.42 | 1.38 (1H, m), 1.74 (1H, m) |
| | 5 | 24.54 | 1.51 (1H, m), 1.68 (1H, m) |
| | 6 | 39.58 | 3.03 (1H, m), 4.42 (1H, m) |
| | 7 | 165.03 | |
| | 8 | 196.50 | |
| | 9 | 97.36 | |
| | 10 | 35.28 | 2.32 (1H, m) |
| | 11 | 33.01 | 1.52 (1H, m), 2.16 (1H, m) |
| | 12 | 73.92 | 3.39 (1H, m) |
| | 13 | 73.09 | 3.68 (1H, dd, *J*=10.0 Hz, 5.0 Hz) |
| | 14 | 75.06 | 3.42 (1H, m) |
| | 15 | 35.79 | 1.35 (1H, m), 1.56 (1H, m) |
| | 16 | 26.67 | 1.66 (1H, m) |
| | 17 | 49.02 | 1.80 (1H, m), 2.17 (1H, m) |
| | 18 | 138.99 | |
| | 19 | 123.33 | 5.01 (1H, d, *J*=5.0 Hz) |
| | 20 | 53.34 | 3.28 (1H, d, *J*=5.0 Hz) |
| | 21 | 213.69 | |
| | 22 | 43.64 | 2.10 (1H, br d, *J*=15 Hz), 2.77 (1H, br d, *J*=15 Hz) |
| | 23 | 70.22 | 3.92 (1H, m) |
| | 24 | 39.97 | 1.89 (1H, m) |
| | 25 | 77.80 | 5.33 (1H, br s) |
| | 26 | 132.47 | |
| | 27 | 130.07 | 5.09 (1H, d, *J*=5.0 Hz) |
| | 28 | 34.93 | 2.18 (1H, m) |
| | 29 | 39.36 | 1.14 (1H, m), 1.91 (1H, m) |
| | 30 | 75.64 | 3.57 (1H, m) |
| | 31 | 75.50 | 3.36 (1H, m) |
| | 32 | 32.36 | 1.35 (1H, m), 1.98 (1H, m) |
| | 33 | 31.23 | 1.06 (1H, m), 1.61 (1H, m) |
| | 34 | 24.78 | 1.45 (1H, m), 1.72 (1H, m) |
| | 35 | 11.91 | 0.86 (3H, t, *J*=7.5 Hz) |
| | 36 | 16.41 | 0.99 (3H, d, *J*=5 Hz) |
| | 37 | 20.66 | 0.93 (3H, d, *J*=5 Hz) |
| | 38 | 16.02 | 1.59 (3H, s) |
| | 39 | 9.71 | 0.90 (3H, d, *J*=5 Hz) |
| | 40 | 14.20 | 1.62 (3H, s) |
| | 41 | 56.55 | 3.38 (3H, s) |
| | 42 | 57.22 | 3.30 (3H, s) |

Although compound No. 6 has a molecular weight of 800.4563, which is very similar to molecular weights of 800.4924 and 800.4927, it was confirmed that the compound consisting of a total of 42 carbon atoms had one carbon-atom difference due to one fewer methoxy group based on ¹H-NMR, ¹³C-NMR, and gHSQC data, and a ketone carbon (δ_{C} 196.50) was observed. Based on 2D-NMR data, the structure of the compound was determined as 31-O-demethyl-FK520.

### Example 7: Preparation of 9-deoxo-31-O-demethyl-FK520

*Streptomyces kanamyceticus* ΔfkbD-fkbM,tcsB (accession number: KCTC13582BP) or *Streptomyces kanamyceticus* ΔfkbD-fkbM,tcsD (accession number: KCTC13585BP), which is a 9-deoxo-31-O-demethyl-FK520-producing strain, was constructed by inactivating *fkbD-fkbM* and *tcsB* genes or *fkbD-fkbM* and *tcsD* genes of *Streptomyces kanamyceticus,* which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD-fkbM* and *tcsB* genes or *fkbD-fkbM* and *tcsD* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD-fkbM* gene, a primer pair of a left-flanking fragment, FkbD-MLF/FkbD-MLR, and a pair of a right-flanking fragment, FkbD-MRF/FkbD-MRR, were designed. For deletion of the *tcsB* gene, a primer pair of a left-flanking fragment, TcsBLF/TcsBLR, and a pair of a right-flanking fragment, TcsBRF/TcsBRR, were designed. For deletion of the *tcsD* gene, a primer pair of a left-flanking fragment, TcsDLF/TcsDLR, and a pair of a right-flanking fragment, TcsDRF/TcsDRR, were designed. All PCR fragments were isolated, digested with HindIII-XbaI or XbaI-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing both C9 hydroxylase and 31-*O*-methyltransferase, pΔfkbD-fkbM, was transferred to *E*. *coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, *i.e*., ΔfkbD-fkbM, was selected as a phenotype of apramycin sensitivity and identified by PCR, and optionally by Southern blotting.

The *tcsB* gene or *tcsD* gene was deleted using the same method as that used in deletion of the *fkbD-fkbM* gene by introducing pΔtcsB or pΔtcsD into the constructed *fkbD-fkbM* gene-deleted *Streptomyces kanamyceticus* ΔfkbD-fkbM. ΔfkbD-fkbM,tcsB or ΔfkbD-fkbM,tcsD was selected as a phenotype of apramycin sensitivity and identified by PCR, and optionally by Southern blotting.

The constructed strain *Streptomyces kanamyceticus* ΔfkbD-fkbM,tcsB from which *fkbD-fkbM* and *tcsB* genes were deleted was deposited at the Korean Collection for Type Cultures (KCTC) of the Korea Research Institute of Bioscience and Biotechnology on July 17, 2018 (accession number: KCTC13582BP), and the constructed strain *Streptomyces kanamyceticus* ΔfkbD-fkbM,tcsD from which *fkbD-fkbM* and *tcsD* genes were deleted was also deposited at the Korean Collection for Type Cultures (KCTC) of the Korea Research Institute of Bioscience and Biotechnology on July 17, 2018 (accession number: KCTC13585BP).

9-Deoxo-31-*O*-demethyl-FK520 was prepared by culturing the constructed production strain *Streptomyces kanamyceticus* ΔfkbD-fkbM,tcsB (accession number: KCTC13582BP) or *Streptomyces kanamyceticus* ΔfkbD-fkbM,tcsD (accession number: KCTC13585BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-31-*O*-demethyl-FK520 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e*., an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-31-O-demethyl-FK520 was confirmed. Aliquot 3 obtained as described above was concentrated using a rotary evaporator and ultimately purified by HPLC.

The concentrate was lyophilized to obtain 9-deoxo-31-*O*-demethyl-FK520 represented by Formula 7 in the form of powder.

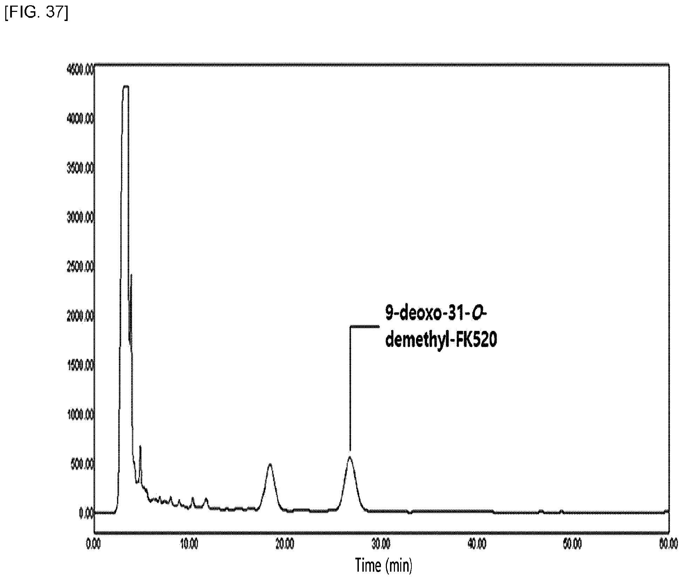

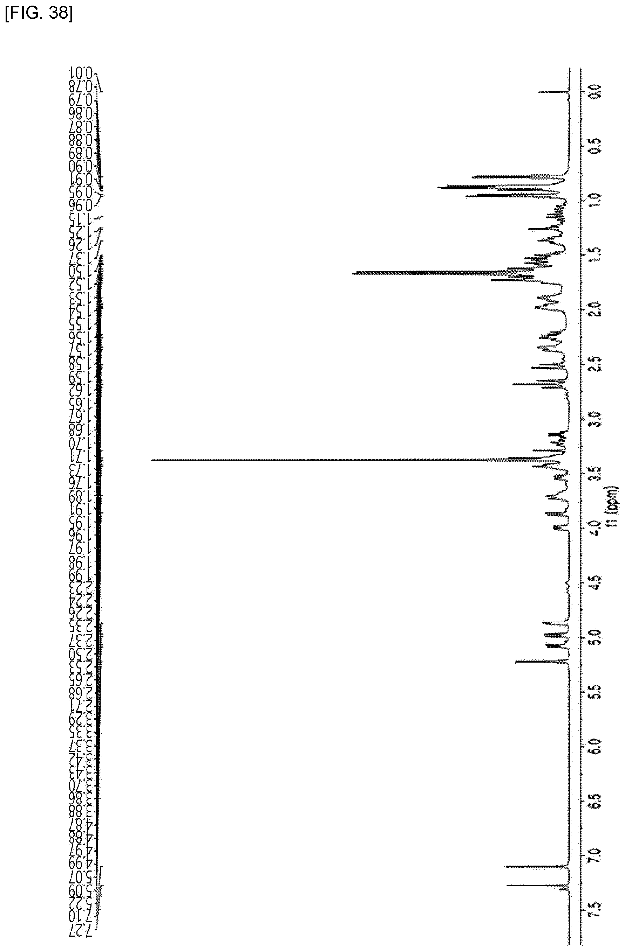

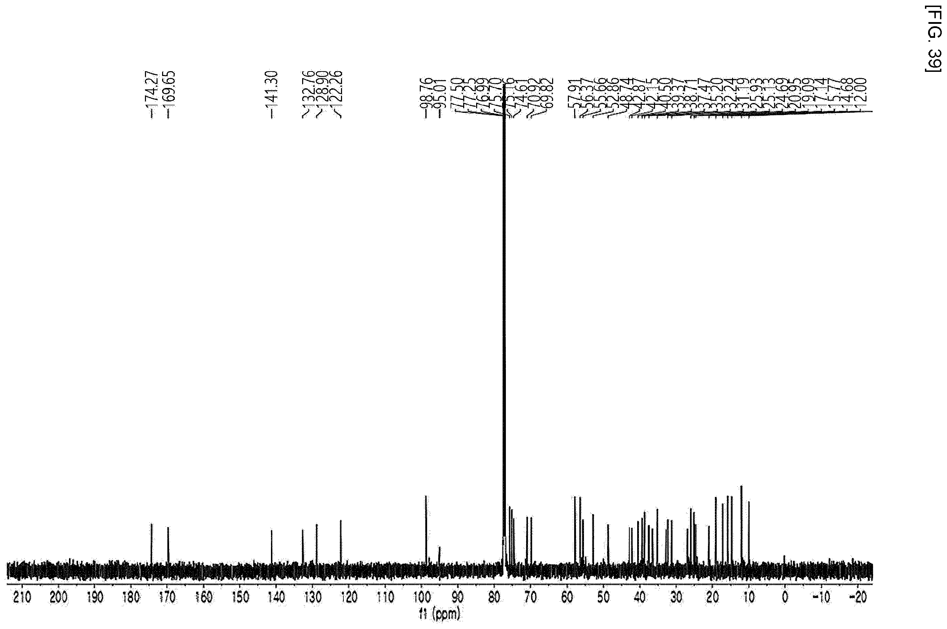

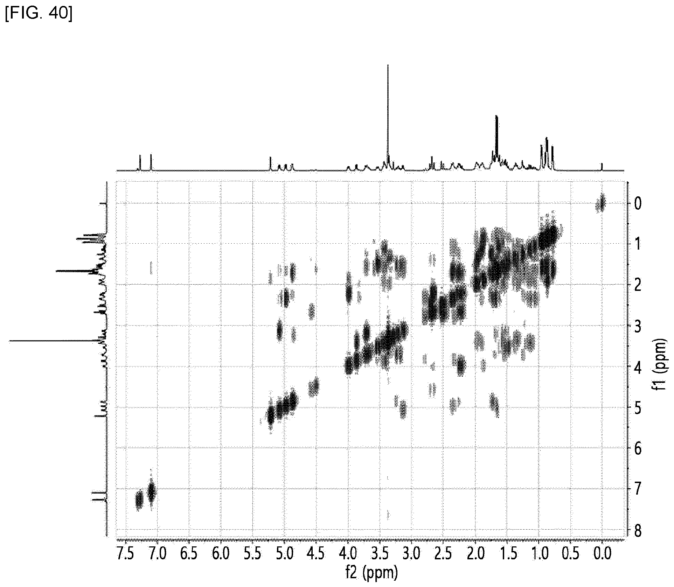

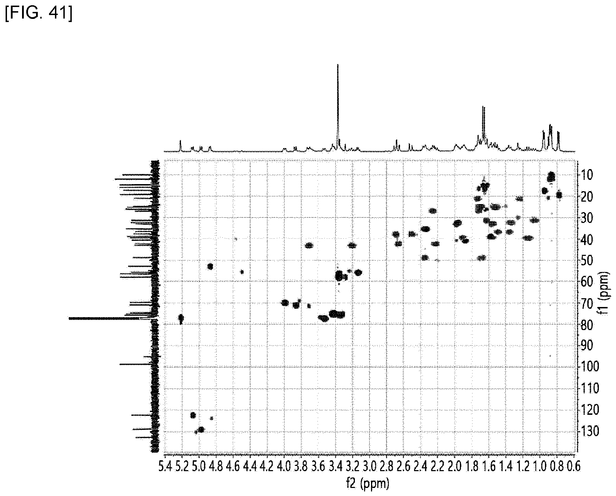

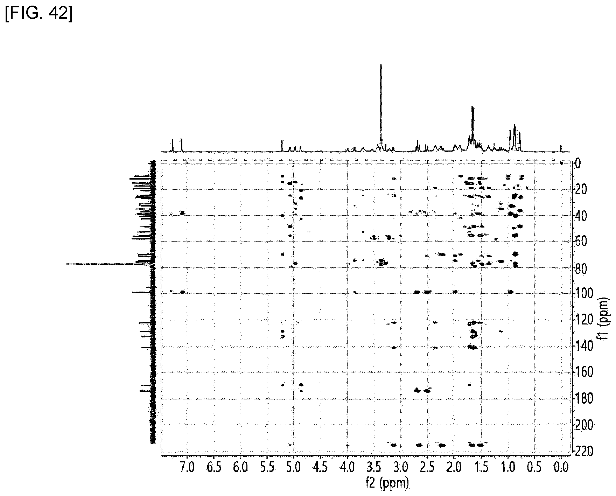

The prepared 9-deoxo-31-*O*-demethyl-FK520 was identified as follows. Specifically, high-performance liquid chromatography analysis, mass spectrometry, and nuclear magnetic resonance analysis were conducted. Analysis results of 9-deoxo-31-*O*-demethyl-FK520 are shown in Table 9 and FIGS. 37 to 42, and it was confirmed that 9-deoxo-31-*O*-demethyl-FK520 was produced by the constructed production strain *Streptomyces kanamyceticus* fkbD-fkbM,tcsB or *Streptomyces kanamyceticus* ΔfkbD-fkbM,tcsD based on the results.

Analysis results of 9-deoxo-31-*O*-demethyl-FK520 (molecular formula: C₄₂H₆₉NO₁₁ and molecular weight: 764.01) are shown in Table 9 below.

**Table 9**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-31-O-demethyl-FK520 was 24 minutes. | | |
| Mass spectrometry | (ESI-HR-MS) Calcd. for C₄₂H₆₉NNaO₁₁⁺: 786.4763, found: *m*/*z* 786.4769 | | |
| Nuclear magnetic resonance analysis | No. | carbon (ppm) | proton (ppm) |
| | 1 | 169.65 | |
| | 2 | 52.86 | 4.87 (1H, br d, *J*=5.0 Hz) |
| | 3 | 26.86 | 1.70 (1H, m), 2.23 (1H, m) |
| | 4 | 20.95 | 1.32 (1H, m), 1.72 (1H, m) |
| | 5 | 24.69 | 1.52 (1H, m), 1.68 (1H, m) |
| | 6 | 42.97 | 3.19 (1H, m), 3.70 (1H, m) |
| | 7 | 174.27 | |
| | 8 | 37.54 | 2.49 (1H, d, *J*=15.0 Hz), 2.67 (1H, d, *J*=15.0 Hz) |
| | 9 | 98.76 | |
| | 10 | 38.78 | 1.56 (1H, m) |
| | 11 | 32.86 | 1.60 (1H, m), 1.94 (1H, m) |
| | 12 | 74.61 | 3.41 (1H, m) |
| | 13 | 70.92 | 3.86 (1H, dd, *J*=10.0 Hz, 5.0 Hz) |
| | 14 | 77.21 | 3.53 (1H, m) |
| | 15 | 36.57 | 1.34 (1H, m), 1.47 (1H, m) |
| | 16 | 25.93 | 1.63 (1H, m) |
| | 17 | 48.49 | 1.64 (1H, m), 2.33 (1H, m) |
| | 18 | 141.30 | |
| | 19 | 122.26 | 5.05 (1H, d, *J*=5.0 Hz) |
| | 20 | 55.66 | 3.20 (1H, d, *J*=5.0 Hz) |
| | 21 | 215.35 | |
| | 22 | 42.20 | 2.20 (1H, br d, *J*=15 Hz), 2.64 (1H, br d, *J*=15 Hz) |
| | 23 | 69.82 | 3.98 (1H, m) |
| | 24 | 40.05 | 1.85 (1H, m) |
| | 25 | 76.90 | 5.20 (1H, br s) |
| | 26 | 132.76 | |
| | 27 | 128.90 | 4.97 (1H, d, *J*=5.0 Hz) |
| | 28 | 35.29 | 2.32 (1H, m) |
| | 29 | 39.45 | 1.12 (1H, m), 1.88 (1H, m) |
| | 30 | 75.22 | 3.53 (1H, m) |
| | 31 | 75.22 | 3.40 (1H, m) |
| | 32 | 32.32 | 1.33 (1H, m), 1.95 (1H, m) |
| | 33 | 31.19 | 1.04 (1H, m), 1.59 (1H, m) |
| | 34 | 25.13 | 1.52 (1H, m), 1.68 (1H, m) |
| | 35 | 12.00 | 0.88 (3H, t, *J*=7.5 Hz) |
| | 36 | 17.14 | 0.95 (3H, d, *J*=5 Hz) |
| | 37 | 19.09 | 0.77 (3H, d, *J*=5 Hz) |
| | 38 | 15.77 | 1.65 (3H, s) |
| | 39 | 9.98 | 0.86 (3H, d, *J*=5 Hz) |
| | 40 | 14.68 | 1.67 (3H, s) |
| | 41 | 56.37 | 3.38 (3H, s) |
| | 42 | 57.91 | 3.38 (3H, s) |

Based on ¹H-NMR, ¹³C-NMR, and gHSQC data, although the obtained compound consisting of a total of 42 carbon atoms exhibited similar results to those of 31-O-demethyl-FK520, a CH₂ functional group (δ_{H} 2.67 and 2.49 and δ_{C} 37.54) generated from the ΔfkbD-fkbM gene was observed. Based on 2D-NMR data, the structure of the compound No. 7 was determined as 9-deoxo-31-O-demethyl-FK520.

### Example 8: Preparation of 9-deoxo-FK523

*Streptomyces kanamyceticus* ΔfkbD,tcsB (accession number: KCTC13579BP), which is a 9-deoxo-FK523-producing strain, was constructed by inactivating *fkbD* and *tcsB* genes of *Streptomyces kanamyceticus,* which is an FK506-producing strain, via an in-frame deletion caused by double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD* and *tcsB* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD* gene, a primer pair of a left-flanking fragment, FkbDLF/FkbDLR, and a pair of a right-flanking fragment, FkbDRF/FkbDRR, were designed. For deletion of the *tcsB* gene, a primer pair of a left-flanking fragment, TcsBLF/TcsBLR, and a pair of a right-flanking fragment, TcsBRF/TcsBRR, were designed. All PCR fragments were isolated, digested with HindIII-XbaI or XbaI-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing C9 hydroxylase, pΔfkbD, was transferred to *E*. *coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, *i.e*., ΔfkbD, was selected as a phenotype of apramycin sensitivity and identified by PCR, and optionally by Southern blotting.

The *tcsB* gene was deleted using the same method as that used in deletion of the *fkbD* gene by introducing pΔtcsB into the constructed *fkbD* gene-deleted *Streptomyces kanamyceticus* ΔfkbD. ΔfkbD,tcsB was selected as a phenotype of apramycin sensitivity and identified by PCR, and optionally by Southern blotting.

The constructed strain *Streptomyces kanamyceticus* ΔfkbD,tcsB from which *fkbD* and *tcsB* genes were deleted was deposited at the Korean Collection for Type Cultures (KCTC) of the Korea Research Institute of Bioscience and Biotechnology on July 17, 2018 (accession number: KCTC13579BP).

9-Deoxo-FK523 was prepared by culturing the constructed production strain *Streptomyces kanamyceticus* ΔfkbD,tcsB (accession number: KCTC13579BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-FK523 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e*., an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-FK523 was confirmed. Aliquot 3 obtained as described above was concentrated using a rotary evaporator and ultimately purified by HPLC.

The concentrate was lyophilized to obtain 9-deoxo-FK523 represented by Formula 8 in the form of powder.

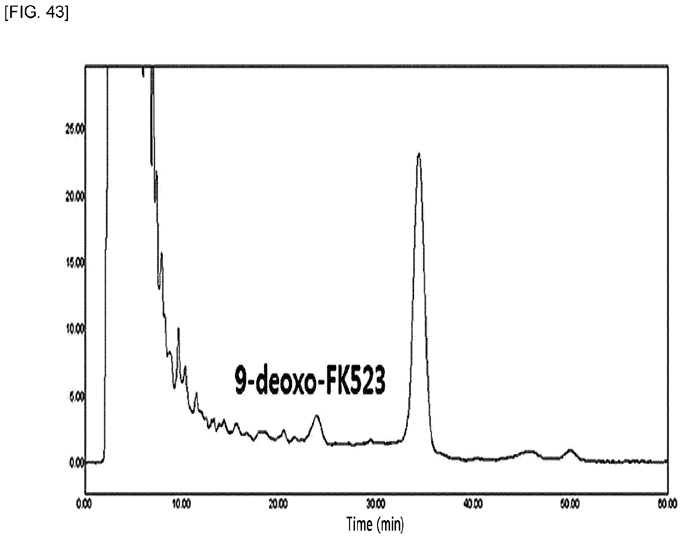

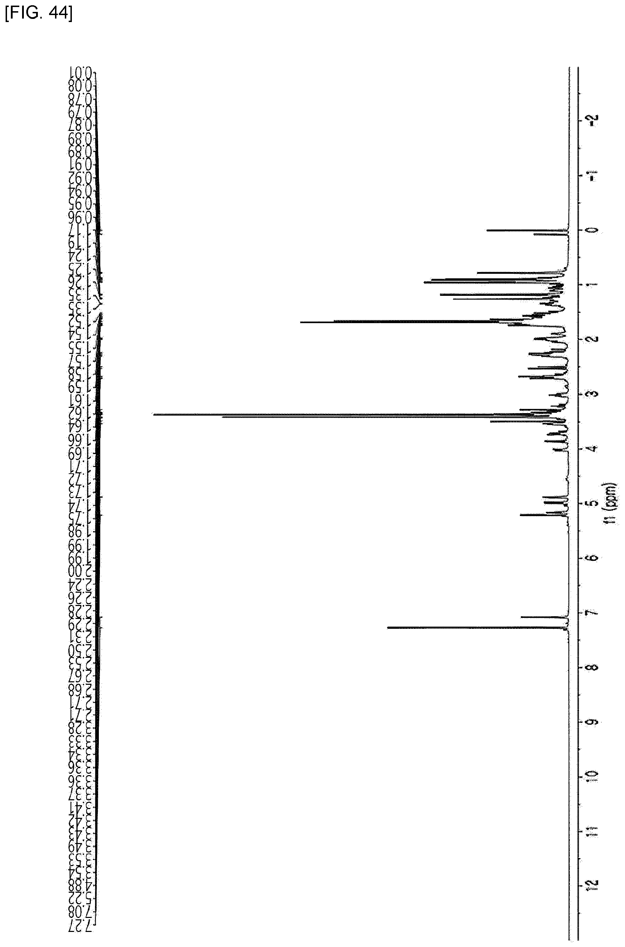

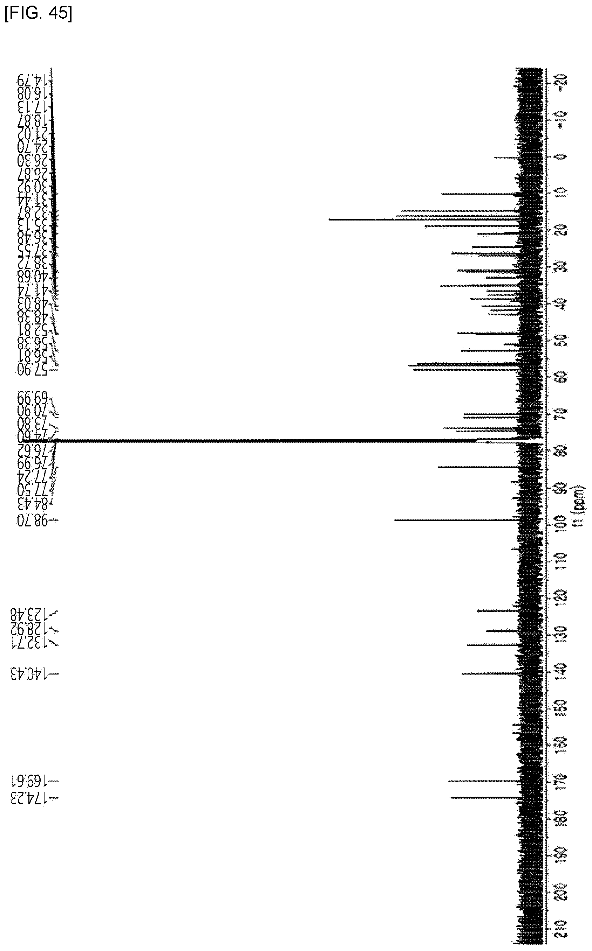

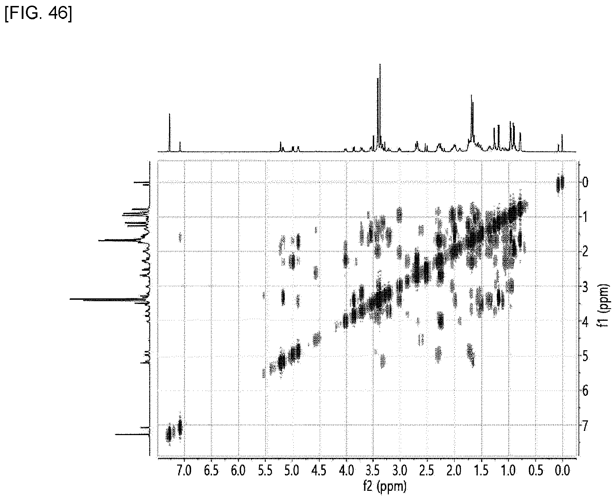

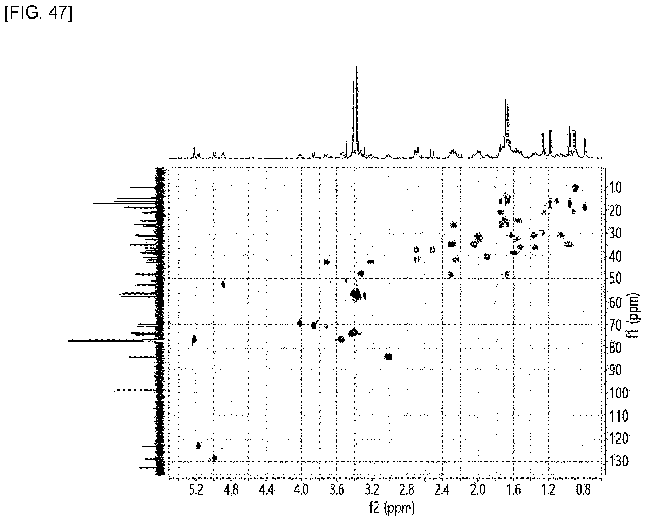

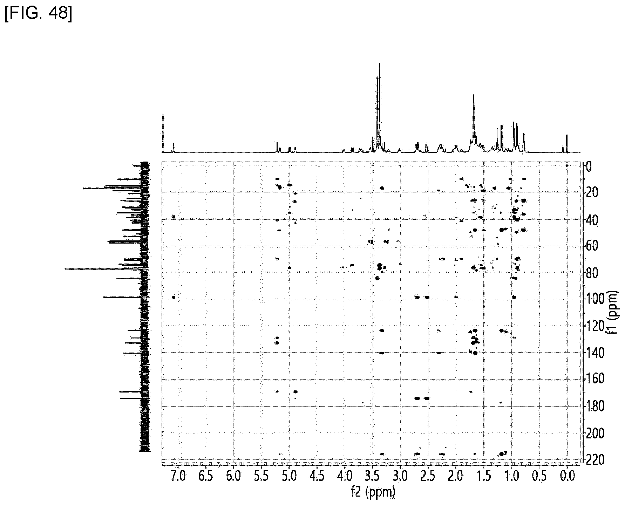

The prepared 9-deoxo-FK523 was identified as follows. Specifically, high-performance liquid chromatography analysis, mass spectrometry, and nuclear magnetic resonance analysis were conducted. Analysis results of 9-deoxo-FK523 are shown in Table 10 and FIGS. 43 to 48, and it was confirmed that 9-deoxo-FK523 was produced by the constructed production strain *Streptomyces kanamyceticus* ΔfkbD,tcsB based on the results.

Analysis results of 9-deoxo-FK523 (molecular formula: C₄₂H₆₉NO₁₁ and molecular weight: 764.01) are shown in Table 10 below.

**Table 10**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-FK523 was 24 minutes. | | |
| Mass spectrometry | (ESI-HR-MS) Calcd. for C₄₂H₆₉NNaO₁₁⁺: 786.4763, found: *m*/*z* 786.4769 | | |
| Nuclear magnetic resonance analysis | No. | carbon (ppm) | proton (ppm) |
| | 1 | 169.61 | |
| | 2 | 52.59 | 4.85 (1H, br d, *J*=5.0 Hz) |
| | 3 | 26.87 | 1.70 (1H, m), 2.24 (1H, m) |
| | 4 | 20.70 | 1.31 (1H, m), 1.72 (1H, m) |
| | 5 | 24.42 | 1.51 (1H, m), 1.68 (1H, m) |
| | 6 | 42.65 | 3.19 (1H, m), 3.69 (1H, m) |
| | 7 | 174.23 | |
| | 8 | 36.25 | 2.51 (1H, d, *J*=15.0 Hz), 2.70 (1H, d, *J*=15.0 Hz) |
| | 9 | 98.70 | |
| | 10 | 38.72 | 1.58 (1H, m) |
| | 11 | 32.84 | 1.60 (1H, m), 1.94 (1H, m) |
| | 12 | 74.60 | 3.40 (1H, m) |
| | 13 | 70.90 | 3.86 (1H, dd, *J*=10.0 Hz, 5.0 Hz) |
| | 14 | 77.11 | 3.53 (1H, m) |
| | 15 | 36.48 | 1.34 (1H, m), 1.47 (1H, m) |
| | 16 | 26.30 | 1.50 (1H, m) |
| | 17 | 48.49 | 1.65 (1H, m), 2.32 (1H, m) |
| | 18 | 140.43 | |
| | 19 | 123.48 | 5.17 (1H, d, *J=*5.0 Hz) |
| | 20 | 53.45 | 3.21 (1H, d, *J*=5.0 Hz) |
| | 21 | 215.35 | |
| | 22 | 41.74 | 2.21 (1H, br d, *J*=15 Hz), 2.66 (1H, br d, *J*=15 Hz) |
| | 23 | 69.99 | 4.01 (1H, m) |
| | 24 | 40.68 | 1.90 (1H, m) |
| | 25 | 76.62 | 5.22 (1H, br s) |
| | 26 | 132.71 | |
| | 27 | 128.92 | 4.98 (1H, d, *J*=5.0 Hz) |
| | 28 | 35.13 | 2.35 (1H, m) |
| | 29 | 35.13 | 0.93 (1H, m), 2.01 (1H, m) |
| | 30 | 84.43 | 3.00 (1H, m) |
| | 31 | 73.80 | 3.39 (1H, m) |
| | 32 | 32.87 | 1.34 (1H, m), 1.98 (1H, m) |
| | 33 | 31.44 | 1.06 (1H, m), 1.61 (1H, m) |
| | 34 | 17.13 | 1.18 (3H, d, *J*=5 Hz) |
| | 35 | 17.13 | 0.96 (3H, d, *J*=5 Hz) |
| | 36 | 18.87 | 0.78 (3H, d, *J*=5 Hz) |
| | 37 | 16.08 | 1.63 (3H, s) |
| | 38 | 10.12 | 0.90 (3H, d, *J*=5 Hz) |
| | 39 | 14.79 | 1.66 (3H, s) |
| | 40 | 56.38 | 3.37 (3H, s) |
| | 41 | 57.90 | 3.37 (3H, s) |
| | 42 | 56.81 | 3.41 (3H, s) |

The obtained compound had the same molecular weight to that of 9-deoxo-31-O-demethyl-FK520. Although the compound consisting of a total of 42 carbon atoms had a very similar structure thereto based on ¹H-NMR, ¹³C-NMR, and gHSQC data, the compound was confirmed as a structural isomer since a methoxy group (δ_{H} 3.41 and δ_{C} 56.81) was additionally observed, and one fewer CH₂ functional group was observed, unlike 9-deoxo-31-O-demethyl-FK520. As a result of identifying proton coupling by gCOSY, it was confirmed that the compound had a pipecolyl backbone based on coupling of H-2 to H-5. Based on gHMBC, the methyl group (δ_{H} 1.18 and δ_{C} 17.13) observed in a double coupled state was correlated with C-21 (δ_{H} 3.21 and δ_{C} 53.45), and thus it was confirmed that a methyl group constituting FK523 was present at C-21. Based thereon, the structure of the compound was determined as 9-deoxo-FK523.

### Example 9: Preparation of 9-deoxo-31-O-demethyl-FK523

*Streptomyces kanamyceticus* ΔfkbD-fkbM,tcsB (accession number: KCTC13582BP), which is a 9-deoxo-31-*O*-demethyl-FK523-producing strain, was constructed by inactivating *fkbD-fkbM* and *tcsB* genes of *Streptomyces kanamyceticus,* which is an FK506-producing strain, via an in-frame deletion caused by a double cross-over homologous recombination, according to a method introduced by Ban, Y. H. et al., (J. Nat. Prod. 2013, 76, 1091-1098).

Specifically, in order to construct a mutant of the FK506-producing *Streptomyces kanamyceticus* strain from which *fkbD-fkbM* and *tcsB* genes were deleted, each gene was cloned to a pKC1139 vector and transferred to *Escherichia coli* ET12567/pUZ8002, and then the FK506-producing *Streptomyces kanamyceticus* strain was transformed with the vector by conjugation.

A method of constructing the strain may be explained more specifically as construction of an in-frame gene deletion plasmid and construction of a gene-deleted strain.

In the construction of the in-frame gene deletion plasmid, the *E. coli-Streptomyces* shuttle vector pKC1139 was used for in-frame gene deletion. Construction of the plasmid was performed by PCR of left- and right-flanking fragments of a target gene for deletion of Fosmid DNA derived from *Streptomyces kanamyceticus.* For deletion of the *fkbD-fkbM* gene, a primer pair of a left-flanking fragment, FkbD-MLF/FkbD-MLR, and a pair of a right-flanking fragment, FkbD-MRF/FkbD-MRR, were designed. For deletion of the *tcsB* gene, a primer pair of a left-flanking fragment, TcsBLF/TcsBLR, and a pair of a right-flanking fragment, TcsBRF/TcsBRR, were designed. All PCR fragments were isolated, digested with HindIII-XbaI or XbaI-EcoRI, and cloned to the pKC1139 vector. Information on the strains, plasmids, and primers used in this example are shown in Tables 1 and 2.

The plasmids used for construction of the gene-deleted strain are shown in Table 1. A plasmid for removing both C9 hydroxylase and 31-*O*-methyltransferase, pΔfkbD-fkbM, was transferred to *E*. *coli* ET12567/pUZ8002 and introduced into *Streptomyces kanamyceticus* by conjugation to delete the target gene by homologous recombination. A strain in which single cross-over occurred between the deletion plasmid and the chromosome of *Streptomyces kanamyceticus* was selected by culturing an apramycin-resistant transconjugant in the presence of apramycin at 37°C (non-permissive temperature for replication of a pSG5-based replicon). Then, acquired colonies were proliferated three times at 28°C without selection to allow second cross-over. The obtained double cross-over mutant, i.e., ΔfkbD-fkbM, was selected as a phenotype of apramycin sensitivity and identified by PCR, and optionally by Southern blotting.

The *tcsB* gene was deleted using the same method as that used in deletion of the *fkbD-fkbM* gene by introducing pΔtcsB into the constructed *fkbD-fkbM* gene-deleted *Streptomyces kanamyceticus* ΔfkbD-fkbM. ΔfkbD-fkbM,tcsB was selected as a phenotype of apramycin sensitivity and identified by PCR, and optionally by Southern blotting.

The constructed strain *Streptomyces kanamyceticus* ΔfkbD-fkbM,tcsB from which *fkbD-fkbM* and *tcsB* genes were deleted was deposited at the Korean Collection for Type Cultures (KCTC) of the Korea Research Institute of Bioscience and Biotechnology on July 17, 2018 (accession number: KCTC13582BP).

9-Deoxo-31-*O*-demethyl-FK523 was prepared by culturing the constructed production strain *Streptomyces kanamyceticus* ΔfkbD-fkbM,tcsB (accession number: KCTC13582BP). This will be described in more detail. 50 mL of an R2YE medium (including 103 g/L sucrose, 10 g/L glucose, 0.25 g/L K₂SO₄, 10.12 g/L MgCl₂·6H₂O, 0.1 g/L casamino acid, 50 mL/L yeast extract (10%), 100 mL/L TES buffer (5.73%, pH 7.2), 10 mL/L potassium phosphate (0.5%), 80 mL/L CaCl₂·2H₂O (3.68%), 15 mL/L L-proline (20%), 2 mL/L trace element solution, and 5 mL/L NaOH (1 N)) was added to a 250 mL baffled flask, and the production strain was inoculated thereinto. Then, the strain was pre-incubated in a rotary shaker incubator at 28°C and 180 rpm for two days. Subsequently, 10 mL of the pre-incubated culture was inoculated into 1 L of the R2YE medium contained in a 3 L Erlenmeyer flask. After inoculation, the strain was incubated at 28°C and 180 rpm for 6 days. After 6 days of the incubation, 9-deoxo-31-*O*-demethyl-FK523 produced thereby was extracted by using a first extraction process.

The first extraction process was performed as follows. First, methanol was added to the culture broth in an equal volume and mixed for 30 minutes, and the microbial cells were removed by centrifugation, and then an extract from which the microbial cells were removed was concentrated using a rotary evaporator. After the concentrated extract was dissolved in water, ethyl acetate was added in twice the volume of the extract and mixed, and the mixture was left until layers were separated. After the layers were separated, an upper layer, *i.e*., an organic solvent layer, was recovered and concentrated using a rotary evaporator, and a weight after concentration was measured. The extract obtained by way of the first extraction process was passed through a column filled with silica gel. In this case, an amount of the silica gel was 15 times the weight of the extract obtained by way of the first extraction process, and methanol and methylene chloride mixed in five ratios (Aliquot 1. 0:100, Aliquot 2. 1:100, Aliquot 3. 1:10, Aliquot 4. 1:1, and Aliquot 5. 100:0) were used as mobile phases. In Aliquot 3, 9-deoxo-31-*O*-demethyl-FK523 was confirmed. Aliquot 3 obtained as described above was concentrated using a rotary evaporator and ultimately purified by HPLC.

The concentrate was lyophilized to obtain 9-deoxo-31-*O*-demethyl-FK523 represented by Formula 9 in the form of powder.

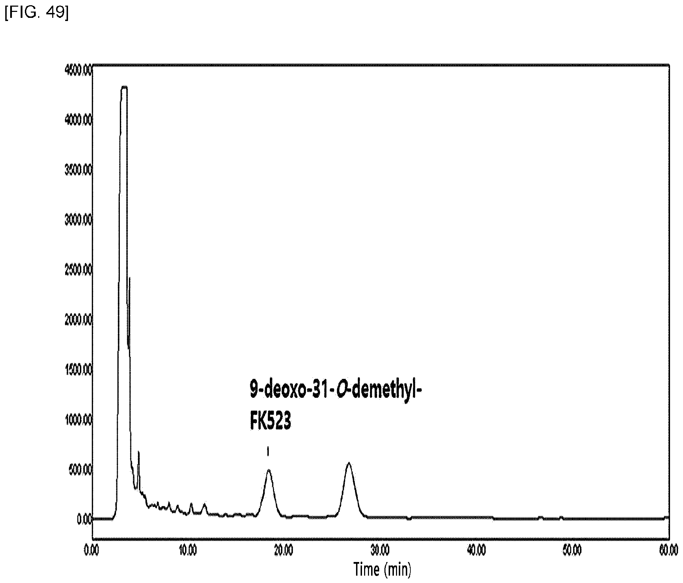

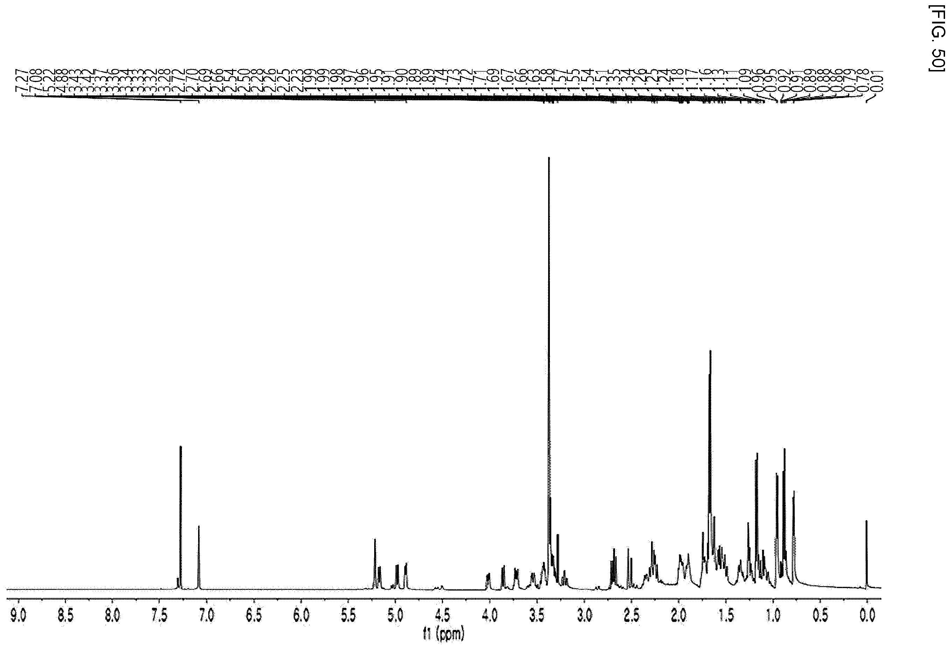

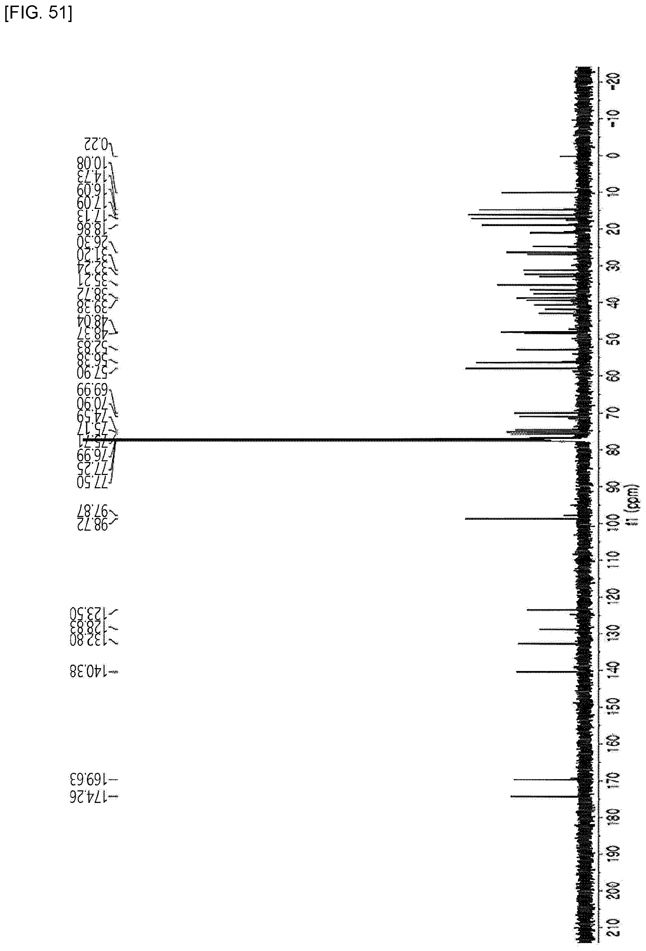

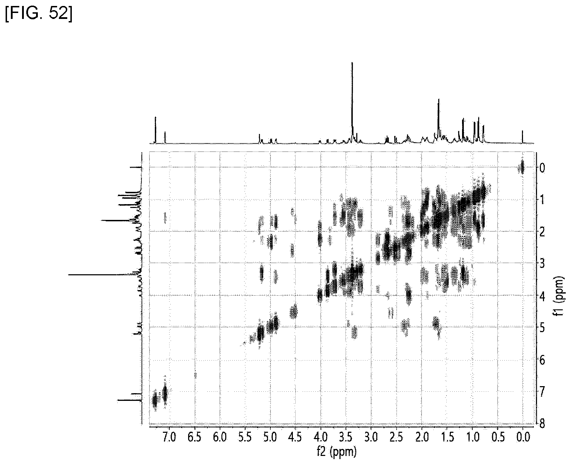

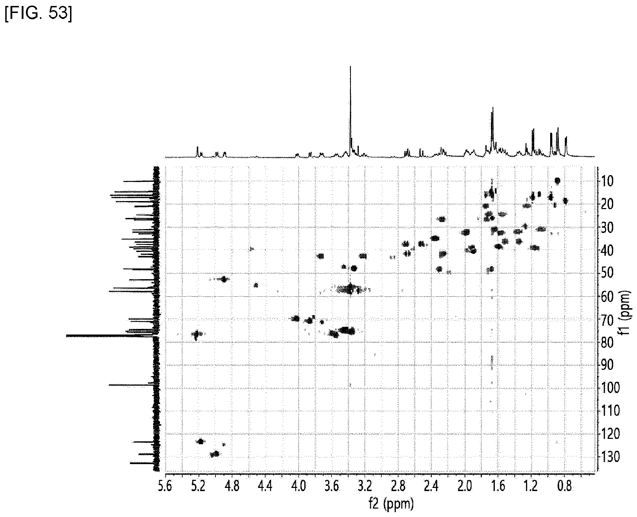

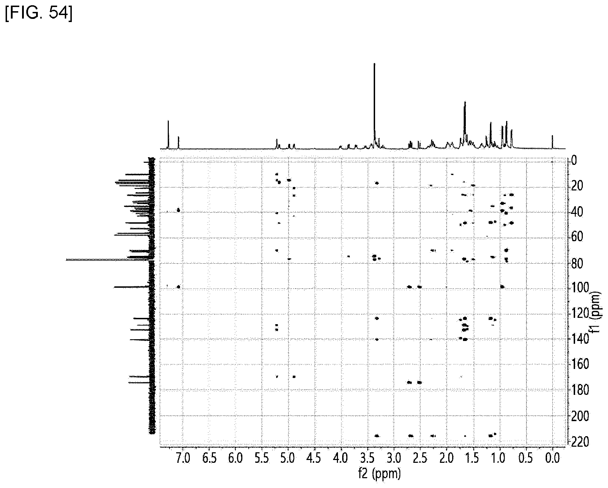

The prepared 9-deoxo-31-*O*-demethyl-FK523 was identified as follows. Specifically, high-performance liquid chromatography analysis, mass spectrometry, and nuclear magnetic resonance analysis were conducted. Analysis results of 9-deoxo-31-*O*-demethyl-FK523 are shown in Table 11 and FIGS. 49 to 54, and it was confirmed that 9-deoxo-31-*O*-demethyl-FK523 was produced by the constructed production strain *Streptomyces kanamyceticus* ΔfkbD-fkbM,tcsB based on the results.

Analysis results of 9-deoxo-31-*O*-demethyl-FK523 (molecular formula: C₄₁H₆₇NO₁₁ and molecular weight: 752.01) are shown in Table 11 below.

**Table 11**

| Analysis method | Analysis results | | |
|---|---|---|---|
| High-performance liquid chromatography analysis | HPLC was performed using a 45-55% acetonitrile aqueous solution as a mobile phase at a flow rate of 1 mL/min and analyzed using a UV detector at 205 nm. | | |
| | In this regard, a retention time of 9-deoxo-31-O-demethyl-FK523 was 17 minutes. | | |
| Mass spectrometry | (ESI-HR-MS) Calcd. for C₄₁H₆₇NNaO₁₁⁺: 772.4606, found: *m*/*z* 772.4612 | | |
| Nuclear magnetic resonance analysis | No. | carbon (ppm) | proton (ppm) |
| | 1 | 169.63 | |
| | 2 | 52.83 | 4.90 (1H, br d, *J*=5.0 Hz) |
| | 3 | 26.88 | 1.70 (1H, m), 2.24 (1H, m) |
| | 4 | 20.70 | 1.31 (1H, m), 1.72 (1H, m) |
| | 5 | 24.70 | 1.51 (1H, m), 1.68 (1H, m) |
| | 6 | 42.89 | 3.21 (1H, m), 3.72 (1H, m) |
| | 7 | 174.26 | |
| | 8 | 36.25 | 2.51 (1H, d, *J*=15.0 Hz), 2.70 (1H, d, *J*=15.0 Hz) |
| | 9 | 98.70 | |
| | 10 | 38.72 | 1.58 (1H, m) |
| | 11 | 32.86 | 1.60 (1H, m), 1.94 (1H, m) |
| | 12 | 74.59 | 3.40 (1H, m) |
| | 13 | 70.90 | 3.86 (1H, dd, *J*=10.0 Hz, 5.0 Hz) |
| | 14 | 77.11 | 3.53 (1H, m) |
| | 15 | 36.48 | 1.34 (1H, m), 1.47 (1H, m) |
| | 16 | 26.30 | 1.50 (1H, m) |
| | 17 | 48.37 | 1.65 (1H, m), 2.32 (1H, m) |
| | 18 | 140.38 | |
| | 19 | 123.50 | 5.17 (1H, d, *J*=5.0 Hz) |
| | 20 | 48.04 | 3.21 (1H, d, *J*=5.0 Hz) |
| | 21 | 215.35 | |
| | 22 | 41.79 | 2.21 (1H, br d, *J*=15 Hz), 2.66 (1H, br d, *J*=15 Hz) |
| | 23 | 69.99 | 4.01 (1H, m) |
| | 24 | 40.60 | 1.90 (1H, m) |
| | 25 | 76.70 | 5.22 (1H, br s) |
| | 26 | 132.80 | |
| | 27 | 128.83 | 4.98 (1H, d, *J*=5.0 Hz) |
| | 28 | 35.21 | 2.35 (1H, m) |
| | 29 | 39.38 | 1.17 (1H, m), 1.91 (1H, m) |
| | 30 | 75.71 | 3.43 (1H, m) |
| | 31 | 75.17 | 3.36 (1H, m) |
| | 32 | 32.24 | 1.34 (1H, m), 1.98 (1H, m) |
| | 33 | 31.20 | 1.06 (1H, m), 1.61 (1H, m) |
| | 34 | 17.09 | 1.18 (3H, d, *J*=5 Hz) |
| | 35 | 17.13 | 0.96 (3H, d, *J*=5 Hz) |
| | 36 | 18.86 | 0.78 (3H, d, *J*=5 Hz) |
| | 37 | 16.09 | 1.66 (3H, s) |
| | 38 | 10.08 | 0.88 (3H, d, *J*=5 Hz) |
| | 39 | 14.73 | 1.67 (3H, s) |
| | 40 | 56.38 | 3.37 (3H, s) |
| | 41 | 57.90 | 3.37 (3H, s) |

Based on ¹H-NMR, ¹³C-NMR, and gHSQC data, the obtained compound consisting of a total of 41 carbon atoms had one fewer methoxy group unlike 9-deoxo-FK523. Together therewith, a CH₂ functional group (δ_{H} 2.70 and 2.51 and δ_{C} 36.25) generated by ΔfkbD-fkbM gene was observed. Based on 2D-NMR data, the structure of the compound was determined as 9-deoxo-31-O-demethyl-FK523.

### Example 10: Identification of immunosuppressive activity of nine compounds

The degrees of decreases in immunosuppressive activity of the nine compounds were identified by a known *in vitro* T cell activity assay (J. Immunol. 143:718-726, 1989).

CD4+ T cell division is an indicator of immune response. When CD4+ T cells stained with Cell Trace^{™} Violet (CTV) proliferate by immune responses, the amount of CTV of each cell decreases, and thus the degrees of immunosuppressive activity were identified using this as an indicator.

Single cells were isolated from spleen of 6- to 8-week-old B6J experimental mice, and CD4+ T cells were isolated using a MagniSort^{®} Mouse CD4 T cell Enrichment Kit (eBioscience). CD4+ T cells were stained with a Cell Trace^{™} Violet (CTV) Cell Proliferation Kit (Molecular Probes), and FK506 or the nine novel compounds were added thereto in concentrations of 0.01 ng/mL, 0.1 ng/mL, 1 ng/mL, 10 ng/mL, 100 ng/mL, and 1000 ng/mL, respectively, followed by incubation for 72 hours. In order to activate T cells, Dynabeads^{®} Mouse T-Activator CD3/CD28 (Gibco) was used. As a control, inactivated T cells were used. After incubation, CTV intensity was analyzed by flow cytometry.

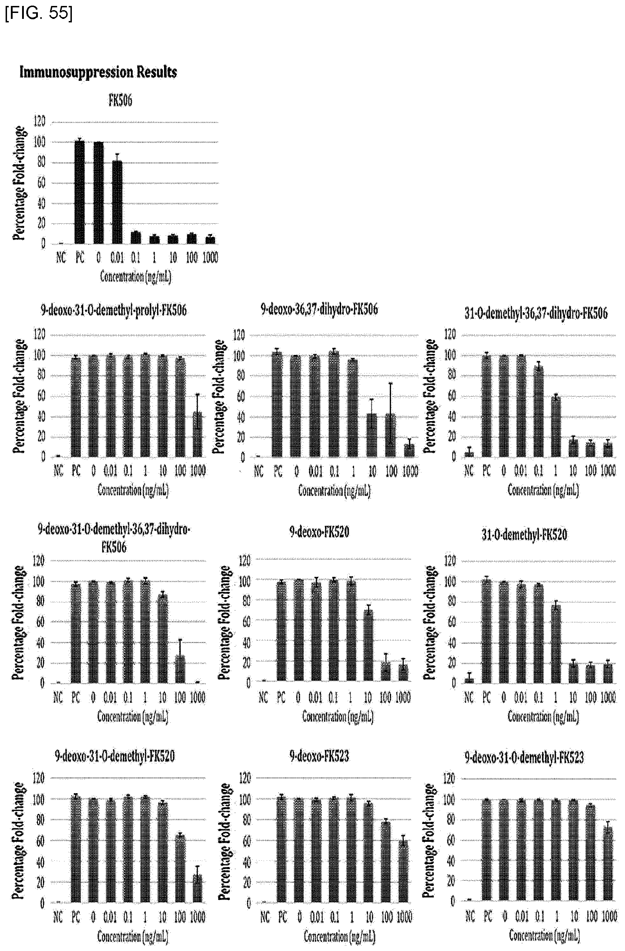

Table 12 below and FIG. 55 show the degrees of T cell proliferation based on CTV intensities measured using a flow cytometer, indicating the degrees of immunosuppressive activity of FK506 and the nine novel compounds. As shown in Table 12 below and FIG. 55, all compounds provided by the present invention exhibited reduced immunosuppressive activity compared to that of FK506.

**Table 12**

| Structural analogs | Immunosuppression IC₅₀ (ng/mL) |
|---|---|
| FK506 | 0.027 |
| 9-deoxo-prolyl-FK506 | 268.3 |
| 9-deoxo-FK506 | 0.513 |
| 31-O-demethyl-FK506 | 0.246 |
| 9-deoxo-31-O-demethyl-FK506 | 15.09 |
| 9-deoxo-31-O-demethyl-prolyl-FK506 | 886.5 |
| 9-deoxo-36,37-dihydro-FK506 | 14.1 |
| 31-*O*-demethyl-36,37-dihydro-FK506 | 1.723 |
| 9-deoxo-31-*O*-demethyl-36,37-dihydro-FK506 | 45.87 |
| 9-deoxo-FK520 | 24.98 |
| 31-*O*-demethyl-FK520 | 3.273 |
| 9-deoxo-31-*O*-demethyl-FK520 | 258.1 |
| 9-deoxo-FK523 | 1985 |
| 9-deoxo-31-*O*-demethyl-FK523 | 3378 |

Based on the results described above, it was confirmed that the nine compounds according to the present invention had significantly decreased immunosuppressive activity compared to FK506, and thus it was determined that the composition for promoting hair growth including at least one compound selected from the nine compounds as an active ingredient may be used without concern as to side effects caused by immunosuppressive activity.

### Example 11: Identification of hair growth-promoting activity of nine compounds

Hair growth-promoting activity of the nine compounds of the present invention relative to that of the parent drug (FK506) was evaluated using vibrissae tissue of mice. The mouth and mouth tissue (whisker pad) around the mouth where thick hair grows were peeled off from each of 6- to 8-week-old experimental black mouse (C57BL/6 mouse) and treated with 70% for a short time of several seconds. The treated mouth tissue was prepared in a state of being immersed in phosphate-buffered saline (PBS) supplemented with 1% penicillin/streptomycin (P/S) on ice. Surrounding subcutaneous fat tissue and connective tissue were removed using forceps and blades while observing the prepared mouth tissue using a dissecting microscope to expose vibrissae tissue. Then, vibrissae were isolated from mouth tissue using forceps and blades with respect to hair follicles, and the vibrissae were added to the PBS containing 1% P/S. Subsequently, a vibrissae culture medium (serum-free Dulbecco's Modified Eagle Medium (DMEM) containing 1% P/S and 12.5 µg/mL gentamicin) was filled in a well plate, and each of the separated vibrissae was added to each well. In addition, they were incubated in an incubator at 37°C with 5% CO₂ while changing the culture medium with a fresh culture medium every 2 days. In order to evaluate effects of the nine compounds on the growth of vibrissae, the nine novel compounds were mixed with the culture medium, and then evaluation was performed by measuring length, area, volume, and the like of vibrissae relative to reference values.

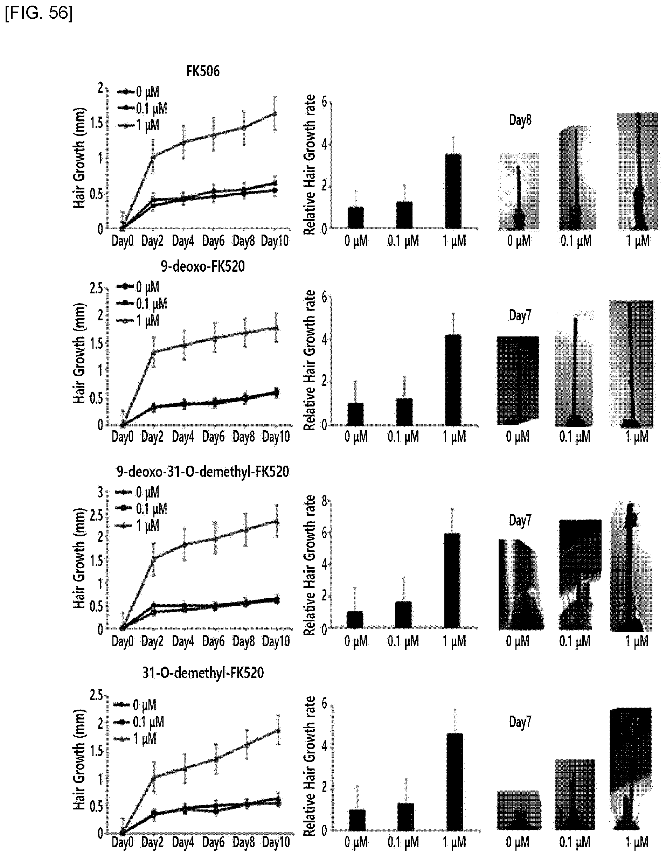

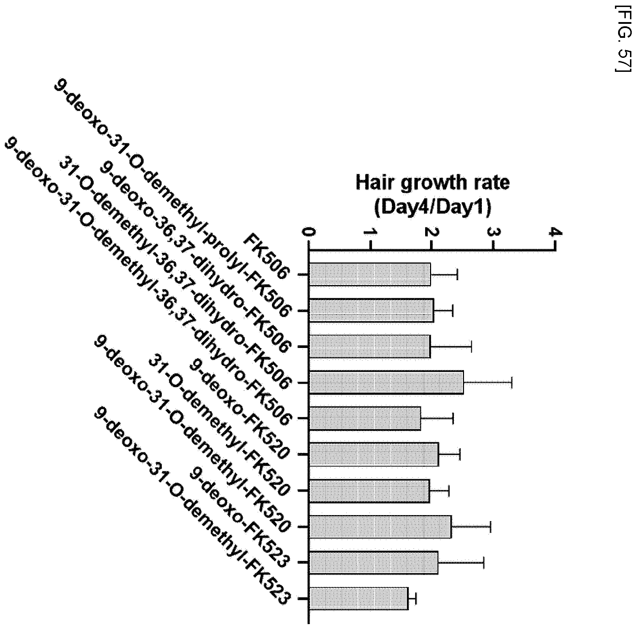

As a result, it was confirmed that all of the nine compounds may provide the same level of hair growth-promoting activity as that of the parent drug, FK506. Results of some of the compounds are shown in FIG. 56 as representative examples. These examples are made for illustrative purposes instead of emphasizing particular compounds. Furthermore, results of measuring the growth of the vibrissae in the length by the compounds are shown in FIG. 57. As shown in FIG. 57, it was confirmed that the lengths of vibrissae increased due to both the compounds and FK506.

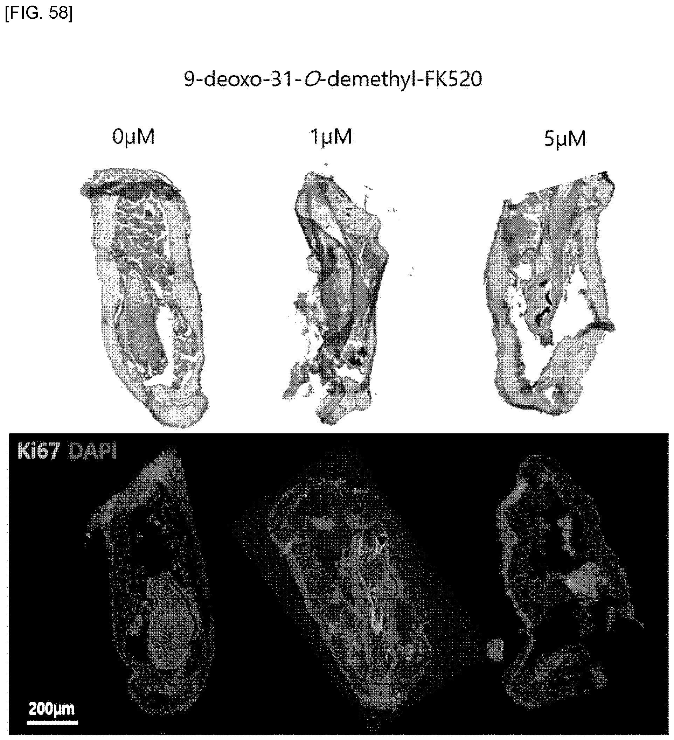

Also, histological results of vibrissae cultured in a state of being treated with some of the compounds are shown in FIG. 58. It was confirmed that a growth marker (Ki67) of cells was overexpressed due to the compound 9-deoxo-31-*O*-demethyl-FK520 compared to an untreated group, and thus it was confirmed that the growth of vibrissae treated with the compound was continuously maintained. Based on the results, it was confirmed that the composition for promoting hair growth, including at least one of the nine compounds of the present invention as an active ingredient, had the effects on promoting hair growth as desired, and thus it was determined that the composition may be effectively used for preventing and treating hair loss.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention.

## Claims

1. A compound for use in alleviating, preventing or treating alopecia, which is selected from the group consisting of 9-deoxo-31-*O*-demethyl-prolyl-FK506 represented by Formula 1 below, 9-deoxo-36,37-dihydro-FK506 represented by Formula 2 below, 31-*O*-demethyl-36,37-dihydro-FK506 represented by Formula 3 below, 9-deoxo-31-*O*-demethyl-36,37-dihydro-FK506 represented by Formula 4 below, 9-deoxo-FK520 represented by Formula 5 below, 31-*O*-demethyl-FK520 represented by Formula 6 below, 9-deoxo-31-*O*-demethyl-FK520 represented by Formula 7 below, 9-deoxo-FK523 represented by Formula 8 below, and 9-deoxo-31-*O*-demethyl-FK523 represented by Formula 9 below, or a pharmaceutically acceptable salt thereof as an active ingredient: 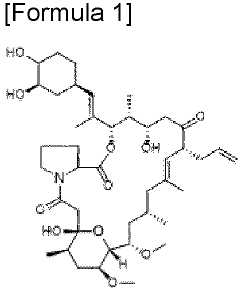 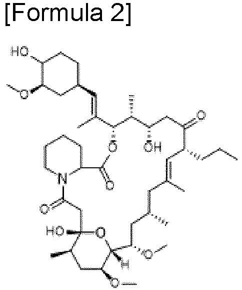 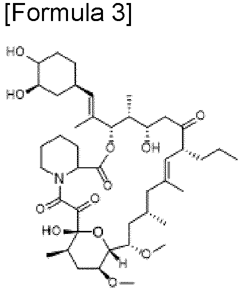 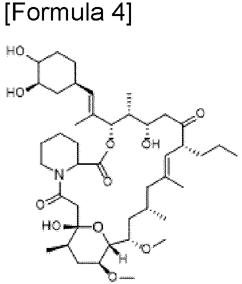 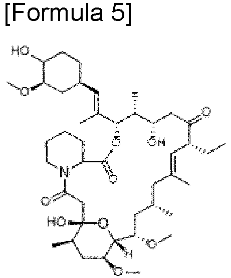 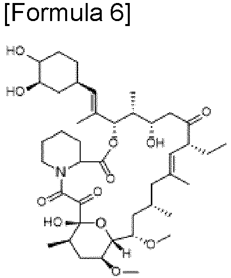 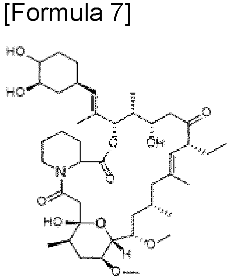 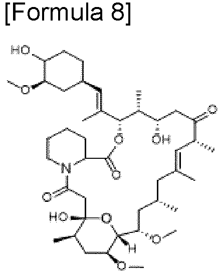 and

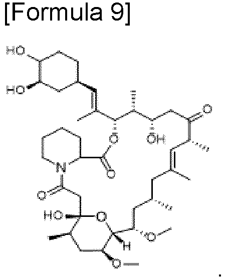

2. The compound for use according to claim 1, wherein the 31-*O*-demethyl-36,37-dihydro-FK506 is produced by culturing *Streptomyces kanamyceticus* ΔfkbM,tcsD (accession number: KCTC13584BP) as a production strain.

3. The compound for use according to claim 1, wherein the 9-deoxo-31-*O-*demethyl-36,37-dihydro-FK506 is produced by culturing *Streptomyces kanamyceticus* ΔfkbD-fkbM,tcsD (accession number: KCTC13585BP) as a production strain.

4. The compound for use according to claim 1, wherein the 9-deoxo-FK520 is produced by culturing *Streptomyces kanamyceticus* ΔfkbD,tcsB (accession number: KCTC13579BP) or *Streptomyces kanamyceticus* ΔfkbD,tcsD (accession number: KCTC13580BP) as a production strain.

5. The compound for use according to claim 1, wherein the 31-*O*-demethyl-FK520 is produced by culturing *Streptomyces kanamyceticus* ΔfkbM,tcsB (accession number: KCTC13583BP) or *Streptomyces kanamyceticus* ΔfkbM,tcsD (accession number: KCTC13584BP) as a production strain.

6. The compound for use according to claim 1, wherein the 9-deoxo-31-*O-*demethyl-FK520 is produced by culturing *Streptomyces kanamyceticus* ΔfkbD-fkbM,tcsB (accession number: KCTC13582BP) or *Streptomyces kanamyceticus* ΔfkbD-fkbM,tcsD (accession number: KCTC13585BP) as a production strain.

7. The compound for use according to claim 1, wherein the alopecia comprises cicatricial alopecia; or at least one non-cicatricial alopecia selected from the group consisting of infectious hair loss, traumatic hair loss, inflammatory hair loss, congenital hair loss, endocrine hair loss, alopecia neoplastica, malnutrition hair loss, drug-induced hair loss, hair loss caused by abnormal hair structure, male-pattern hair loss, female-pattern hair loss, and alopecia areata.

8. A cosmetic method of promoting hair growth, or alleviating, preventing, or treating hair loss, the method comprising:
administering a composition comprising a compound selected from the group consisting of 9-deoxo-31-*O*-demethyl-prolyl-FK506 represented by Formula 1 below, 9-deoxo-36,37-dihydro-FK506 represented by Formula 2 below, 31-*O*-demethyl-36,37-dihydro-FK506 represented by Formula 3 below, 9-deoxo-31-*O*-demethyl-36,37-dihydro-FK506 represented by Formula 4 below, 9-deoxo-FK520 represented by Formula 5 below, 31-*O*-demethyl-FK520 represented by Formula 6 below, 9-deoxo-31-*O*-demethyl-FK520 represented by Formula 7 below, 9-deoxo-FK523 represented by Formula 8 below, and 9-deoxo-31-*O*-demethyl-FK523 represented by Formula 9 below, or a cosmetically acceptable salt thereof as an active ingredient: 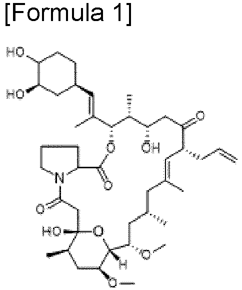 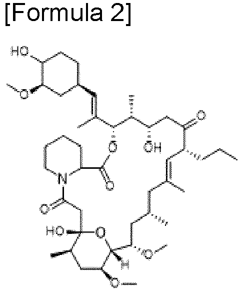 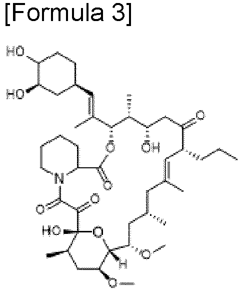 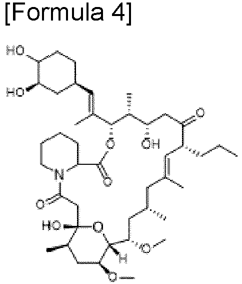 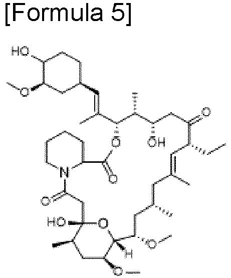 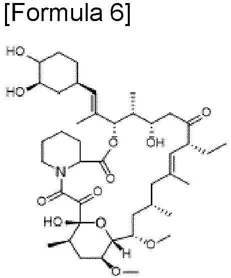 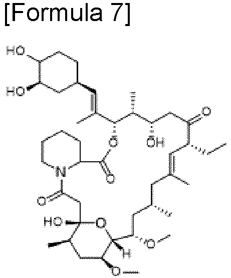 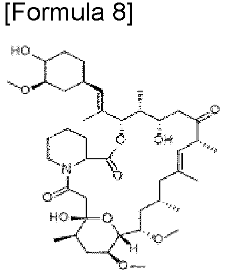 and

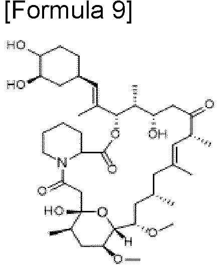

9. A composition for use in alleviating, preventing or treating alopecia, comprising a compound selected from the group consisting of 9-deoxo-31-*O*-demethyl-prolyl-FK506 represented by Formula 1 below, 9-deoxo-36,37-dihydro-FK506 represented by Formula 2 below, 31-*O*-demethyl-36,37-dihydro-FK506 represented by Formula 3 below, 9-deoxo-31-*O*-demethyl-36,37-dihydro-FK506 represented by Formula 4 below, 9-deoxo-FK520 represented by Formula 5 below, 31-*O*-demethyl-FK520 represented by Formula 6 below, 9-deoxo-31-*O*-demethyl-FK520 represented by Formula 7 below, 9-deoxo-FK523 represented by Formula 8 below, and 9-deoxo-31-*O*-demethyl-FK523 represented by Formula 9 below, or a pharmaceutically acceptable salt thereof as an active ingredient, and a pharmaceutically acceptable excipient: 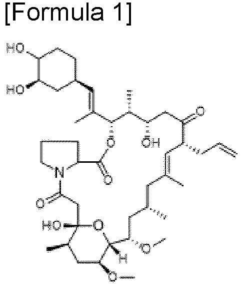 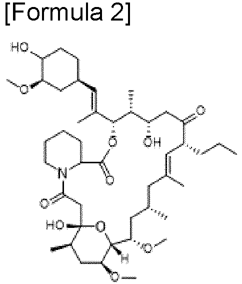 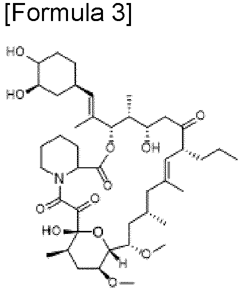 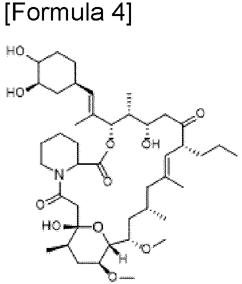 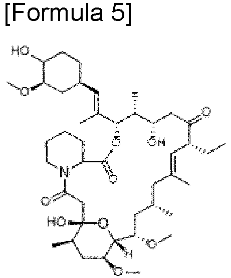 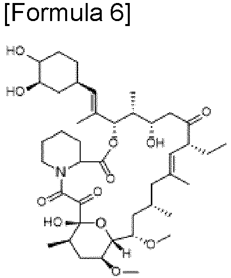 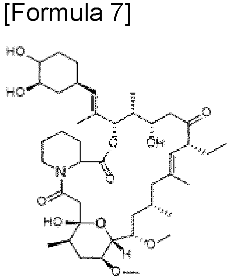 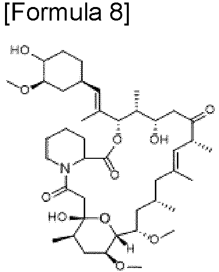 and

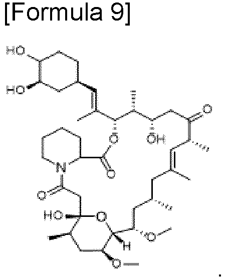

10. A compound selected from the group consisting of 9-deoxo-31-*O*-demethyl-prolyl-FK506 represented by Formula 1 below, 9-deoxo-36,37-dihydro-FK506 represented by Formula 2 below, 9-deoxo-FK523 represented by Formula 8 below, and 9-deoxo-31-*O*-demethyl-FK523 represented by Formula 9 below, or a cosmetically acceptable salt thereof: 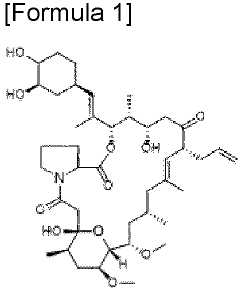 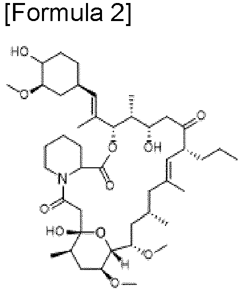 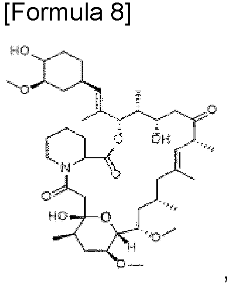 and

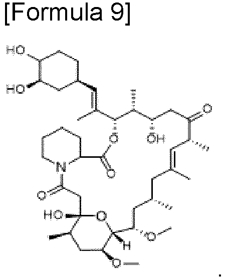

11. The compound of claim 10, wherein the 9-deoxo-31-*O-*demethyl-prolyl-FK506 is produced by culturing *Streptomyces kanamyceticus* ΔfkbD-fkbM (accession number: KCTC13581BP) as a production strain.

12. The compound of claim 10, wherein the 9-deoxo-36,37-dihydro-FK506 is produced by culturing *Streptomyces kanamyceticus* ΔfkbD,tcsD (accession number: KCTC13580BP) as a production strain.

13. The compound of claim 10, wherein the 9-deoxo-FK523 is produced by culturing *Streptomyces kanamyceticus* ΔfkbD,tcsB (accession number: KCTC13579BP) as a production strain.

14. The compound of claim 10, wherein the 9-deoxo-31-*O*-demethyl-FK523 is produced by culturing *Streptomyces kanamyceticus* ΔfkbD-fkbM,tcsB (accession number: KCTC13582BP) as a production strain.

## Patentansprüche

1. Verbindung zur Verwendung bei der Linderung, Prävention oder Behandlung von Alopezie, die ausgewählt ist aus der Gruppe bestehend aus 9-Deoxo-31-*O*-demethyl-prolyl-FK506, dargestellt durch die folgende Formel 1, 9-Deoxo-36,37-dihydro-FK506, dargestellt durch die folgende Formel 2, 31-*O*-demethyl-36,37-dihydro-FK506, dargestellt durch die folgende Formel 3, 9-Deoxo-31-*O*-demethyl-36,37-dihydro-FK506 dargestellt durch die folgende Formel 4, 9-Deoxo-FK520 dargestellt durch die folgende Formel 5, 31-*O*-demethyl-FK520 dargestellt durch die folgende Formel 6, 9-Deoxo-31-*O*-demethyl-FK520 dargestellt durch die folgende Formel 7, 9-Deoxo-FK523 dargestellt durch die folgende Formel 8, und 9-Deoxo-31-*O*-demethyl-FK523 dargestellt durch die folgende Formel 9, oder ein pharmazeutisch akzeptables Salz davon als Wirkstoff: 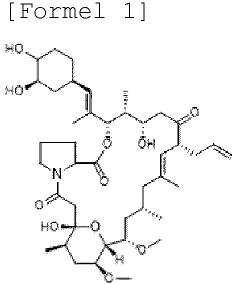 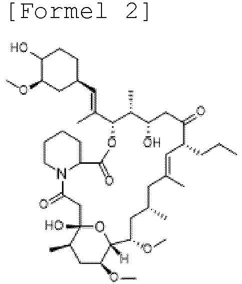 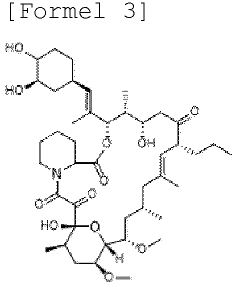 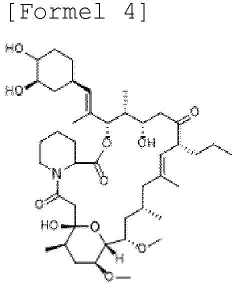 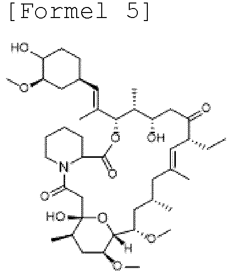 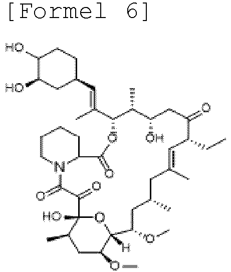 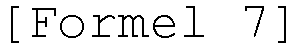 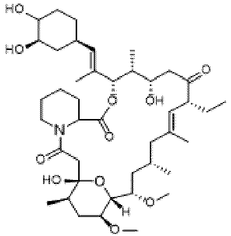 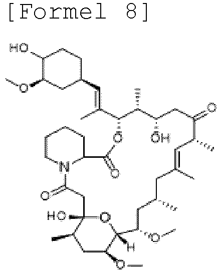 und

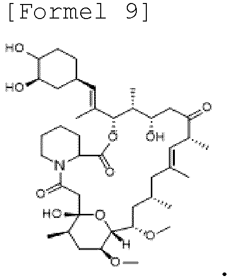

2. Verbindung zur Verwendung nach Anspruch 1, wobei das 31-*O-*Demethyl-36,37-dihydro-FK506 durch Kultivierung von *Streptomyces kanamyceticus* ΔfkbM, tcsD (Hinterlegungsnummer: KCTC13584BP) als Produktionsstamm hergestellt wird.

3. Verbindung zur Verwendung nach Anspruch 1, wobei das 9-Deoxo-31-*O*-demethyl-36,37-dihydro-FK506 durch Kultivierung von *Streptomyces kanamyceticus* ΔfkbD-fkbM, tcsD (Hinterlegungsnummer: KCTC13585BP) als Produktionsstamm hergestellt wird.

4. Verbindung zur Verwendung nach Anspruch 1, wobei das 9-Deoxo-FK520 durch Kultivierung von *Streptomyces kanamyceticus* ΔfkbD,tcsB (Hinterlegungsnummer: KCTC13579BP) oder *Streptomyces kanamyceticus* ΔfkbD,tcsD (Hinterlegungsnummer: KCTC13580BP) als Produktionsstamm hergestellt wird.

5. Verbindung zur Verwendung nach Anspruch 1, wobei das 31-*O-*Demethyl-FK520 durch Kultivierung von *Streptomyces kanamyceticus* ΔfkbM, tcsB (Hinterlegungsnummer: KCTC13583BP) oder *Streptomyces kanamyceticus* ΔfkbM, tcsD (Hinterlegungsnummer: KCTC13584BP) als Produktionsstamm hergestellt wird.

6. Verbindung zur Verwendung nach Anspruch 1, wobei das 9-Deoxo-31-O-demethyl-FK520 durch Kultivierung von *Streptomyces kanamyceticus* ΔfkbD-fkbM, tcsB (Hinterlegungsnummer: KCTC13582BP) oder *Streptomyces kanamyceticus* ΔfkbD-fkbM, tcsD (Hinterlegungsnummer: KCTC13585BP) als Produktionsstamm hergestellt wird.

7. Verbindung zur Verwendung nach Anspruch 1, wobei die Alopezie narbige Alopezie umfasst; oder mindestens eine nicht-narbige Alopezie, ausgewählt aus der Gruppe bestehend aus infektiösem Haarausfall, traumatischem Haarausfall, entzündlichem Haarausfall, kongenitalem Haarausfall, endokrinem Haarausfall, Alopezie neoplastica, Haarausfall durch Mangelernährung, Haarausfall durch Arzneimittel, Haarausfall verursacht durch abnorme Haarstruktur, Haarausfall männlichen Musters, Haarausfall weiblichen Musters und Alopezie areata.

8. Kosmetisches Verfahren zur Förderung des Haarwachstums oder zur Linderung, Verhinderung oder Behandlung von Haarausfall, wobei das Verfahren umfasst:
Anwendung einer Zusammensetzung, die einen Wirkstoff umfasst, ausgewählt aus der Gruppe bestehend aus 9-Deoxo-31-*O*-demethyl-prolyl-FK506, dargestellt durch die Formel 1 unten, 9-Deoxo-36,37-dihydro-FK506, dargestellt durch die Formel 2 unten, 31-*O*-demethyl-36,37-dihydro-FK506, dargestellt durch die Formel 3 unten, 9-Deoxo-31-*O*-demethyl-36,37-dihydro-FK506, dargestellt durch die Formel 4 unten, 9-Deoxo-FK520, dargestellt durch die Formel 5 unten, 31-*O*-demethyl-FK520, dargestellt durch die Formel 6 unten, 9-Deoxo-31-*O*-demethyl-FK520, dargestellt durch die Formel 7 unten, 9-Deoxo-FK523, dargestellt durch die Formel 8 unten, und 9-Deoxo-31-*O*-demethyl-FK523, dargestellt durch die Formel 9 unten, oder ein kosmetisch akzeptables Salz davon als aktiven Inhaltsstoff: 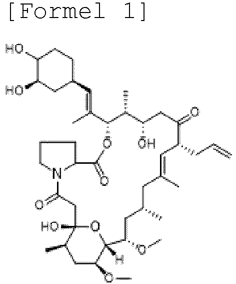 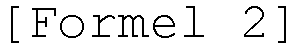 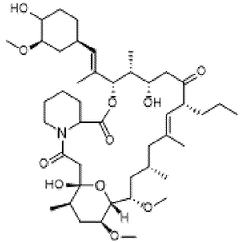 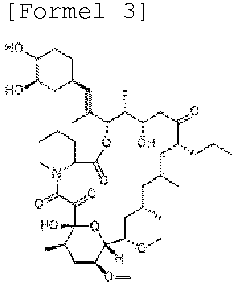 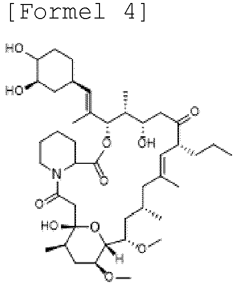 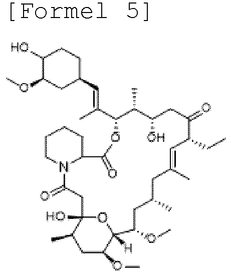 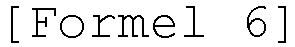 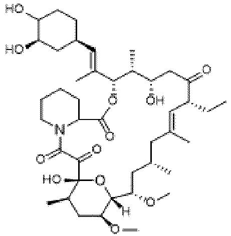 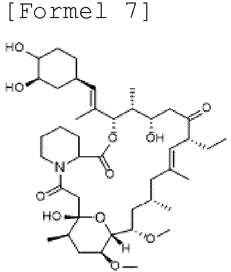 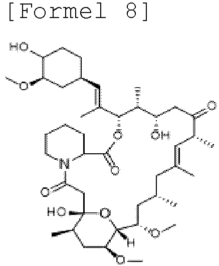 und

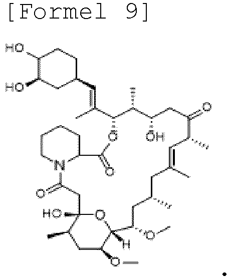

9. Zusammensetzung zur Verwendung bei der Linderung, Prävention oder Behandlung von Alopezie, die einen Wirkstoff umfasst, ausgewählt aus der Gruppe bestehend aus 9-Deoxo-31-*O*-demethyl-prolyl-FK506, dargestellt durch Formel 1 unten, 9-Deoxo-36,37-dihydro-FK506, dargestellt durch Formel 2 unten, 31-*O*-demethyl-36,37-dihydro-FK506, dargestellt durch Formel 3 unten, 9-Deoxo-31-*O*-demethyl-36,37-dihydro-FK506, dargestellt durch Formel 4 unten, 9-Deoxo-FK520, dargestellt durch Formel 5 unten, 31-*O-*demethyl-FK520, dargestellt durch Formel 6 unten, 9-Deoxo-31-*O-*demethyl-FK520, dargestellt durch Formel 7 unten, 9-Deoxo-FK523, dargestellt durch Formel 8 unten, und 9-Deoxo-31-*O*-demethyl-FK523, dargestellt durch Formel 9 unten, oder ein pharmazeutisch akzeptables Salz davon als Wirkstoff, sowie einen pharmazeutisch akzeptablen Hilfsstoff: 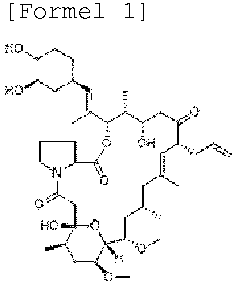 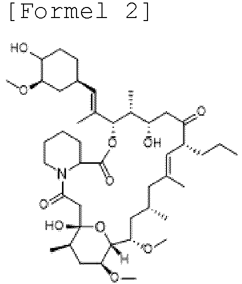 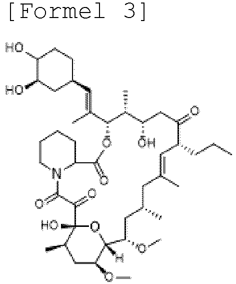 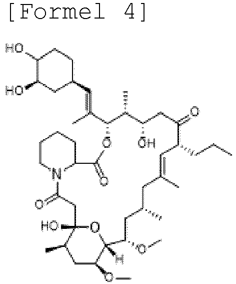 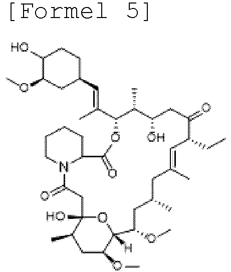 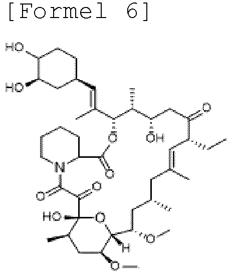 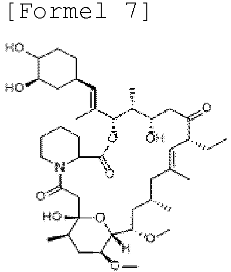 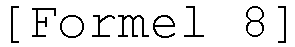 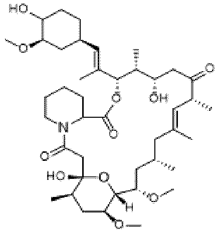 und

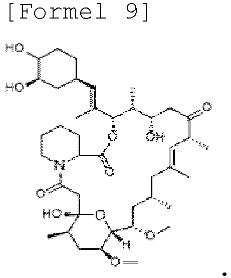

10. Verbindung ausgewählt aus der Gruppe bestehend aus 9-Deoxo-31-*O*-demethyl-prolyl-FK506, dargestellt durch die folgende Formel 1, 9-Deoxo-36,37-dihydro-FK506, dargestellt durch die folgende Formel 2, 9-Deoxo-FK523, dargestellt durch die folgende Formel 8, und 9-Deoxo-31- *O*-demethyl-FK523, dargestellt durch die folgende Formel 9, oder ein kosmetisch akzeptables Salz davon: 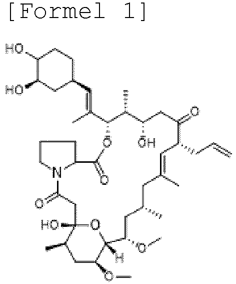 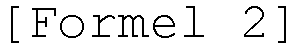 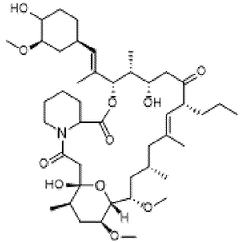 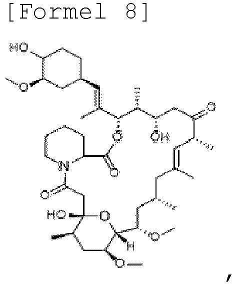 und

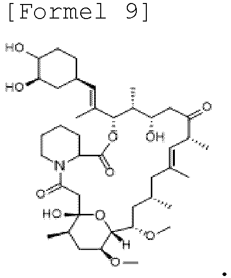

11. Verbindung nach Anspruch 10, wobei die 9-Deoxo-31-*O*-demethyl-prolyl-FK506 durch Kultivierung von *Streptomyces kanamyceticus* ΔfkbD-fkbM (Hinterlegungsnummer: KCTC13581BP) als Produktionsstamm hergestellt wird.

12. Verbindung nach Anspruch 10, wobei das 9-Deoxo-36,37-dihydro-FK506 durch Kultivierung von *Streptomyces kanamyceticus* ΔfkbD,tcsD (Hinterlegungsnummer: KCTC13580BP) als Produktionsstamm hergestellt wird.

13. Verbindung nach Anspruch 10, wobei das 9-Deoxo-FK523 durch Kultivierung von *Streptomyces kanamyceticus* ΔfkbD,tcsB (Hinterlegungsnummer: KCTC13579BP) als Produktionsstamm hergestellt wird.

14. Verbindung nach Anspruch 10, wobei das 9-Deoxo-31-*O*-demethyl-FK523 durch Kultivierung von *Streptomyces kanamyceticus* ΔfkbD-fkbM,tcsB (Hinterlegungsnummer: KCTC13582BP) als Produktionsstamm hergestellt wird.

## Revendications

1. Composé pour une utilisation pour soulager, prévenir ou traiter l'alopécie, qui est choisi dans le groupe constitué par le 9-désoxo-31-*O*-déméthyl-prolyl-FK506 représenté par la formule 1 ci-dessous, le 9-désoxo-36,37-dihydro-FK506 représenté par la formule 2 ci-dessous, le 31-*O*-déméthyl-36,37-dihydro-FK506 représenté par la formule 3 ci-dessous, le 9-désoxo-31-*O-*déméthyl-36,37-dihydro-FK506 représenté par la formule 4 ci-dessous, le 9-désoxo-FK520 représenté par la formule 5 ci-dessous, le 31-*O*-déméthyl-FK520 représenté par la formule 6 ci-dessous, le 9-désoxo-31-*O*-déméthyl-FK520 représenté par la formule 7 ci-dessous, le 9-désoxo-FK523 représenté par la formule 8 ci-dessous, et le 9-désoxo-31-*O*-déméthyl-FK523 représenté par la formule 9 ci-dessous, ou un sel pharmaceutiquement acceptable de celui-ci en tant qu'ingrédient actif : 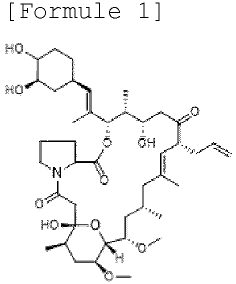 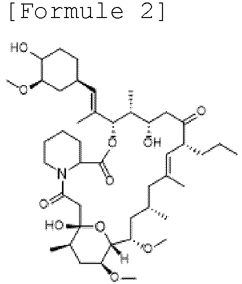 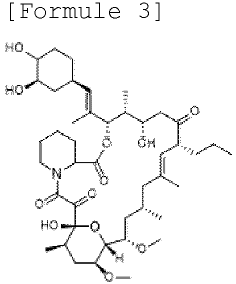 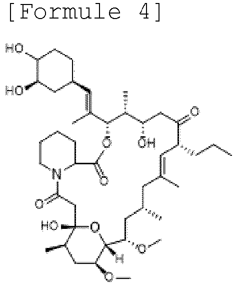 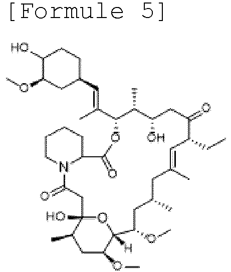 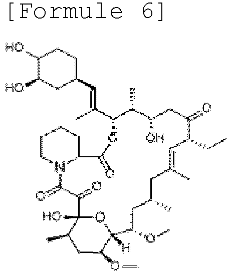 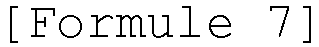 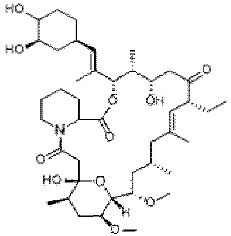 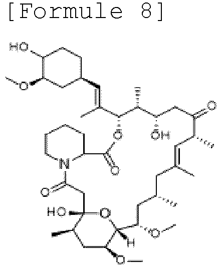 et

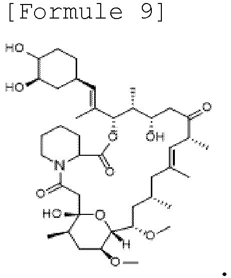

2. Composé pour une utilisation selon la revendication 1, dans lequel le 31-*O*-déméthyl-36,37-dihydro-FK506 est produit par culture de *Streptomyces kanamyceticus* ΔfkbM, tcsD (numéro d'accès : KCTC13584BP) en tant que souche de production.

3. Composé pour une utilisation selon la revendication 1, dans lequel le 9-déoxo-31-*O*-déméthyl-36,37-dihydro-FK506 est produit par culture de *Streptomyces kanamyceticus* ΔfkbD-fkbM,tcsD (numéro d'accès : KCTC13585BP) en tant que souche de production.

4. Composé pour une utilisation selon la revendication 1, dans lequel le 9-déoxo-FK520 est produit par culture de *Streptomyces kanamyceticus* ΔfkbD,tcsB (numéro d'accès : KCTC13579BP) ou de *Streptomyces kanamyceticus* ΔfkbD,tcsD (numéro d'accès : KCTC13580BP) en tant que souche de production.

5. Composé pour une utilisation selon la revendication 1, dans lequel le 31-*O*-déméthyl-FK520 est produit par culture de *Streptomyces kanamyceticus* ΔfkbM,tcsB (numéro d'accès : KCTC13583BP) ou *Streptomyces kanamyceticus* ΔfkbM,tcsD (numéro d'accès : KCTC13584BP) en tant que souche de production.

6. Composé pour une utilisation selon la revendication 1, dans lequel le 9-déoxo-31-*O*-déméthyl-FK520 est produit par culture de *Streptomyces kanamyceticus* ΔfkbD-fkbM,tcsB (numéro d'accès : KCTC13582BP) ou *Streptomyces kanamyceticus* ΔfkbD-fkbM,tcsD (numéro d'accès : KCTC13585BP) en tant que souche de production.

7. Composé pour une utilisation selon la revendication 1, dans lequel l'alopécie comprend une alopécie cicatricielle ; ou au moins une alopécie non cicatricielle choisie dans le groupe constitué par la perte de cheveux infectieuse, la perte de cheveux traumatique, la perte de cheveux inflammatoire, la perte de cheveux congénitale, la perte de cheveux endocrinienne, l'alopécie néoplasique, la perte de cheveux due à la malnutrition, la perte de cheveux induite par des médicaments, la perte de cheveux causée par une structure capillaire anormale, la perte de cheveux de type masculin, la perte de cheveux de type féminin et l'alopécie areata.

8. Procédé cosmétique pour favoriser la croissance des cheveux, ou pour soulager, prévenir ou traiter la chute des cheveux, le procédé comprenant : l'administration d'une composition comprend un composé choisi dans le groupe constitué par le 9-désoxo-31-*O*-déméthyl-prolyl-FK506 représenté par la formule 1 ci-dessous, le 9-désoxo-36,37-dihydro-FK506 représenté par la formule 2 ci-dessous, le 31-*O-*déméthyl-36,37-dihydro-FK506 représenté par la formule 3 ci-dessous, le 9-désoxo-31-*O*-déméthyl-36,37-dihydro-FK506 représenté par la formule 4 ci-dessous, le 9-désoxo-FK520 représenté par la formule 5 ci-dessous, le 31-*O*-déméthyl-FK520 représenté par la formule 6 ci-dessous, le 9-désoxo-31-*O-*déméthyl-FK520 représenté par la formule 7 ci-dessous, le 9-désoxo-FK523 représenté par la formule 8 ci-dessous, et le 9-désoxo-31-*O*-déméthyl-FK523 représenté par la formule 9 ci-dessous, ou un sel acceptable sur le plan cosmétique de celui-ci en tant qu'ingrédient actif : 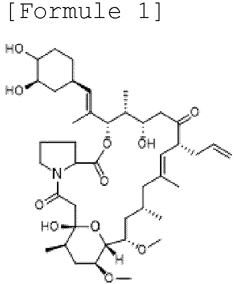 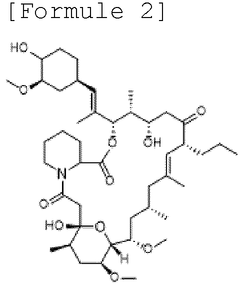 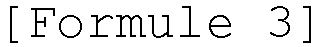 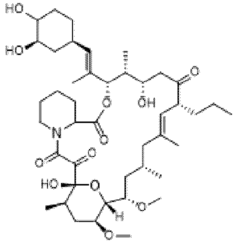 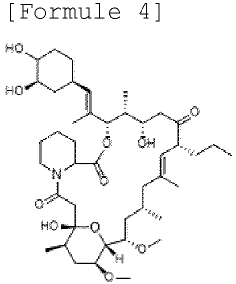 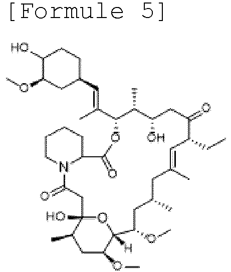 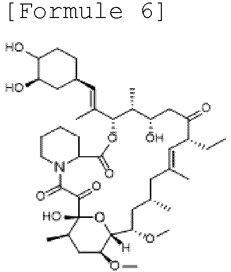 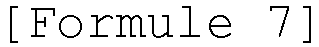 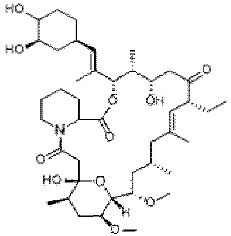 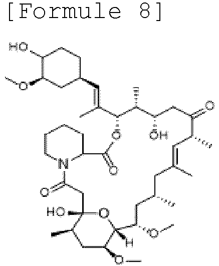 et

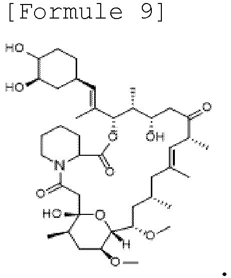

9. Composition pour une utilisation pour soulager, prévenir ou traiter l'alopécie, comprenant un composé choisi dans le groupe constitué par le 9-désoxo-31-*O*-déméthyl-prolyl-FK506 représenté par la formule 1 ci-dessous, le 9-désoxo-36,37-dihydro-FK506 représenté par la formule 2 ci-dessous, le 31-*O*-déméthyl-36,37-dihydro-FK506 représenté par la formule 3 ci-dessous, le 9-désoxo-31-*O*-déméthyl-36,37-dihydro-FK506 représenté par la formule 4 ci-dessous, le 9-désoxo-FK520 représenté par la formule 5 ci-dessous, le 31-*O*-déméthyl-FK520 représenté par la formule 6 ci-dessous, le 9-désoxo-31-*O*-déméthyl-FK520 représenté par la formule 7 ci-dessous, le 9-désoxo-FK523 représenté par la formule 8 ci-dessous, et le 9-désoxo-31-*O-*déméthyl-FK523 représenté par la formule 9 ci-dessous, ou un sel pharmaceutiquement acceptable de celui-ci en tant qu'ingrédient actif, et un excipient pharmaceutiquement acceptable : 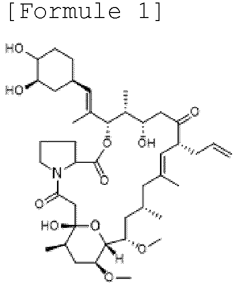 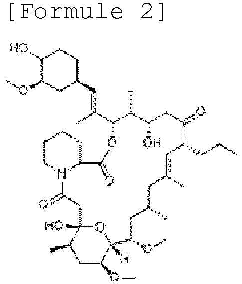 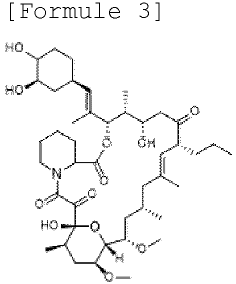 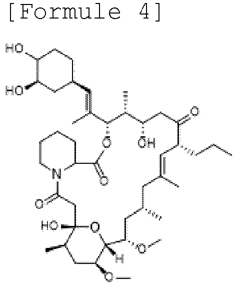 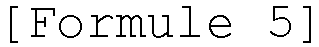 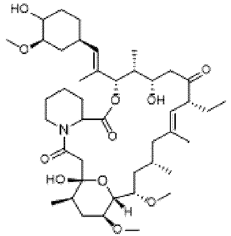 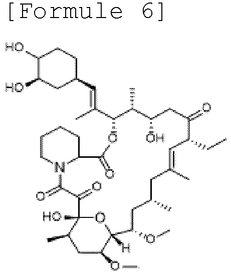 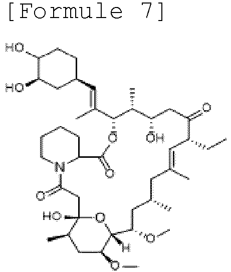 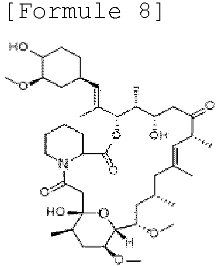 et

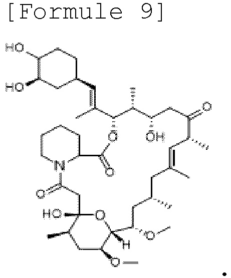

10. Composé choisi dans le groupe constitué par le 9-déoxo-31-*O-*déméthyl-prolyl-FK506 représenté par la formule 1 ci-dessous, le 9-déoxo-36,37-dihydro-FK506 représenté par la formule 2 ci-dessous, le 9-déoxo-FK523 représenté par la formule 8 ci-dessous et le 9-déoxo-31-*O*-déméthyl-FK523 représenté par la formule 9 ci-dessous, ou un sel acceptable sur le plan cosmétique de celui-ci : 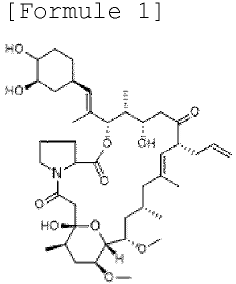 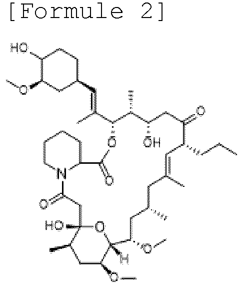 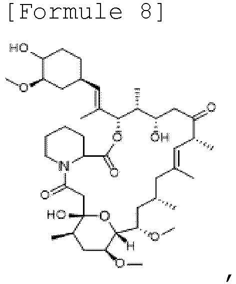 et

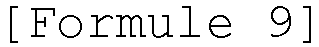

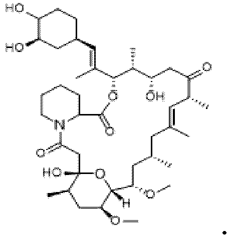

11. Composé selon la revendication 10, dans lequel le 9-déoxo-31-*O-*déméthyl-prolyl-FK506 est produit par culture de *Streptomyces kanamyceticus* ΔfkbD-fkbM (numéro d'accès : KCTC13581BP) en tant que souche de production.

12. Composé selon la revendication 10, dans lequel le 9-déoxo-36,37-dihydro-FK506 est produit par culture de *Streptomyces kanamyceticus* ΔfkbD,tcsD (numéro d'accès : KCTC13580BP) en tant que souche de production.

13. Composé selon la revendication 10, dans lequel le 9-déoxo-FK523 est produit par culture de *Streptomyces kanamyceticus* ΔfkbD,tcsB (numéro d'accès : KCTC13579BP) en tant que souche de production.

14. Composé selon la revendication 10, dans lequel le 9-déoxo-31-*O-*déméthyl-FK523 est produit par culture de *Streptomyces kanamyceticus* ΔfkbD-fkbM,tcsB (numéro d'accès : KCTC13582BP) en tant que souche de production.
